# EUROPEAN PATENT APPLICATION

(11) **EP 4 617 290 A1**
(43) Date of publication of application: **17.09.2025**
(21) Application number: 23905914.0
(22) Date of filing: 19.12.2023
(51) Int. Cl.: C07K 19/00, C07K 16/28, C12N 15/13, A61K 39/00, A61P 35/00

(54) **ANTIBODY AND USE THEREOF**

(30) Priority: 21.12.2022 CN 202211646964; 27.06.2023 CN 202310771156
(71) Applicant: Sichuan Huiyu Pharmaceutical Co., Ltd., Neijiang, Sichuan 641000 (CN); Sichuan Huiyu Seacross Pharmaceutical Technology Co., Ltd., Chengdu, Sichuan 610200 (CN)
(72) Inventor: HU, Yan, Neijiang, Sichuan 641000 (CN); GUO, Sanyou, Neijiang, Sichuan 641000 (CN); LIU, Mengting, Neijiang, Sichuan 641000 (CN); WEI, Tao, Neijiang, Sichuan 641000 (CN); DING, Zhao, Neijiang, Sichuan 641000 (CN)
(74) Representative: Murgitroyd & Company
(86) International application number: PCT/CN2023/139722
(87) International publication number: WO 2024/131751

(57) **Abstract**

The present invention relates to a humanized single-domain antibody or an antigen-binding fragment thereof targeting MSLN or PD-L1. The present invention also relates to a multispecific antibody, and particularly provides a multispecific antibody targeting CD3, a tumor-associated antigen and an immune checkpoint, wherein the multispecific antibody has good medication safety and anti-tumor efficacy. The present invention further relates to use of the single-domain antibody or the multispecific antibody in disease treatment.

## Description

### CROSS REFERENCE TO RELATED APPLICATIONS

The present application claims the priority of Chinese Patent Application 202211646964.4 filed on December 21, 2022 and Chinese Patent Application 202310771156.9 filed on June 27, 2023, both of which are incorporated herein by reference in their entirety.

### TECHNICAL FIELD

The present disclosure relates to a single-domain antibody targeting MSLN or PD-L1. The present disclosure further relates to a multispecific antibody, and specifically provides a multispecific antibody targeting CD3, tumor-associated antigens, and immune checkpoints. The present disclosure further relates to a use of the single-domain antibody or multispecific antibody in the treatment of diseases.

### BACKGROUND

Mesothelin (MSLN) is a cell surface glycoprotein encoded by the MSLN gene. Compared with normal tissues, MSLN is highly expressed in various cancers such as mesothelioma, ovarian cancer, lung cancer, esophageal cancer, pancreatic cancer, stomach cancer, cholangiocarcinoma, endometrial cancer, thymic carcinoma, colon cancer, and breast cancer, and its abnormal expression plays an important role in tumor cell proliferation, adhesion, and drug resistance. PD-L1, also known as B7-H1 or CD274, is the first characterized functional ligand of the coinhibitory programmed death receptor 1 (PD-1). Studies have found that PD-L1 is highly expressed in various types of tumors including melanoma, ovarian cancer, lung cancer, and kidney cancer. PD-L1 plays a key role in preventing autoimmunity in normal tissues and maintaining peripheral tolerance. Tumor cells can use the PD-1/PD-L1 signaling pathway to escape anti-tumor immune responses and eventually spread and metastasize. Therefore, blocking the PD-1/PD-L1 signaling pathway can activate endogenous anti-tumour immune responses, thereby exerting a therapeutic effect on tumors.

Currently, MSLN or PD-L1 antibodies of the art are mostly traditional IgG heavy-light chain antibodies. Nanobodies, also known as single-domain antibodies (sdAbs), are merely composed of heavy chains, having the characteristics of small molecular weight, good stability, and strong permeability, *etc.* However, there is currently still a lack of satisfactory nanobodies against MSLN or PD-L1 in the art, and therefore, there is a need to develop new specific nanobodies against MSLN or PD-L1 effectively. In addition, treating solid tumors with CD3 bispecific antibody (T Cell engage, TCE) drugs based on T cell activation has received widespread attention. TCE contains two domains, one domain binds to the CD3 of T cells, and the other domain targets and binds to cancer cells. Its molecular design is to help T cells recognize cancer cells and fully activation, releasing cytokines such as TNFα, perforin, granzyme B, and IFN-γ, and killing cancer cells. Multispecific antibody drugs associated with MSLN, as a TAA for solid tumors, are still under investigation, with HPN536 showing faster clinical progress. However, in clinical practice, treating solid tumors with TCE has poor effect, involving a risk of CRS, or problems such as T cell dysfunction resulted from T cell over-activation. Therefore, there is a need in the art to develop T cell activating drugs with better performance.

### CONTENT OF THE INVENTION

In regard with the above problems, the present disclosure provides a nanobody targeting MSLN or PD-L1 with excellent properties, and further provides a multispecific antibody targeting CD3, tumor-associated antigens (TAAs), and immune checkpoints, among which the part targeting CD3 can moderately activate T cells to improve drug safety, and further combining the parts targeting tumor-associated antigens (TAAs) and immune checkpoints can significantly increase the anti-tumor effect. The following aspects are thereby provided.

### CD3 antibody

In a first aspect, the present disclosure provides an antibody or an antigen-binding fragment thereof capable of specifically binding to CD3, having a moderate affinity for CD3, moderate ability to activate T cells, and reduced cytokine release, thereby improving medication safety. The antibody or the antigen-binding fragment thereof comprises a heavy chain variable region (VH) and a light chain variable region (VL), wherein
the VH comprises HCDR1 as set forth in SEQ ID NO: 55, HCDR2 as set forth in SEQ ID NO: 56, and HCDR3 as set forth in any one of SEQ ID NOs: 57-59 and 65; and/or,
the VL comprises LCDR1 as set forth in SEQ ID NO: 60, LCDR2 as set forth in SEQ ID NO: 61, and LCDR3 as set forth in SEQ ID NO: 62.

In some embodiments, the VH comprises the sequence as set forth in any one of SEQ ID NOs: 40-42 and 64.

In some embodiments, the VL comprises the sequence as set forth in SEQ ID NO: 43.

In some embodiments, the antibody or the antigen-binding fragment thereof that specifically binds to CD3 comprises a VH as set forth in SEQ ID NO: 40 and a VL as set forth in SEQ ID NO: 43.

In some embodiments, the antibody or the antigen-binding fragment thereof that specifically binds to CD3 comprises a VH as set forth in SEQ ID NO: 41 and a VL as set forth in SEQ ID NO: 43.

In some embodiments, the antibody or the antigen-binding fragment thereof that specifically binds to CD3 comprises a VH as set forth in SEQ ID NO: 42 and a VL as set forth in SEQ ID NO: 43.

In some embodiments, the antibody or the antigen-binding fragment thereof that specifically binds to CD3 comprises a VH as set forth in SEQ ID NO: 64 and a VL as set forth in SEQ ID NO: 43.

In some embodiments, the antibody or the antigen-binding fragment thereof specifically binds to human and/or cynomolgus monkey CD3.

### MSLN single-domain antibody

In a second aspect, the present disclosure relates to a humanized single-domain antibody or an antigen-binding fragment thereof capable of specifically binding to MSLN. The single-domain antibody is typically composed of four framework regions (FRs) and three complementarity determining regions (CDRs), referred to as FR1, CDR1, FR2, CDR2, FR3, CDR3, and FR4, and the antigen-binding fragment thereof comprises at least a portion of the single-domain antibody that is sufficient to confer the fragment the ability of specifically binding to an antigen (*e.g*., MSLN). The single-domain antibody can be truncated at the N- or C-terminus to comprise only a portion of FR1 and/or FR4, or to lack one or two of those framework regions, so long as it substantially retains antigen binding and specificity. The single-domain antibody can be humanized to form a humanized single-domain antibody, in which one or more of the framework regions in the VHH have been essentially replaced by a human framework region. In some embodiments, the humanized single-domain antibody or the antigen-binding fragment thereof comprises a camelid CDR region and a heavy chain framework region derived from a human immunoglobulin (*e.g.,* a heavy chain framework region comprised in an amino acid sequence encoded by a human heavy chain germline antibody gene), and the heavy chain framework region optionally comprises one or more (*e.g.,* 1, 2, 3, 4, 5, 6, 7, 8, 9, or 10) reverse mutations from a humanized residue to a camelid residue.

In some embodiments, the humanized single-domain antibody or the antigen-binding fragment thereof of the present disclosure comprises the VHH sequence as set forth in any one of SEQ ID NOs: 1-8 or a variant thereof, and the variant has at least 70%, at least 80%, at least 90%, at least 91%, at least 92%, at least 93%, at least 94%, at least 95%, at least 96%, at least 97%, at least 98%, or at least 99% sequence identity compared to the sequence from which it is derived, or has one or more amino acid substitutions, deletions, or additions; preferably, the substitution is a conservative substitution.

In some embodiments, the humanized single-domain antibody or the antigen-binding fragment thereof of the present disclosure comprises CDR1 as set forth in SEQ ID NO: 52, CDR2 as set forth in SEQ ID NO: 53, and CDR3 as set forth in SEQ ID NO: 54, as defined by the IMGT numbering system.

In some embodiments, the single-domain antibody or the antigen-binding fragment thereof specifically binds to human and/or cynomolgus monkey MSLN.

### PD-L1 single-domain antibody

In a third aspect, the present disclosure relates to a single-domain antibody or an antigen-binding fragment thereof capable of specifically binding to PD-L1.

In some embodiments, the single-domain antibody or the antigen-binding fragment thereof comprises the following CDRs as defined by the IMGT numbering system: (1A) CDR1 comprising the sequence as set forth in SEQ ID NO: 11 or 63 or a variant thereof, CDR2 comprising the sequence as set forth in SEQ ID NO: 12 or a variant thereof, and CDR3 comprising the sequence as set forth in SEQ ID NO: 13 or a variant thereof; or (1B) CDR1 comprising the sequence as set forth in SEQ ID NO: 68 or a variant thereof, CDR2 comprising the sequence as set forth in SEQ ID NO: 69 or a variant thereof, and CDR3 comprising the sequence as set forth in SEQ ID NO: 70 or a variant thereof; wherein the variant has one or more amino acid substitutions, deletions, or additions compared to the sequence from which it is derived; preferably, the substitution is a conservative substitution. In certain embodiments, the single-domain antibody or the antigen-binding fragment thereof comprises the following CDRs as defined by the IMGT numbering system: (1A) CDR1 comprising the sequence as set forth in SEQ ID NO: 11 or 63, CDR2 comprising the sequence as set forth in SEQ ID NO: 12, and CDR3 comprising the sequence as set forth in SEQ ID NO: 13; or (1B) CDR1 comprising the sequence as set forth in SEQ ID NO: 68, CDR2 comprising the sequence as set forth in SEQ ID NO: 69, and CDR3 comprising the sequence as set forth in SEQ ID NO: 70.

In some embodiments, the single-domain antibody or the antigen-binding fragment thereof comprises the following CDRs as defined by the Kabat numbering system: (2A) CDR1 comprising the sequence as set forth in SEQ ID NO: 14 or a variant thereof, CDR2 comprising the sequence as set forth in SEQ ID NO: 15 or a variant thereof, and CDR3 comprising the sequence as set forth in SEQ ID NO: 16 or a variant thereof; or (2B) CDR1 comprising the sequence as set forth in SEQ ID NO: 71 or a variant thereof, CDR2 comprising the sequence as set forth in SEQ ID NO: 72 or a variant thereof, and CDR3 comprising the sequence as set forth in SEQ ID NO: 73 or a variant thereof, wherein the variant has one or more amino acid substitutions, deletions, or additions compared to the sequence from which it is derived; preferably, the substitution is a conservative substitution. In some embodiments, the single-domain antibody or the antigen-binding fragment thereof comprises the following CDRs as defined by the Kabat numbering system: CDR1 comprising the sequence as set forth in SEQ ID NO: 14, CDR2 comprising the sequence as set forth in SEQ ID NO: 15, and CDR3 comprising the sequence as set forth in SEQ ID NO: 16; or (2B) CDR1 comprising the sequence as set forth in SEQ ID NO: 71, CDR2 comprising the sequence as set forth in SEQ ID NO: 72, and CDR3 comprising the sequence as set forth in SEQ ID NO: 73.

In some embodiments, the single-domain antibody or the antigen-binding fragment thereof comprises the following CDRs as defined by the AbM numbering system: (3A) CDR1 comprising the sequence as set forth in SEQ ID NO: 17 or a variant thereof, CDR2 comprising the sequence as set forth in SEQ ID NO: 18 or a variant thereof, and CDR3 comprising the sequence as set forth in SEQ ID NO: 16 or a variant thereof; or (3B) CDR1 comprising the sequence as set forth in SEQ ID NO: 74 or a variant thereof, CDR2 comprising the sequence as set forth in SEQ ID NO: 75 or a variant thereof, and CDR3 comprising the sequence as set forth in SEQ ID NO: 73 or a variant thereof; wherein the variant has one or more amino acid substitutions, deletions, or additions compared to the sequence from which it is derived; preferably, the substitution is a conservative substitution. In certain embodiments, the single-domain antibody or the antigen-binding fragment thereof comprises the following CDRs as defined by the AbM numbering system: CDR1 comprising the sequence as set forth in SEQ ID NO: 17, CDR2 comprising the sequence as set forth in SEQ ID NO: 18, and CDR3 comprising the sequence as set forth in SEQ ID NO: 16; or (3B) CDR1 comprising the sequence as set forth in SEQ ID NO: 74, CDR2 comprising the sequence as set forth in SEQ ID NO: 75, and CDR3 comprising the sequence as set forth in SEQ ID NO: 73.

In some embodiments, the single-domain antibody or the antigen-binding fragment thereof comprises the following CDRs as defined by the Chothia numbering system: (4A) CDR1 comprising the sequence as set forth in SEQ ID NO: 19 or a variant thereof, CDR2 comprising the sequence as set forth in SEQ ID NO: 20 or a variant thereof, and CDR3 comprising the sequence as set forth in SEQ ID NO: 16 or a variant thereof; or (4B) CDR1 comprising the sequence as set forth in SEQ ID NO: 76 or a variant thereof, CDR2 comprising the sequence as set forth in SEQ ID NO: 77 or a variant thereof, and CDR3 comprising the sequence as set forth in SEQ ID NO: 73 or a variant thereof; wherein the variant has one or more amino acid substitutions, deletions, or additions compared to the sequence from which it is derived; preferably, the substitution is a conservative substitution. In some embodiments, the single-domain antibody or the antigen-binding fragment thereof comprises the following CDRs as defined by the Chothia numbering system: CDR1 comprising the sequence as set forth in SEQ ID NO: 19, CDR2 comprising the sequence as set forth in SEQ ID NO: 20, and CDR3 comprising the sequence as set forth in SEQ ID NO: 16; or (4B) CDR1 comprising the sequence as set forth in SEQ ID NO: 76, CDR2 comprising the sequence as set forth in SEQ ID NO: 77, and CDR3 comprising the sequence as set forth in SEQ ID NO: 73.

In some embodiments, the single-domain antibody or the antigen-binding fragment thereof comprises the following CDRs as defined by the Contact numbering system: (5A) CDR1 comprising the sequence as set forth in SEQ ID NO: 21 or a variant thereof, CDR2 comprising the sequence as set forth in SEQ ID NO: 22 or a variant thereof, and CDR3 comprising the sequence as set forth in SEQ ID NO: 23 or a variant thereof; or (5B) CDR1 comprising the sequence as set forth in SEQ ID NO: 78 or a variant thereof, CDR2 comprising the sequence as set forth in SEQ ID NO: 79 or a variant thereof, and CDR3 comprising the sequence as set forth in SEQ ID NO: 80 or a variant thereof; wherein the variant has one or more amino acid substitutions, deletions, or additions compared to the sequence from which it is derived; preferably, the substitution is a conservative substitution. In some embodiments, the single-domain antibody or the antigen-binding fragment thereof comprises the following CDRs as defined by the Contact numbering system: CDR1 comprising the sequence as set forth in SEQ ID NO: 21, CDR2 comprising the sequence as set forth in SEQ ID NO: 22, and CDR3 comprising the sequence as set forth in SEQ ID NO: 23; or (5B) CDR1 comprising the sequence as set forth in SEQ ID NO: 78, CDR2 comprising the sequence as set forth in SEQ ID NO: 79, and CDR3 comprising the sequence as set forth in SEQ ID NO: 80.

In some embodiments, the single-domain antibody or the antigen-binding fragment thereof comprises CDR1, CDR2, and CDR3 comprised in the VHH as set forth in any one of SEQ ID NOs: 10, 24-39, 67, and 81-88. In some embodiments, the CDRs are determined by IMGT, Kabat, AbM, Chothia, or Contact numbering systems.

In some embodiments, the single-domain antibody or the antigen-binding fragment thereof comprises the VHH sequence as set forth in SEQ ID NO: 10 or 67 or a variant thereof, and the variant has at least 70%, at least 80%, at least 85%, at least 90%, at least 91%, at least 92%, at least 93%, at least 94%, at least 95%, at least 96%, at least 97%, at least 98%, or at least 99% sequence identity compared to the sequence from which it is derived, or has one or more amino acid substitutions, deletions, or additions compared to it; preferably, the substitution is a conservative substitution.

In some embodiments, the single-domain antibody or the antigen-binding fragment thereof is humanized, in which one or more framework regions in its VHH have been substantially replaced with human framework regions. In some embodiments, the humanized single-domain antibody or the antigen-binding fragment thereof comprises a heavy chain framework region derived from a human immunoglobulin (*e.g.,* a heavy chain framework region comprised in an amino acid sequence encoded by a human heavy chain germline antibody gene), and the heavy chain framework region optionally comprises one or more (*e.g.,* 1, 2, 3, 4, 5, 6, 7, 8, 9, or 10) reverse mutations from a humanized residue to a camelid residue.

In some embodiments, the humanized single-domain antibody or the antigen-binding fragment thereof comprises the VHH sequence as set forth in any one of SEQ ID NOs: 24-39 and 81-88 or a variant thereof, and the variant has at least 70%, at least 80%, at least 85%, at least 90%, at least 91%, at least 92%, at least 93%, at least 94%, at least 95%, at least 96%, at least 97%, at least 98%, or at least 99% sequence identity compared to the sequence from which it is derived, or has one or more amino acid substitutions, deletions, or additions compared to it; preferably, the substitution is a conservative substitution.

In some embodiments, the single-domain antibody or the antigen-binding fragment thereof specifically binds to human PD-L1, mouse PD-L1, and/or cynomolgus monkey PD-L1, for example, the single-domain antibody is as defined in (1A) to (5A) in the above embodiments. In some embodiments, the single-domain antibody or the antigen-binding fragment thereof specifically binds to human PD-L1 and/or cynomolgus monkey PD-L1, for example, the single-domain antibody is as defined in (1B) to (5B) in the above embodiments.

### Polypeptide construct

In a fourth aspect, the present disclosure relates to a polypeptide construct comprising the single-domain antibody or the antigen-binding fragment thereof as described in any one of the above aspects, and an immunoglobulin Fc domain.

In some embodiments, the polypeptide construct comprises the single-domain antibody that specifically binds to MSLN as described in the second aspect, and an immunoglobulin Fc domain.

In some embodiments, the polypeptide construct comprises the single-domain antibody that specifically binds to PD-L1 as described in the third aspect, and an immunoglobulin Fc domain.

In this context, the Fc domain, also known as Fc region, refers to a portion of the heavy chain constant region comprising CH2 and CH3. In certain embodiments, the Fc domain comprises a hinge, CH2, and CH3. When the Fc domain comprises a hinge, the hinge regulates the dimerization between two Fc-containing polypeptides. The Fc domain can be an isotype of any antibody heavy chain constant region. In some embodiments, the Fc domain is IgG1, IgG2, IgG3, or IgG4.

In some embodiments, the Fc domain comprised in the polypeptide construct of the present disclosure is a native Fc region comprising an amino acid sequence consistent with the amino acid sequence of the Fc region found in nature. For example, the Fc domain can be a native sequence human IgG1 Fc region, a native sequence human IgG2 Fc region, a native sequence human IgG3 Fc region, or a native sequence human IgG4 Fc region. The native Fc region can have effector functions. Exemplary "effector function" comprises Fc receptor binding; Clq binding and complement-dependent cytotoxicity (CDC); antibody-dependent cell-mediated cytotoxicity (ADCC); phagocytosis; down-regulation of cell surface receptors, such as B cell receptors; B cell activation, *etc.* Functional alteration can be produced by replacing at least one amino acid residue in the native Fc region with a different residue or by chemical modification, *e.g.,* altering the affinity of the antibody for an effector ligand *(e.g.,* FcR or complement C1q), thus altering effector function (*e.g.,* reducing or enhancing).

In some embodiments, the Fc domain comprised in the polypeptide construct of the present disclosure can also be a variant Fc region, which can comprise one or more (*e.g.,* 1-10, *e.g.,* 1-5) amino acid mutations or chemical modifications compared to the native Fc region to alter one or more of the following characteristics of the antibody of the present disclosure: Fc receptor binding, antibody glycosylation, number of cysteine residues, effector cell function, complement function, *etc.*

In some embodiments, the immunoglobulin Fc domain is optionally linked to the N-terminus and/or C-terminus (*e.g.,* C-terminus) of the single-domain antibody or the antigen-binding fragment thereof via a peptide linker.

In some embodiments, the immunoglobulin Fc domain is optionally linked to the C-terminus of the single-domain antibody or the antigen-binding fragment thereof via a peptide linker.

In some embodiments, the immunoglobulin Fc domain is an Fc domain of IgG (*e.g.,* an Fc domain of IgG1).

In some embodiments, the immunoglobulin Fc domain comprises a sequence as set forth in SEQ ID NO: 48, or a sequence having at least 70%, at least 80%, at least 85%, at least 90%, at least 91%, at least 92%, at least 93%, at least 94%, at least 95%, at least 96%, at least 97%, at least 98%, or at least 99% sequence identity compared to SEQ ID NO: 48, or a sequence having one or more amino acid substitutions, deletions, or additions (*e.g.,* 1, 2, 3, 4, or 5 amino acid substitutions, deletions, or additions) compared to SEQ ID NO: 48.

In some embodiments, the peptide linker is a peptide linker comprising one or more glycines and/or one or more serines. In some embodiments, the peptide linker is (G4S)n, n being 1, 2, 3, or 4. In some embodiments, the peptide linker comprises the sequence as set forth in SEQ ID NO: 50.

### Multispecific antibodies

In a fifth aspect, the present disclosure relates to a multispecific antibody comprising the humanized single-domain antibody or the antigen-binding fragment thereof targeting MSLN as described in the second aspect and/or the single-domain antibody or the antigen-binding fragment thereof targeting PD-L1 as described in the third aspect.

In some embodiments, the multispecific antibody is a bispecific antibody, a trispecific antibody, or a tetraspecific antibody.

In some embodiments, the multispecific antibody comprises the humanized single-domain antibody or the antigen-binding fragment thereof targeting MSLN as described in the second aspect as an antigen-binding domain targeting MSLN, and at least one additional antigen-binding domain that binds to a different target than the antigen-binding domain.

In some embodiments, the multispecific antibody comprises the single-domain antibody or the antigen-binding fragment thereof targeting PD-L1 as described in the third aspect as an antigen-binding domain targeting PD-L1, and at least one additional antigen-binding domain that binds to a different target than the antigen-binding domain.

In some embodiments, the multispecific antibody comprises the humanized single-domain antibody or the antigen-binding fragment thereof targeting MSLN as described in the second aspect as an antigen-binding domain targeting MSLN, the single-domain antibody or the antigen-binding fragment thereof targeting PD-L1 as described in the third aspect as an antigen-binding domain targeting PD-L1, and further comprises at least one additional antigen-binding domain that binds to a different target than the antigen-binding domain.

In a sixth aspect, the present disclosure relates to a multispecific antibody comprising a first antigen-binding domain targeting CD3, a second antigen-binding domain targeting MSLN, and a third antigen-binding domain targeting PD-L1, wherein
(i) the first antigen-binding domain comprises the antibody or the antigen-binding fragment thereof that specifically binds to CD3 as described in the first aspect;
(ii) the second antigen-binding domain comprises a VHH, wherein the VHH comprises CDR1, CDR2, and CDR3 comprised in the VHH as set forth in any one of SEQ ID NOs: 1-9; preferably, the VHH comprises CDR1 as set forth in SEQ ID NO: 52, CDR2 as set forth in SEQ ID NO: 53, and CDR3 as set forth in SEQ ID NO: 54; preferably, the second antigen-binding domain comprises the humanized single-domain antibody or the antigen-binding fragment thereof that specifically binds to MSLN as described in the second aspect or comprises the VHH sequence as set forth in SEQ ID NO: 9;
   and/or,
(iii) the third antigen-binding domain comprises the single-domain antibody or the antigen-binding fragment thereof that specifically binds to PD-L1 as described in the third aspect.

In some embodiments, in (i), the first antigen-binding domain comprises a VH and a VL, wherein the VH comprises HCDR1 as set forth in SEQ ID NO: 55, HCDR2 as set forth in SEQ ID NO: 56, and HCDR3 as set forth in any one of SEQ ID NOs: 57-59 and 65; and/or, the VL comprises LCDR1 as set forth in SEQ ID NO: 60, LCDR2 as set forth in SEQ ID NO: 61, and LCDR3 as set forth in SEQ ID NO: 62; preferably, the VH comprises the sequence as set forth in any one of SEQ ID NOs: 40-42 and 64; preferably, the VL comprises the sequence as set forth in SEQ ID NO: 43.

In some embodiments, the first antigen-binding domain comprises the antibody or the antigen-binding fragment thereof that specifically binds to CD3 as described in the first aspect; the second antigen-binding domain comprises an antibody or an antigen-binding fragment thereof that specifically binds to MSLN, *e.g.,* a full-length antibody, Fv fragment, Fab fragment, F(ab')₂ fragment, scFv, or VHH; the third antigen-binding domain comprises an antibody or an antigen-binding fragment thereof that specifically binds to PD-L1, *e.g.,* a full-length antibody, Fv fragment, Fab fragment, F(ab')₂ fragment, scFv, or VHH.

In some embodiments, the first antigen-binding domain comprises an antibody or an antigen-binding fragment thereof that specifically binds to CD3, *e.g.,* a full-length antibody, Fv fragment, Fab fragment, F(ab')₂ fragment, scFv, or VHH; the second antigen-binding domain comprises the humanized single-domain antibody or the antigen-binding fragment thereof that specifically binds to MSLN as described in the second aspect; the third antigen-binding domain comprises an antibody or an antigen-binding fragment thereof that specifically binds to PD-L1, *e.g.,* a full-length antibody, Fv fragment, Fab fragment, F(ab')₂ fragment, scFv, or VHH.

In some embodiments, the first antigen-binding domain comprises an antibody or an antigen-binding fragment thereof that specifically binds to CD3, *e.g.,* a full-length antibody, Fv fragment, Fab fragment, F(ab')₂ fragment, scFv, or VHH; the second antigen-binding domain comprises an antibody or an antigen-binding fragment thereof that specifically binds to MSLN, *e.g.,* a full-length antibody, Fv fragment, Fab fragment, F(ab')₂ fragment, scFv, or VHH; the third antigen-binding domain comprises the single-domain antibody or the antigen-binding fragment thereof that specifically binds to PD-L1 as described in the third aspect.

In some embodiments, the first antigen-binding domain comprises the antibody or the antigen-binding fragment thereof that specifically binds to CD3 as described in the first aspect; the second antigen-binding domain comprises the humanized single-domain antibody or the antigen-binding fragment thereof that specifically binds to MSLN as described in the second aspect; the third antigen-binding domain comprises an antibody or an antigen-binding fragment thereof that specifically binds to PD-L1, *e.g.,* a full-length antibody, Fv fragment, Fab fragment, F(ab')₂ fragment, scFv, or VHH.

In some embodiments, the first antigen-binding domain comprises the antibody or the antigen-binding fragment thereof that specifically binds to CD3 as described in the first aspect; the second antigen-binding domain comprises an antibody or an antigen-binding fragment thereof that specifically binds to MSLN, *e.g.,* a full-length antibody, Fv fragment, Fab fragment, F(ab')₂ fragment, scFv, or VHH; the third antigen-binding domain comprises the single-domain antibody or the antigen-binding fragment thereof that specifically binds to PD-L1 as described in the third aspect.

In some embodiments, the first antigen-binding domain comprises an antibody or an antigen-binding fragment thereof that specifically binds to CD3, *e.g.,* a full-length antibody, Fv fragment, Fab fragment, F(ab')₂ fragment, scFv, or VHH; the second antigen-binding domain comprises the humanized single-domain antibody or the antigen-binding fragment thereof that specifically binds to MSLN as described in the second aspect; the third antigen-binding domain comprises the single-domain antibody or the antigen-binding fragment thereof that specifically binds to PD-L1 as described in the third aspect.

In some embodiments, the first antigen-binding domain comprises the antibody or the antigen-binding fragment thereof that specifically binds to CD3 as described in the first aspect; the second antigen-binding domain comprises the humanized single-domain antibody or the antigen-binding fragment thereof that specifically binds to MSLN as described in the second aspect; the third antigen-binding domain comprises the single-domain antibody or the antigen-binding fragment thereof that specifically binds to PD-L1 as described in the third aspect.

In a seventh aspect, the present disclosure relates to a multispecific antibody comprising: a first antigen-binding domain targeting CD3, a second antigen-binding domain targeting a tumor-associated antigen (TAA), and a third antigen-binding domain targeting an immune checkpoint, wherein the first antigen-binding domain is Fab, and the second and third antigen-binding domains are VHH.

In certain embodiments, the multispecific antibody further comprises an Fc domain comprising a first Fc monomer and a second Fc monomer; wherein:
the N-terminus of the first Fc monomer is optionally linked to the first antigen-binding domain (*e.g.,* the heavy chain CH1 domain thereof) via a linker, and the C-terminal thereof is optionally linked to the second antigen-binding domain via a linker;
the N-terminus of the second Fc monomer is optionally linked to another second antigen-binding domain via a linker, and the C-terminal thereof is optionally linked to the third antigen-binding domain via a linker.

In some embodiments, the tumor-associated antigen is selected from CD19, BCMA, EGFR, HER2, HER3, HER4, PSMA, EpCAM, EphA2, CD33, CD123, CD38, CLDN18, MSLN, TROP2, Mucin1, AFP, CD79b, GUCY2C, LRRC15, gp100, STEAP1, ROR1, 5T4, CEA, DLL3, CD20, CD7, PRAME, CDH19, CDH17, GPA33, HLA-A2, CD34, FAP, GPRC5D, GPC3, B7-H3, CLL-1, CLDN6, Flt3, NY-ESO-1, PSCA, NECTIN-4, ENPP3, IGFR-1, TSA1, Melan-A, MUC16 (CA125), MUC17, SSTR2, c-Met, B7-H6, CSPG4, CAIX, MCSP, BIRC5, BIRC7, BRCA1, BORIS, CCR5, GD2, GD3, GloboH, GM3, hTERT, LMP2, p53, PAP, PAX3, PAX5, PCTA-1, PLAC1, PRLR, Ras, SART-3, TRP-1, TRP-2, CD22, CD30, FOLR1, or any combination thereof.

In some embodiments, the tumor-associated antigen is MSLN.

In some embodiments, the immune checkpoint is selected from PD-1, PD-L1, PD-L2 CTLA-4, TIM-3, Lag-3, TIGIT, CD73, VISTA, B7-H3, NKG2D, NKG2A, OX40, OX40L, CD40, CD47, LIGHT, ICOS, HVEM, BTLA, B7-H4, 4-1BB, 4-1BBL, or any combination thereof.

In some embodiments, the immune checkpoint is selected from PD1, PD-L1, TIGIT, or LAG3.

In some embodiments, the immune checkpoint is selected from PD1, PD-L1, or a combination thereof. In some embodiments, the immune checkpoint is PD-L1.

In some embodiments, the multispecific antibody comprises an Fc domain comprising modifications to promote dimerization of the first Fc monomer and the second Fc monomer. Thus, (hetero)dimerization occurs between the polypeptide comprising the first Fc monomer and the polypeptide comprising the second Fc monomer, forming a complex.

Such modifications are known to those skilled in the art and can comprise separately modifying each of the two Fc subunits (*i.e.,* the first and second monomers of Fc) that are desired to be associated, where the modifications are complementary to each other, thereby facilitating the association of the two Fc subunits. For example, modifications that promote association can alter the structure or charge of one or both Fc subunits to promote their association sterically or electrostatically, respectively. For example, modifications that promote association comprise amino acid mutations (e.g., amino acid substitutions) in Fc. In some embodiments, the modification is in the CH3 domain of Fc.

In some embodiments, the CH3 domains of the two monomers of the Fc domain comprise amino acid substitutions.

In some embodiments, the modification comprises a "knob" modification in one of the two monomers of the Fc domain and a "hole" modification in the other of the two monomers of the Fc domain to form a "knob-into-hole" modification. Typically, the method involves introducing a protuberance ("knob") at the interface of the first polypeptide and a corresponding cavity ("hole") at the interface of the second polypeptide such that the protuberance can be placed in the cavity to promote heterodimer formation and hinder homodimer formation. The protuberance is constructed by replacing the small amino acid side chain from the first polypeptide interface with a larger side chain (*e.g.,* tyrosine or tryptophan). Complementary cavities of the same or similar size as the protuberances are created in the interface of the second polypeptide by replacing large amino acid side chains with smaller amino acid side chains (*e.g.,* alanine or threonine).

In some exemplary embodiments, one amino acid in the CH3 domain of the first Fc monomer is substituted with an amino acid residue having a larger side chain volume, thereby forming a protuberance within the CH3 domain of the first Fc monomer, one amino acid in the CH3 domain of the second Fc monomer is substituted with an amino acid residue having a smaller side chain volume, thereby forming a complementary cavity within the CH3 domain of the second Fc monomer having the same or similar size as the protuberance; or one amino acid in the CH3 domain of the second Fc monomer is substituted with an amino acid residue having a larger side chain volume, thereby forming a protuberance within the CH3 domain of the second Fc monomer, one amino acid in the CH3 domain of the first Fc monomer is substituted with an amino acid residue having a smaller side chain volume, thereby forming a complementary cavity within the CH3 domain of the first Fc monomer having the same or similar size as the protuberance.

In some embodiments, the amino acid residue having a larger side chain volume is selected from tryptophan (W), arginine (R), phenylalanine (P), and tyrosine (Y).

In some embodiments, the amino acid residue having a smaller side chain volume is selected from valine (V), alanine (A), serine (S), and threonine (T).

In some exemplary embodiments, the first Fc monomer and the second Fc monomer of the Fc domain comprise the amino acid sequences as set forth in SEQ ID NOs: 46 and 47, respectively.

In some embodiments, the linker is selected from peptide linkers comprising one or more glycines and/or one or more serines, *e.g*., comprising (G4S)n, n being 1, 2, 3, or 4, *e.g.,* comprising the sequence as set forth in any one of SEQ ID NOs: 49-51.

In some embodiments, the multispecific antibody comprises:
(i) a first peptide chain comprising a VL and a light chain constant region (CL) of the first antigen-binding domain; preferably, the CL is a kappa light chain constant region;
(ii) a second peptide chain comprising a VH, CH1, and a first Fc monomer of the first antigen-binding domain and the second antigen-binding domain; preferably, the first Fc monomer is IgG, *e.g.,* IgG1 or IgG4; preferably, the first Fc monomer comprises a hinge region, CH2, and CH3; preferably, the second antigen-binding domain is linked to the C-terminus of the first Fc monomer by a linker (such as a flexible peptide comprising (G4S)n);
   and
(iii) a third peptide chain comprising the second antigen-binding domain, the second Fc monomer, and the third antigen-binding domain; preferably, the second Fc monomer is IgG, *e.g*., IgG1 or IgG4; preferably, the second Fc monomer comprises a hinge region, CH2, and CH3; preferably, the second antigen-binding domain is linked to the N-terminus of the second Fc monomer by a linker (such as a flexible peptide comprising (G4S)n); preferably, the third antigen-binding domain is linked to the C-terminus of the second Fc monomer by a linker (such as a flexible peptide comprising (G4S)n).

In some embodiments, the second Fc monomer of the third peptide chain is capable of forming a dimer with the first Fc monomer of the second peptide chain.

In some embodiments, the first Fc monomer of the second peptide chain and the second Fc monomer of the third peptide chain comprise modifications to promote dimerization. Preferably, the modification comprises an amino acid substitution in the CH3 domain of the Fc domain. Preferably, the modification comprises a "knob" modification in one of the two Fc monomers and a "hole" modification in the other of the two Fc monomers to form a "knob-into-hole" modification.

In some embodiments, the two Fc monomers comprise the amino acid sequences as set forth in SEQ ID NOs: 46 and 47, respectively. In some embodiments, the first Fc monomer of the second peptide chain comprises the amino acid sequence as set forth in SEQ ID NO: 46. In some embodiments, the second peptide chain comprises the heavy chain constant region sequence as set forth in SEQ ID NO: 45. In some embodiments, the second Fc monomer of the third peptide chain comprises the amino acid sequence as set forth in SEQ ID NO: 47.

In some embodiments, the first antigen-binding domain comprises a VH and a VL, wherein the VH comprises HCDR1 as set forth in SEQ ID NO: 55, HCDR2 as set forth in SEQ ID NO: 56, and HCDR3 as set forth in any one of SEQ ID NOs: 57-59 and 65; and/or, the VL comprises LCDR1 as set forth in SEQ ID NO: 60, LCDR2 as set forth in SEQ ID NO: 61, and LCDR3 as set forth in SEQ ID NO: 62.

In some embodiments, the VH comprises the sequence as set forth in any one of SEQ ID NOs: 40-42 and 64.

In some embodiments, the VL comprises the sequence as set forth in SEQ ID NO: 43.

In some embodiments, the second antigen-binding domain comprises a VHH, wherein the VHH comprises CDR1, CDR2, and CDR3 comprised in the VHH as set forth in any one of SEQ ID NOs: 1-9; preferably, the VHH comprises CDR1 as set forth in SEQ ID NO: 52, CDR2 as set forth in SEQ ID NO: 53, and CDR3 as set forth in SEQ ID NO: 54.

In some embodiments, the second antigen-binding domain comprises the VHH as set forth in any one of SEQ ID NOs: 1-9 or a variant thereof, and the variant has at least 90%, at least 91%, at least 92%, at least 93%, at least 94%, at least 95%, at least 96%, at least 97%, at least 98%, or at least 99% sequence identity compared to the sequence from which it is derived, or has one or more amino acid substitutions, deletions, or additions compared to it; preferably, the substitution is a conservative substitution.

In some embodiments, the third antigen-binding domain comprises the single-domain antibody or the antigen-binding fragment thereof capable of specifically binding to PD-L1 as described in the third aspect.

In some embodiments, the multispecific antibody comprises:
(i) a first peptide chain having a structure shown in [VL]-[CL],
(ii) a second peptide chain having a structure shown in [VH]-[CH]-[L1]-[VHH1], and
(iii) a third peptide chain having a structure shown in [VHH1]-[L2]-[Fc monomer]-[L3]-[VHH2];
   wherein the multispecific antibody is selected from any one of the following:
   (1) the VL comprises the sequence as set forth in SEQ ID NO: 43, and the CL comprises the sequence as set forth in SEQ ID NO: 44; the VH comprises the sequence as set forth in SEQ ID NO: 40, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 81; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
   (2) the VL comprises the sequence as set forth in SEQ ID NO: 43, and the CL comprises the sequence as set forth in SEQ ID NO: 44; the VH comprises the sequence as set forth in SEQ ID NO: 40, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 29; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51;
   (3) the VL comprises the sequence as set forth in SEQ ID NO: 43, and the CL comprises the sequence as set forth in SEQ ID NO: 44; the VH comprises the sequence as set forth in SEQ ID NO: 41, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 29; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51;
   (4) the VL comprises the sequence as set forth in SEQ ID NO: 43, and the CL comprises the sequence as set forth in SEQ ID NO: 44; the VH comprises the sequence as set forth in SEQ ID NO: 42, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 29; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51;
   (5) the VL comprises the sequence as set forth in SEQ ID NO: 43, and the CL comprises the sequence as set forth in SEQ ID NO: 44; the VH comprises the sequence as set forth in SEQ ID NO: 40, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 30; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51;
   (6) the VL comprises the sequence as set forth in SEQ ID NO: 43, and the CL comprises the sequence as set forth in SEQ ID NO: 44; the VH comprises the sequence as set forth in SEQ ID NO: 41, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 30; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51;
   (7) the VL comprises the sequence as set forth in SEQ ID NO: 43, and the CL comprises the sequence as set forth in SEQ ID NO: 44; the VH comprises the sequence as set forth in SEQ ID NO: 42, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 30; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51;
   (8) the VL comprises the sequence as set forth in SEQ ID NO: 43, and the CL comprises the sequence as set forth in SEQ ID NO: 44; the VH comprises the sequence as set forth in SEQ ID NO: 40, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 38; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51;
   (9) the VL comprises the sequence as set forth in SEQ ID NO: 43, and the CL comprises the sequence as set forth in SEQ ID NO: 44; the VH comprises the sequence as set forth in SEQ ID NO: 41, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 38; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51;
   (10) the VL comprises the sequence as set forth in SEQ ID NO: 43, and the CL comprises the sequence as set forth in SEQ ID NO: 44; the VH comprises the sequence as set forth in SEQ ID NO: 42, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 38; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51;
   (11) the VL comprises the sequence as set forth in SEQ ID NO: 43, and the CL comprises the sequence as set forth in SEQ ID NO: 44; the VH comprises the sequence as set forth in SEQ ID NO: 40, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 31; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51;
   (12) the VL comprises the sequence as set forth in SEQ ID NO: 43, and the CL comprises the sequence as set forth in SEQ ID NO: 44; the VH comprises the sequence as set forth in SEQ ID NO: 41, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 31; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51;
   (13) the VL comprises the sequence as set forth in SEQ ID NO: 43, and the CL comprises the sequence as set forth in SEQ ID NO: 44; the VH comprises the sequence as set forth in SEQ ID NO: 42, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 31; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51;
   (14) the VL comprises the sequence as set forth in SEQ ID NO: 43, and the CL comprises the sequence as set forth in SEQ ID NO: 44; the VH comprises the sequence as set forth in SEQ ID NO: 40, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 39; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51;
   (15) the VL comprises the sequence as set forth in SEQ ID NO: 43, and the CL comprises the sequence as set forth in SEQ ID NO: 44; the VH comprises the sequence as set forth in SEQ ID NO: 41, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 39; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51;
   (16) the VL comprises the sequence as set forth in SEQ ID NO: 43, and the CL comprises the sequence as set forth in SEQ ID NO: 44; the VH comprises the sequence as set forth in SEQ ID NO: 42, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 39; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51;
   (17) the VL comprises the sequence as set forth in SEQ ID NO: 43, and the CL comprises the sequence as set forth in SEQ ID NO: 44; the VH comprises the sequence as set forth in SEQ ID NO: 64, the CH comprises the sequence as set forth in SEQ ID NO: 45, and the VHH1 comprises the sequence as set forth in SEQ ID NO: 9; the Fc monomer comprises the sequence as set forth in SEQ ID NO: 47, and the VHH2 comprises the sequence as set forth in SEQ ID NO: 10; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51; or,
   (18) the VL comprises the sequence as set forth in SEQ ID NO: 43, and the CL comprises the sequence as set forth in SEQ ID NO: 44; the VH comprises the sequence as set forth in SEQ ID NO: 40, the CH comprises the sequence as set forth in SEQ ID NO: 45, and the VHH1 comprises the sequence as set forth in SEQ ID NO: 9; the Fc monomer comprises the sequence as set forth in SEQ ID NO: 47, and the VHH2 comprises the sequence as set forth in SEQ ID NO: 10; L1, L2, and L3 are peptide linkers, preferably each independently selected from peptide linkers comprising one or more glycines and/or one or more serines (such as peptide linkers shown in (G4S)n), preferably L1 is the same as L3, preferably, L1 is SEQ ID NO: 51, L2 is SEQ ID NO: 49, and L3 is SEQ ID NO: 51.

### Preparation of antibody

The antibody, single-domain antibody, polypeptide construct, or multispecific antibody as described in any one of the above aspects can be prepared by various methods known in the art, such as by genetic engineering recombinant technology. For example, the DNA molecules encoding them are obtained by chemical synthesis or PCR amplification. The resulting DNA molecules are inserted into the expression vector and then transfected into host cells. Then, the transfected host cells are cultured under specific conditions, and the antibody, single-domain antibody, polypeptide construct, or multispecific antibody of the present disclosure is expressed.

In another aspect, the present disclosure provides an isolated nucleic acid molecule encoding:
- the antibody or the antigen-binding fragment thereof as described in the first aspect, or a heavy chain variable region thereof and/or a light chain variable region thereof;
- the humanized single-domain antibody or the antigen-binding fragment thereof as described in the second aspect;
- the single-domain antibody or the antigen-binding fragment thereof as described in the third aspect;
- the polypeptide construct as described in the fourth aspect;
- the multispecific antibody as described in the fifth, sixth, or seventh aspect, or a polypeptide chain thereof.

In another aspect, the present disclosure provides a vector comprising the nucleic acid molecule as described above. In certain embodiments, the vector is a cloning vector or an expression vector.

In certain embodiments, the vector comprises a nucleotide sequence encoding each peptide chain of the multispecific antibody of the present disclosure, and the nucleotide sequences encoding each peptide chain are present on the same or on different vectors.

In another aspect, the present disclosure provides a host cell comprising the nucleic acid molecule or the vector as described above. Such host cells include, but are not limited to, prokaryotic cells such as bacterial cells (*e.g*., Escherichia coli cells), eukaryotic cells such as fungal cells (*e.g.,* yeast cells), insect cells, plant cells, and animal cells (*e.g.,* mammalian cells, *e.g*., mouse cells and human cells).

In another aspect, the present disclosure provides a method for preparing the antibody, single-domain antibody, polypeptide construct, or multispecific antibody as described in any one of the above aspects, wherein the method comprises: culturing the host cell as described above under conditions allowing protein expression, and collecting the antibody, single-domain antibody, polypeptide construct, or multispecific antibody from the cultured host cell culture.

### Conjugate

As one of the derivatives of antibodies, the present disclosure provides a conjugate comprising the antibody, single-domain antibody, polypeptide construct, or multispecific antibody as described in any of the above aspects and a coupling moiety.

In certain embodiments, the coupling moiety is selected from a detectable label, such as a radioisotope, a fluorescent substance, a luminescent substance, a colored substance, or an enzyme.

In certain embodiments, the coupling moiety is selected from a therapeutic agent. In certain embodiments, the therapeutic agent is preferably an anti-tumor agent, such as cytotoxic agents, cytokines, toxins, radionuclides, immune agonists, immunosuppressants, and other active substances that inhibit the growth of tumor cells and promote apoptosis or necrosis of tumor cells.

### Pharmaceutical composition

The antibodies, single-domain antibodies, polypeptide constructs, multispecific antibodies, or conjugates (also known as active ingredients) disclosed herein can be incorporated into pharmaceutical compositions suitable for administration.

In another aspect, the present disclosure relates to a pharmaceutical composition comprising the antibody, the single-domain antibody, the polypeptide construct, the multispecific antibody, the isolated nucleic acid molecule, the vector, the host cell, or the conjugate as described in any one of the above aspects, and a pharmaceutically acceptable carrier and/or excipient.

In certain embodiments, the pharmaceutical composition of the present disclosure comprises the humanized single-domain antibody or the antigen-binding fragment thereof as described in the second aspect or the nucleic acid molecule or the vector encoding them.

In certain embodiments, the pharmaceutical composition of the present disclosure comprises the single-domain antibody or the antigen-binding fragment thereof as described in the third aspect or the nucleic acid molecule or the vector encoding them.

In certain embodiments, the pharmaceutical composition of the present disclosure comprises the polypeptide construct as described in the fourth aspect or the nucleic acid molecule or the vector encoding them.

In certain embodiments, the pharmaceutical composition of the present disclosure comprises the multispecific antibody as described in the fifth aspect or the nucleic acid molecule or the vector encoding them.

In certain embodiments, the pharmaceutical composition of the present disclosure comprises the multispecific antibody as described in the sixth aspect or the nucleic acid molecule or the vector encoding them.

In certain embodiments, the pharmaceutical composition of the present disclosure comprises the multispecific antibody as described in the seventh aspect or the nucleic acid molecule or the vector encoding them.

In certain embodiments, the pharmaceutical composition can further comprise an additional pharmaceutically active agent, such as an anti-tumor agent. In certain embodiments, the additional pharmaceutically active agent is provided separately from the antibody, the single-domain antibody, the polypeptide construct, the multispecific antibody, the isolated nucleic acid molecule, the vector, or the host cell of the present disclosure, or is provided as a component of the same composition.

### Therapeutic use

In another aspect, the present disclosure relates to a method for the prevention and/or treatment and/or neoadjuvant treatment and/or adjuvant treatment of a disease, comprising administering to a subject in need thereof the antibody, the single-domain antibody, the polypeptide construct, the multispecific antibody, the isolated nucleic acid molecule, the vector, the host cell, the conjugate, or the pharmaceutical composition as described in any one of the above aspects. The present disclosure further relates to the antibody, the single-domain antibody, the polypeptide construct, the multispecific antibody, the isolated nucleic acid molecule, the vector, the host cell, the conjugate, or the pharmaceutical composition as described in any one of the above aspects for use in the prevention and/or treatment and/or neoadjuvant treatment and/or adjuvant treatment of a disease, or a use of the antibody, the single-domain antibody, the polypeptide construct, the multispecific antibody, the isolated nucleic acid molecule, the vector, the host cell, the conjugate, or the pharmaceutical composition as described in any one of the above aspects in the preparation of a medicament for the prevention and/or treatment and/or neoadjuvant treatment and/or adjuvant treatment of a disease.

In certain embodiments, the disease is a tumor. In certain embodiments, the tumor is a solid tumor or a hematological tumor. In certain embodiments, the solid tumor is selected from mesothelioma, ovarian cancer, pancreatic cancer, cervical cancer, breast cancer, prostate cancer, cholangiocarcinoma, colon cancer, gastric cancer, fallopian tube cancer, lung cancer, or colorectal cancer; the hematological tumor comprises acute myeloid leukemia.

In another aspect, the present disclosure relates to a method for the prevention and/or treatment and/or neoadjuvant treatment and/or adjuvant treatment of a disease, comprising administering to a subject in need thereof the humanized single-domain antibodies as described in the second aspect, a polypeptide construct or a multispecific antibody or a conjugate comprising them, a nucleic acid molecule or a vector or a host cell encoding them, or a pharmaceutical composition comprising any one of the foregoing. The present disclosure further relates to the humanized single-domain antibodies as described in the second aspect, a polypeptide construct or a multispecific antibody or a conjugate comprising them, a nucleic acid molecule or a vector or a host cell encoding them, or a pharmaceutical composition comprising any one of the foregoing for use in the prevention and/or treatment and/or neoadjuvant treatment and/or adjuvant treatment of a disease, or a use of the humanized single-domain antibodies as described in the second aspect, a polypeptide construct or a multispecific antibody or a conjugate comprising them, a nucleic acid molecule or a vector or a host cell encoding them, or a pharmaceutical composition comprising any one of the foregoing in the preparation of a medicament for the prevention and/or treatment and/or neoadjuvant treatment and/or adjuvant treatment of a disease.

In certain embodiments, the disease is a tumor.

In certain embodiments, the tumor is an MSLN positive tumor.

In certain embodiments, the tumor is a solid tumor or a hematological tumor, such as gastric cancer, lung cancer, ovarian cancer, esophageal cancer, pancreatic cancer, cervical cancer, mesothelioma, breast cancer, prostate cancer, bladder cancer, ovarian cancer, colorectal cancer, head and neck squamous cell carcinoma, pancreatic cancer, cholangiocarcinoma, colon cancer, fallopian tube cancer, malignant melanoma, soft tissue cancer, chronic lymphocytic leukemia, acute myeloid leukemia, or acute lymphocytic leukemia.

In certain embodiments, the tumor is selected from a solid tumor, such as mesothelioma, ovarian cancer, pancreatic cancer, cervical cancer, breast cancer, prostate cancer, cholangiocarcinoma, colon cancer, gastric cancer, fallopian tube cancer, lung cancer, or colorectal cancer.

In certain embodiments, the tumor is selected from a hematological tumor, such as acute myeloid leukemia.

In another aspect, the present disclosure relates to a method for the prevention and/or treatment and/or neoadjuvant treatment and/or adjuvant treatment of a disease, comprising administering to a subject in need thereof the single-domain antibodies as described in the third aspect, a polypeptide construct or a multispecific antibody or a conjugate comprising them, a nucleic acid molecule or a vector or a host cell encoding them, or a pharmaceutical composition comprising any one of the foregoing. The present disclosure further relates to the single-domain antibodies as described in the third aspect, a polypeptide construct or a multispecific antibody or a conjugate comprising them, a nucleic acid molecule or a vector or a host cell encoding them, or a pharmaceutical composition comprising any one of the foregoing for use in the prevention and/or treatment of a disease, or a use of the single-domain antibodies as described in the third aspect, a polypeptide construct or a multispecific antibody or a conjugate comprising them, a nucleic acid molecule or a vector or a host cell encoding them, or a pharmaceutical composition comprising any one of the foregoing in the preparation of a drug for the prevention and/or treatment and/or neoadjuvant treatment and/or adjuvant treatment of a disease.

In certain embodiments, the disease is a disease associated with PD-L1. In certain embodiments, the disease associated with PD-L1 is a tumor.

In certain embodiments, the tumor is a PD-L1 positive tumor.

In certain embodiments, the tumor is a solid tumor or a hematological tumor. In certain embodiments, the tumor is selected from: gastric cancer, liver cancer, kidney tumor, lung cancer, small intestine cancer, bone cancer, prostate cancer, colorectal cancer, breast cancer, colon cancer, cervical cancer, lymphoma, adrenal tumor, bladder tumor, melanoma, head and neck tumor, esophageal cancer, and skin cancer.

In another aspect, the present disclosure relates to a method for the prevention and/or treatment and/or neoadjuvant treatment and/or adjuvant treatment of a disease, comprising administering to a subject in need thereof the multispecific antibodies as described in the fifth, sixth, or seventh aspect, a nucleic acid molecule or a vector or a host cell encoding them, or a pharmaceutical composition comprising any one of the foregoing. The present disclosure further relates to the multispecific antibodies as described in the fifth, sixth, or seventh aspect, a nucleic acid molecule or a vector or a host cell encoding them, or a pharmaceutical composition comprising any one of the foregoing for use in the prevention and/or treatment of a disease, or a use of the multispecific antibodies as described in the fifth, sixth, or seventh aspect, a nucleic acid molecule or a vector or a host cell encoding them, or a pharmaceutical composition comprising any one of the foregoing in the preparation of a medicament for the prevention and/or treatment and/or neoadjuvant treatment and/or adjuvant treatment of a disease.

The multispecific antibodies as described in the fifth, sixth, or seventh aspect of the present disclosure can be used in the treatment of any disease, provided that the effector mechanism of cytotoxic T cells is required in the treatment of the disease. For example, when the multispecific antibody of the present disclosure comprises a CD3 binding arm for T-cell recruitment and a tumor targeting arm specific for tumor-associated antigens (TAAs), it brings the T-cells into close contact with target tumor cells, locally activates the T-cells, and subsequently destroys the target cells with perforins and granzymes released by the T-cytotoxic granules. When further comprising a targeting arm specific for immune checkpoint molecules, it activates T cells in the tumor microenvironment, subsequently kills tumor cells, and enhances the anti-tumor effect.

In certain embodiments, the disease is a tumor.

In certain embodiments, the tumor is a solid tumor or a hematological tumor, such as gastric cancer, lung cancer, ovarian cancer, esophageal cancer, pancreatic cancer, cervical cancer, mesothelioma, breast cancer, prostate cancer, bladder cancer, ovarian cancer, colorectal cancer, head and neck squamous cell carcinoma, pancreatic cancer, cholangiocarcinoma, colon cancer, fallopian tube cancer, malignant melanoma, chronic lymphocytic leukemia, acute myeloid leukemia, or acute lymphocytic leukemia.

In certain embodiments, the treatment of the disease involves the effector mechanism of T cells (e.g., cytotoxic T cells). In certain embodiments, the multispecific antibody is used for recruiting T cells around target cells, activating T cells, and inducing a T-cell dependent cellular cytotoxicity (TDCC) effect, thereby effectively killing the target cells to treat or prevent the disease.

In certain embodiments, the tumor is an MSLN positive tumor, such as a solid tumor, such as mesothelioma, ovarian cancer, pancreatic cancer, breast cancer, cervical cancer, cholangiocarcinoma, prostate cancer, colon cancer, gastric cancer, fallopian tube cancer, lung cancer, or colorectal cancer; such as a hematological tumor, such as acute myeloid leukemia.

In certain embodiments, the tumor is a PD-L1 positive tumor, such as a solid tumor, such as gastric cancer, liver cancer, kidney tumor, lung cancer, small intestine cancer, bone cancer, prostate cancer, colorectal cancer, breast cancer, colon cancer, cervical cancer, lymphoma, adrenal tumor, bladder tumor, melanoma, head and neck tumor, esophageal cancer, and skin cancer.

In any one of the above aspects, the single-domain antibodies, polypeptide constructs, multispecific antibodies of the present disclosure or pharmaceutical compositions comprising them can be formulated into any dosage form known in the medical field.

In any one of the above aspects, the single-domain antibodies, polypeptide constructs, multispecific antibodies of the present disclosure or pharmaceutical compositions comprising them can be administered by any suitable method known in the art.

In any one of the above aspects, the single-domain antibodies, polypeptide constructs, multispecific antibodies of the present disclosure or pharmaceutical compositions comprising them can be formulated in dosage unit form for easy administration.

In any one of the above aspects, the single-domain antibodies, polypeptide constructs, multispecific antibodies of the present disclosure or pharmaceutical compositions comprising them can be administered alone or in combination with another pharmaceutically active agent (*e.g.,* an anti-tumor agent) or another therapy (*e.g.,* an anti-tumor therapy).

In any one of the above aspects, the subject can be a mammal, such as a human.

### Detection application

In another aspect, the present disclosure provides a kit comprising the antibody, the single-domain antibody, the polypeptide construct, the multispecific antibody, the isolated nucleic acid molecule, the vector, the host cell, the conjugate, or the pharmaceutical composition as described in any one of the above aspects.

In certain embodiments, the kit comprises instructions for use, *e.g.,* using the antibody, the single-domain antibody, the polypeptide construct, the multispecific antibody, the isolated nucleic acid molecule, the vector, the host cell, the conjugate, or the pharmaceutical composition to detect the presence or level of PD-L1, MSLN, and/or CD3 in a sample and/or instructions for diagnosing or differentially diagnosing a disease associated with PD-Ll, MSLN, and/or CD3.

In certain embodiments, the kit comprises the antibody or the antigen-binding fragment thereof that specifically binds to CD3 as described in the first aspect. The antibody or the antigen-binding fragment thereof optionally carries a detectable label. The kit is used for detecting the presence or level of CD3 in a sample and/or diagnosing or differentially diagnosing a disease associated with CD3.

In certain embodiments, the kit comprises the humanized single-domain antibody or the antigen-binding fragment thereof that specifically binds to MSLN as described in the second aspect or the polypeptide construct thereof. The humanized single-domain antibody or the antigen-binding fragment thereof or the polypeptide construct thereof optionally carries a detectable label. The kit is used for detecting the presence or level of MSLN in a sample and/or diagnosing or differentially diagnosing a disease associated with MSLN.

In certain embodiments, the kit comprises the single-domain antibody or the antigen-binding fragment thereof that specifically binds to PD-L1 as described in the third aspect or the polypeptide construct thereof. The single-domain antibody or the antigen-binding fragment thereof or the polypeptide construct thereof optionally carries a detectable label. The kit is used for detecting the presence of PD-L1 or its level in a sample and/or diagnosing or differentially diagnosing a disease associated with PD-L1.

In certain embodiments, the kit comprises the multispecific antibody as described in the fifth, sixth, or seventh aspect. The multispecific antibody optionally carries a detectable label. The kit is used for detecting the presence or level of PD-L1, MSLN, and/or CD3 in a sample and/or diagnosing or differentially diagnosing a disease associated with PD-L1, MSLN, and/or CD3.

In this context, the detectable label can be any substance detectable by fluorescent, spectral, photochemical, biochemical, immunological, electrical, optical, or chemical means. Particularly preferably, such labels can be adapted for immunological assays (*e.g.,* enzyme-linked immunoassay, radioimmunoassay, fluorescent immunoassay, and chemiluminescent immunoassay).

In another aspect, the present disclosure provides a method for detecting the presence or level of PD-L1, MSLN, and/or CD3 in a sample, comprising contacting the sample with the antibody, the single-domain antibody, the polypeptide construct, the multispecific antibody, the isolated nucleic acid molecule, the vector, the host cell, the conjugate, or the pharmaceutical composition as described in any one of the above aspects under conditions allowing the formation of antibody-antigen immune complexes, and detecting the formation of the complex. The formation of the complex indicates the presence of the target antigen or cells expressing the target antigen. The target antigen refers to the antigen targeted by the above detection reagent used. The methods can be used for diagnostic purposes or non-diagnostic purposes.

In certain embodiments, the methods are performed *in vitro.*

In certain embodiments, the method is an immunological assay (*e.g.,* enzyme-linked immunoassay, radioimmunoassay, fluorescent immunoassay, and chemiluminescent immunoassay).

In certain embodiments, the antibody, the single-domain antibody, the polypeptide construct, the multispecific antibody, the isolated nucleic acid molecule, the vector, the host cell, the conjugate, or the pharmaceutical composition further carries a detectable label.

In certain embodiments, the method uses the antibody or the antigen-binding fragment thereof that specifically binds to CD3 as described in the first aspect and is used to detect the presence or level of CD3 in a sample and/or to diagnose or differentially diagnose a disease associated with CD3.

In certain embodiments, the method uses the humanized single-domain antibody or the antigen-binding fragment thereof that specifically binds to MSLN as described in the second aspect or the polypeptide construct thereof, and is used to detect the presence or level of MSLN in a sample and/or to diagnose or differentially diagnose a disease associated with MSLN.

In certain embodiments, the method uses the single-domain antibody or the antigen-binding fragment thereof that specifically binds to PD-L1 as described in the third aspect or the polypeptide construct thereof, and is used to detect the presence or level of PD-L1 in a sample and/or to diagnose or differentially diagnose a disease associated with PD-L1.

In certain embodiments, the method uses the multispecific antibody as described in the fifth, sixth, or seventh aspect, and is used to detect the presence or level of PD-L1, MSLN, and/or CD3 in a sample and/or to diagnose or differentially diagnose a disease associated with PD-L1, MSLN, and/or CD3.

In another aspect, the present disclosure provides a method for diagnosing or differentially diagnosing a disease associated with PD-L1, MSLN, and/or CD3, wherein the method comprises using the antibody, the single-domain antibody, the polypeptide construct, the multispecific antibody, the isolated nucleic acid molecule, the vector, the host cell, the conjugate, or the pharmaceutical composition as described in any one of the above aspects.

In certain embodiments, the method detects the presence or level of PD-L1, MSLN, and/or CD3 in a sample from a subject by using the detection method described above, thereby diagnosing or differentially diagnosing a disease.

In certain embodiments, the disease is associated with a target antigen, *e.g*., characterized by expression or abnormal expression of the target antigen. The target antigen refers to the antigen targeted by the above detection reagent used.

In certain embodiments, the method can further comprise the step of comparing the detected value of the target antigen in the obtained sample with a reference value. The reference value can be the level of the target antigen in a sample of a healthy control (also referred to as a "negative reference value"). For example, if the amount of the target antigen in a sample from the subject is elevated relative to a negative reference value, it is an indication that the subject suffers from a disease associated with the target antigen.

In certain embodiments, the disease is a tumor. In certain embodiments, the tumor is a solid tumor or a hematological tumor; more preferably, the solid tumor is selected from mesothelioma, ovarian cancer, pancreatic cancer, breast cancer, cervical cancer, cholangiocarcinoma, prostate cancer, colon cancer, stomach cancer, fallopian tube cancer, lung cancer, or colorectal cancer; the hematological cancer comprises acute myeloid leukemia.

In another aspect, provided is a use of the antibody, the single-domain antibody, the polypeptide construct, the multispecific antibody, the isolated nucleic acid molecule, the vector, the host cell, the conjugate, the pharmaceutical composition, or the kit as described in any one of the above aspects in the preparation of a detection reagent, wherein the detection reagent is used for detecting the presence or level of PD-L1, MSLN, and/or CD3 in a sample and/or diagnosing or differentially diagnosing a diseases associated with PD-L1, MSLN, and/or CD3.

### Definition of terms

In the present disclosure, all scientific and technical terms used herein have the meanings commonly understood by a person skilled in the art unless specified otherwise. To better understand the present disclosure, definitions and explanations of related terms are provided below.

When the terms "such as", "as", "for example", "comprise", "include" or variations thereof are used herein, such terms will not be considered as limiting terms, but will be interpreted to mean "but not limited to" or "not limited to".

Unless otherwise indicated herein or clearly contradicted by context, the terms "a" and "an" and "the" and similar referents should be interpreted to cover both singular and plural forms in the context of describing the present disclosure, especially in the context of the following claims.

As used herein, the term "antibody" refers to an immunoglobulin-derived molecule capable of specifically binding to a target antigen, and the immunoglobulin-derived molecule binds to the target antigen through at least one antigen-binding site located in its variable region. When referring to the term "antibody", it includes not only intact antibodies but also antigen-binding fragments capable of specifically binding to a target antigen, unless the context clearly indicates otherwise. An "intact antibody" typically consists of two pairs of polypeptide chains, each pair having a light chain (LC) and a heavy chain (HC). Antibody light chains can be classified into κ (kappa) and λ (lambda) light chains. Heavy chains can be classified into µ, δ, γ, α, or ε, and the isotypes of antibodies are defined as IgM, IgD, IgG, IgA, and IgE, respectively. In the light chain and heavy chain, the variable region and constant region are linked by the "J" region of about 12 or more amino acids, and the heavy chain also comprises the "D" region of about 3 or more amino acids. Each heavy chain consists of a heavy chain variable region (VH) and a heavy chain constant region (CH). The heavy chain constant region consists of three domains (CH1, CH2, and CH3). Each light chain consists of a light chain variable region (VL) and a light chain constant region (CL). The light chain constant region consists of one domain CL. Constant domains do not directly participate in the binding of antigens and antibodies, but exhibit a variety of effector functions, such as being capable of mediating the binding of immunoglobulins to host tissues or factors, including various cells of the immune system (e.g., effector cells) and the first component of the classical complement system (C1q). The VH region and VL region can also be subdivided into regions with high variability called complementary determining region (CDR), among which more conservative regions called framework regions (FRs) are interspersed. Each V_{H} or V_{L} consists of 3 CDRs and 4 FRs arranged in the following order: FR1, CDR1, FR2, CDR2, FR3, CDR3, and FR4 from amino terminus to carboxy terminus. The variable regions (VH and VL) of each heavy chain/light chain pair form antigen binding sites respectively.

As used herein, the term "complementarity determining region" or "CDR" refers to the amino acid residues in the variable region of an antibody responsible for antigen binding. The variable region of the heavy chain and the variable region of the light chain each contain three CDRs, designated CDR1, CDR2, and CDR3. The precise boundaries of these CDRs can be defined in accordance with various numbering systems known in the art, for example, defined in accordance with the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al., (1989) Nature 342:878-883), the IMGT numbering system (Lefranc et al., Dev. Comparat. Immunol. 27:55-77, 2003), the AbM numbering system (Martin ACR, Cheetham JC, Rees AR (1989) Modelling antibody hypervariable loops: A combined algorithm. Proc Natl Acad Sci USA 86:9268-9272), or the Contact numbering system (MacCallum, R. M., Martin, A. C. R., & Thornton, J. M. (1996). Antibody-antigen Interactions: Contact Analysis and Binding Site Topography. Journal of Molecular Biology, 262(5), 732-745.). For a given antibody, one skilled in the art will easily identify the CDR defined according to each numbering system. Moreover, correspondence between different numbering systems is well known to those skilled in the art.

In the present disclosure, the CDR contained in the antibody or the antigen-binding fragment thereof of the present disclosure can be determined according to various numbering systems known in the art, such as by Kabat, Chothia, IMGT, AbM, or Contact numbering systems. In certain embodiments, the anti-CD3 antibodies or the antigen-binding fragments thereof involved in the present disclosure contain CDRs determined by the Kabat numbering system. In certain embodiments, the anti-MSLN or PD-L1 single-domain antibodies or the antigen-binding fragments thereof of the present disclosure contain CDRs determined by the Kabat, Chothia, IMGT, AbM, or Contact numbering systems.

As used herein, the terms "framework region" or "FR" residues refer to those amino acid residues in the variable region of an antibody other than the CDR residues as defined above.

The term "antibody" is not limited by any particular method of producing antibodies. For example, it includes recombinant antibodies, monoclonal antibodies, and polyclonal antibodies. Antibodies can be antibodies of different isotypes, for example, IgG (*e.g.,* IgG1, IgG2, IgG3, or IgG4 subtypes), IgA1, IgA2, IgD, IgE, or IgM antibodies.

As used herein, the term "multispecific antibody" refers to antibodies having binding specificities for at least two (*e.g*., two, three, or four) different antigens (or epitopes). Multispecific antibodies comprise multiple antigen-binding domains with binding specificities for different antigens (or epitopes), and are thus capable of binding at least two different binding sites and/or target molecules. Each antigen-binding domain contained in the multispecific antibody can be independently selected from full-length antibodies (*e.g.*, IgG antibodies) or antigen-binding fragments thereof (*e.g.,* Fv fragments, Fab fragments, F(ab')₂ fragments, or scFv). In some cases, each antigen-binding domain is linked by a peptide linker.

As used herein, the term "antigen-binding fragment" of an antibody refers to a polypeptide comprising a fragment of a full-length antibody that retains the ability to specifically bind to the same antigen to which the full-length antibody binds, and/or competes with the full-length antibody to specifically bind to the antigen, and is also referred to as "antigen-binding portion". Antigen-binding fragments of antibodies can be produced by recombinant DNA technology or by enzymatic or chemical cleavage of intact antibodies. Non-limiting examples of antigen-binding fragments include Fab, Fab', F(ab')₂, Fd, Fv, complementarity determining region (CDR) fragments, scFv, diabodies, single-domain antibodies, chimeric antibodies, linear antibodies, nanobodies (technology from Domantis), probodies, and polypeptides that contain at least a portion of an antibody sufficient to confer specific antigen binding capacity to the polypeptide.

As used herein, the term "full-length antibody" means an antibody consisting of two "full-length heavy chains" and two "full-length light chains". Among them, "full-length heavy chain" refers to a polypeptide chain that consists of, in a direction from the N-terminus to the C-terminus, a heavy chain variable region (VH), a heavy chain constant region CH1 domain, a hinge region (HR), a heavy chain constant region CH2 domain, and a heavy chain constant region CH3 domain; moreover, when the full-length antibody is an IgE isotype, it optionally further comprises a heavy chain constant region CH4 domain. Preferably, the "full-length heavy chain" is a polypeptide chain consisting of VH, CH1, HR, CH2, and CH3 in the N-terminal to C-terminal direction. "Full-length light chain" is a polypeptide chain that consists of, in a direction from the N-terminus to the C-terminus, a light chain variable region (VL) and a light chain constant region (CL). The two pairs of full-length antibody chains are linked together by a disulfide bond between CL and CH1 and a disulfide bond between the HRs of the two full-length heavy chains. The full-length antibody contains two antigen-binding sites formed by VH and VL pairs, respectively, the two antigen-binding sites specifically recognize/bind the same antigen.

As used herein, the term "single-domain antibody (sdAb)" has the meaning commonly understood by those skilled in the art to refer to an antibody fragment consisting of a single monomeric variable antibody domain (*e.g.,* a single heavy chain variable region), typically derived from the variable region of a heavy chain antibody (*e.g*., a camelid antibody or a shark antibody). Typically, the nanobody consists of four framework regions and three complementarity determining regions, having the structure of FR1-CDR1-FR2-CDR2-FR3-CDR3-FR4. The single-domain antibody can be truncated at the N- or C-terminus to comprise only a portion of FR1 and/or FR4, or to lack one or both of those framework regions, so long as it substantially retains antigen binding and specificity. Single-domain antibodies are also called nanobodies, and the two are used interchangeably. As used herein, the term "antigen-binding fragment" of a single-domain antibody refers to a polypeptide comprising a fragment of a single-domain antibody that retains the ability to specifically bind to the same antigen bound by the single-domain antibody, and/or competes with the single-domain antibody for specific binding of the antigen. Antigen-binding fragments of single-domain antibodies of the present disclosure can be produced by recombinant DNA techniques or by enzymatic or chemical cleavage of the single-domain antibodies of the present disclosure. In some embodiments, the "antigen-binding fragment" of the single-domain antibody can be truncated at the N-terminus or C-terminus compared to the full-length single-domain antibody so that it contains only a portion of FR1 and/or FR4, or lacks one or both of those framework regions, as long as it substantially retains antigen binding and specificity.

As used herein, the term "Fab fragment" means an antibody fragment consisting of the VL, VH, CL, and CH1 domains. The term "F(ab')₂ fragment "means an antibody fragment comprising two Fab fragments linked by a disulfide bridge on the hinge region. The term "Fab' fragment" means a fragment obtained by reducing disulfide bonds connecting two heavy chain fragments in the F(ab')₂ fragment and consisted of an intact light chain and the Fd fragment (consisting of the VH and CH1 domains) of the heavy chain.

As used herein, the term "Fv" means an antibody fragment consisting of the VL and VH domains of the antibody's single arm. The Fv fragment is generally considered to be the smallest antibody fragment that can form an intact antigen binding site. It is generally believed that the six CDRs confer antigen binding specificity to an antibody. However, even one variable region (such as an Fd fragment, which contains only three antigen-specific CDRs) is able to recognize and bind to an antigen, although it may have a lower affinity than an intact binding site.

As used herein, the term "Fc domain" or "Fc region" means a portion of the constant region of the heavy chain comprising CH2 and CH3. The Fc fragment of an antibody has a number of different functions, but is not involved in antigen binding. "Effector function" mediated by the Fc region includes Fc receptor binding; Clq binding and complement-dependent cytotoxicity (CDC); antibody-dependent cell-mediated cytotoxicity (ADCC); phagocytosis; down-regulation of cell surface receptors, such as B cell receptors; B cell activation, *etc.* In some embodiments, the Fc region comprises a hinge, CH2, and CH3. When the Fc region comprises a hinge, the hinge regulates the dimerization between two Fc-containing polypeptides. The Fc region can be any antibody heavy chain constant region isotype, such as IgG1, IgG2, IgG3, or IgG4.

The Fc domain can include either a native Fc region or a variant Fc region. The native Fc region includes amino acid sequences consistent with the amino acid sequences of the Fc region found in nature, for example, the native human Fc region sequence includes the native human IgG1 Fc region sequence (non-A and A allotypes); native human IgG2 Fc region sequence; native human IgG3 Fc region sequence; and the native human IgG4 Fc region sequence, and naturally occurring variants thereof. The variant Fc region includes an amino acid sequence that differs from the amino acid sequence of the native Fc region sequence due to at least one amino acid modification. In some embodiments, the variant Fc region can have effector functions (e.g., Fc receptor binding, antibody glycosylation, number of cysteine residues, effector cell function, or complement function) that are altered compared to the native Fc region.

As used herein, the term "scFv" refers to a single polypeptide chain comprising the VL and VH domains, wherein the VL and VH are linked by a linker. Such scFv molecules can have a general structure: NH₂-VL-linker-VH-COOH or NH₂-VH-linker-VL-COOH. Suitable linkers in the prior art consist of repeated GGGGS amino acid sequences or variants thereof. For example, a linker having amino acid sequence (GGGGS)₂ can be used, and variants thereof can also be used. In some cases, a disulfide bond can also exist between VH and VL of the scFv.

As used herein, the term "diabody" means that the VH and VL domains thereof are expressed on a single polypeptide chain, but the used linker is too short to allow pairing between the two domains of the same chain, thereby forcing one domain to pair with the complementary domain of another chain and generating two antigen-binding sites.

Each of the above antibody fragments retains the ability to specifically bind to the same antigen bound by the full-length antibody, and/or competes with the full-length antibody for specific binding to the antigen.

Conventional techniques known to those skilled in the art (for example, recombinant DNA technology or enzymatic or chemical cleavage methods) can be used to obtain from a given antibody (for example, the antibody provided by the present disclosure) the antigen-binding fragments of the antibody (for example, the above antibody fragments) which can be screened for specificity in the same manner by which intact antibodies are screened.

As used herein, the term "identity" is used to refer to the matching of sequences between two polypeptides or between two nucleic acids. In order to determine the percentage identity of two amino acid sequences or two nucleic acid sequences, the sequences are aligned for optimal comparison purposes (*e.g.,* a gap can be introduced in the first amino acid sequence or nucleic acid sequence for optimal alignment with the second amino acid or nucleic acid sequence). The amino acid residues or nucleotides at corresponding amino acid positions or nucleotide positions are then compared. When a position in the first sequence is occupied by an amino acid residue or nucleotide that is the same as the corresponding position in the second sequence, the molecules are identical in that position. The percentage identity between two sequences is a function of the number of identical positions shared by the sequences (*i.e.,* percentage identity = number of the identical overlapping positions/total number of positions × 100%). In certain embodiments, both sequences are of the same length.

The determination of percentage identity between the two sequences can also be achieved using mathematical algorithms. One non-limiting example of a mathematical algorithm for the comparison of two sequences is the algorithm of Karlin and Altschul, 1990, Proc. Natl. Acad. Sci. U.S.A. 87: 2264-2268, as modified in Karlin and Altschul, 1993, Proc. Natl. Acad. Sci. U.S.A. 90: 5873-5877. This algorithm is integrated into the NBLAST and XBLAST programs of Altschul et al., 1990, J. Mol. Biol. 215:403.

As used herein, the term "variant", in the context of polypeptides (including polypeptides), also refers to polypeptides or peptides containing amino acid sequences that have been altered by the introduction of amino acid residue substitution, deletion, or addition. In certain cases, the term "variant" also refers to a polypeptide or peptide that has been modified (*i.e.,* by covalently linking any type of molecule to the polypeptide or peptide). For example, but not by way of limitation, the polypeptide can be modified, such as by glycosylation, acetylation, pegylation, phosphorylation, amidation, derivatization by known protecting/blocking groups, proteolytic cleavage, attachment to cell ligands or other proteins, *etc.* Derived polypeptides or peptides can be produced by chemical modification using techniques known to those skilled in the art, and the techniques include but are not limited to specific chemical cleavage, acetylation, formylation, metabolic synthesis of tunicamycin, *etc.* In addition, variants have similar, identical, or improved functions to the polypeptide or peptide from which they are derived. In certain embodiments, the variant has at least 90%, at least 91%, at least 92%, at least 93%, at least 94%, at least 95%, at least 96%, at least 97%, at least 98%, or at least 99% sequence identity compared to the sequence from which it is derived.

As used herein, the term "specific binding" refers to a non-random binding reaction between two molecules, such as the reaction between an antibody and the antigen it targets. The strength or affinity of a specific binding interaction can be expressed by the equilibrium dissociation constant (K_{D}) of the interaction. In the present disclosure, the term "K_{D}" refers to the equilibrium dissociation constant of a specific antibody-antigen interaction, which is used to describe the binding affinity between an antibody and an antigen. The smaller the equilibrium dissociation constant, the more tightly the antibody-antigen binding, the higher the affinity between the antibody and the antigen.

The specific binding properties between the two molecules can be determined using methods known in the art. One method involves measuring the rate at which antigen binding sites/antigen complexes form and dissociate. Both "binding rate constant" (ka or kon) and "dissociation rate constant" (kdis or koff) can be calculated by concentration and actual rates of association and dissociation (see Malmqvist M, Nature,1993, 361:186-187). The ratio of kdis/kon is equal to the dissociation constant K_{D} (see Davies et al., Annual Rev Biochem, 1990; 59:439-473). K_{D}, kon, and kdis values can be measured by any effective method. In certain embodiments, dissociation constants can be measured using surface plasmon resonance (SPR) in Biacore, and dissociation constants can also be measured using bioluminescence interferometry or Kinexa.

As used herein, the term "vector" refers to a nucleic acid delivery vehicle in which polynucleotides can be inserted. When the vector enables a protein encoded by the inserted polynucleotide to be expressed, the vector is called an expression vector. Vectors can be transformed, transduced, or transfected into host cells, and the genetic material elements carried by them can be expressed in host cells. Vectors are well known to those skilled in the art and include, but are not limited to: plasmids; phagemids; cosmids; artificial chromosomes, such as yeast artificial chromosomes (YACs), bacterial artificial chromosomes (BACs), or P1-derived artificial chromosomes (PACs); bacteriophages such as lambda phage or M13 phage, and animal viruses. Animal viruses that can be used as vectors include, but are not limited to, retroviruses (including lentiviruses), adenoviruses, adeno-associated viruses, herpes viruses (such as herpes simplex virus), poxviruses, baculoviruses, papillomavirus, and papovaviruses (such as SV40). A vector can contain a plurality of elements controlling expression, including but not limited to promoter sequences, transcription start sequences, enhancer sequences, selection elements, and reporter genes. In addition, the vector can also contain a replication initiation site.

As used herein, the term "host cell" refers to cells that can be used to introduce vectors, and includes but is not limited to prokaryotic cells such as *Escherichia coli* or *Bacillus subtilis,* fungal cells such as yeast cells or *Aspergillus,* insect cells such as S2 *Drosophila* cells or Sf9 cells, or animal cells such as fibroblasts, CHO cells, COS cells, NSO cells, HeLa cells, BHK cells, HEK 293 cells, or human cells.

As used herein, the term "conservative substitution" means an amino acid substitution that does not adversely affect or alter the expected properties of proteins/peptides containing amino acid sequences. For example, conserved substitutions can be introduced by standard techniques known in the art, such as site-directed mutagenesis and PCR-mediated mutagenesis. Conservative amino acid substitution includes the substitution of amino acid residues with amino acid residues having similar side chains, for example, substitution with residues that are physically or functionally similar to the corresponding amino acid residues (*e.g.,* having similar size, shape, charge, chemical properties, including the ability to form covalent or hydrogen bonds, *etc*.). Families of amino acid residues with similar side chains have been defined in the art. These families include amino acids with basic side chains (*e.g.,* lysine, arginine, and histidine), acidic side chains (*e.g.,* aspartic acid and glutamic acid), uncharged polar side chains (*e.g.,* glycine, asparagine, glutamine, serine, threonine, tyrosine, cysteine, and tryptophan), nonpolar side chains (*e.g.,* alanine, valine, leucine, isoleucine, proline, phenylalanine, and methionine), beta-branched side chains (*e.g.,* threonine, valine, and isoleucine), and aromatic side chains (*e.g.,* tyrosine, phenylalanine, tryptophan, and histidine). Therefore, it is preferable to replace the corresponding amino acid residue with another amino acid residue from the same side chain family. In addition, amino acid residues can be divided into categories defined according to optional physical and functional properties. For example, alcohol-containing residues (S and T), aliphatic residues (I, L, V, and M), cycloalkenyl-related residues (F, H, W, and Y), hydrophobic residues (A, C, F, G, H, I, L, M, R, T, V, W, and Y), negatively charged residues (D and E), polar residues (C, D, E, H, K, N, Q, R, S, and T), positively charged residues (H, K, and R), small residues (A, C, D, G, N, P, S, T, and V), minimal residues (A, G, and S), residues related to corner formation (A, C, D, E, G, H, K, N, Q, R, S, P, and T), and flexible residues (Q, T, K, S, G, P, D, E, and R). Methods for identifying conservative substitutions of amino acids are well known in the art (see, *e.g*., Brummell et al., Biochem. 32:1180-1187 (1993); Kobayashi et al. Protein Eng. 12 (10):879-884 (1999); and Burks et al. Proc. Natl Acad. Set USA 94:412-417 (1997), which are incorporated herein by reference).

The twenty conventional amino acids involved in the present disclosure are written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference. In the present disclosure, the terms "polypeptide" and "protein" have the same meaning and are used interchangeably. In the present disclosure, amino acids are typically denoted by one-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient and that is well known in the art (see, for example, Remington's Pharmaceutical Sciences. Edited by Gennaro AR, 19th ed. Pennsylvania: Mack Publishing Company, 1995), and includes but is not limited to: pH regulators, surfactants, adjuvants, ionic strength enhancers, diluents, reagents for maintaining osmotic pressure, reagents for delayed absorption, and preservatives.

As used herein, the term "prevention" refers to a method implemented to prevent or delay the onset of a disease or condition or symptom in a subject's body. As used herein, the term "treatment" refers to a method implemented in order to obtain a beneficial or desired clinical outcome. For the objective of the present disclosure, beneficial or necessary clinical outcomes include, but are not limited to, alleviating symptoms, narrowing the extent of the disease, stabilizing (*i.e.,* preventing from getting worse) the state of the disease, delaying or slowing down disease progression, ameliorating or alleviating the state of the disease, and relieving symptoms (whether partially or fully), whether detectable or undetectable. In addition, "treatment" can also refer to an extended survival period compared to the expected survival period (if not treated). Herein, treatment can include neoadjuvant treatment and/or adjuvant treatment. "Neoadjuvant treatment" refers to a therapy that is administered to a patient prior to a planned surgery for treating the disease. "Adjuvant treatment" refers to a therapy that is administered to a patient after a surgery for treating the disease.

As used herein, the term "subject" refers to a mammal, such as a primate mammal, such as a human. In certain embodiments, the subject (*e.g.,* a human) has a tumor, an inflammatory disease, or an autoimmune disease, or is at risk of suffering from the above diseases.

As used herein, the term "effective amount" refers to an amount sufficient to obtain, or at least partially obtain, the desired effect. For example, an effective amount for preventing a disease refers to an amount sufficient to prevent, stop, or delay the occurrence of a disease; an effective amount for treating a disease refers to an amount sufficient to cure or at least partially stop a disease and its complications in a patient who already suffers from the disease. The determination of such an effective amount is well within the competence of those skilled in the art. For example, the effective amount for therapeutic use will depend on the severity of the disease to be treated, the general state of the patient's own immune system, the general condition of the patient such as age, weight, and sex, the manner in which the drug is administered, other treatments administered at the same time, *etc.*

### Beneficial effects of the present disclosure

The nanobodies targeting PD-L1 of the present disclosure have good binding affinity and specificity to PD-L1, can effectively promote the secretion of IL2 and IFNγ, and can be used to prevent and/or treat diseases or conditions associated with PD-L1, and the humanized antibodies of the nanobodies retain the functions and properties of the parent camel-derived antibody, and also have a high degree of humanization, thus having the potential to be administered to human subjects. The humanized nanobodies targeting MSLN of the present disclosure have a high degree of humanization, are less likely to cause immune side reactions, and have good binding affinity and specificity to MSLN, thereby achieving efficient targeting of tumors expressing MSLN. In addition, compared with known multispecific T cell activation structures, the multispecific antibodies targeting CD3, tumor-associated antigens, and immune checkpoints further provided by the present disclosure have obvious advantages. The multispecific antibodies of the present disclosure can moderately activate T cells and have improved drug safety. At the same time, the combination of targeting tumor-associated antigens and immune checkpoints significantly increases the anti-tumor effect. In particular, the present disclosure specifically provides a trispecific antibody targeting CD3, MSLN, and PD-L1, which can reduce cytokine release to ensure safety while having a good anti-tumor effect.

Embodiments of the present disclosure will be described in detail below in conjunction with the drawings and examples, but it will be understood by those skilled in the art that the following drawings and examples are intended only to illustrate the present disclosure and not to limit the scope of the present disclosure. The various purposes and advantages of the present disclosure will become apparent to those skilled in the art according to the drawings and the following detailed description of preferred embodiments.

### BRIEF DESCRIPTION OF THE DRAWINGS

FIGs. 1A-1C sequentially show the blocking activity of the 4A7 nanobody against the binding of human PD-L1 to human PD-1 (A), mouse PD-L1 to mouse PD-1 (B), and monkey PD-L1 to monkey PD-1 (C) in Example 2.
FIG. 2 shows the binding activity detection of Benchmark-ATE, 7B11, and 7B11_huGS_HM4 to human PD-L1 cells respectively in Example 2.
FIG. 3 shows the binding activity detection of Benchmark-ATE, 7B11, and 7B11_huGS_HM4 to monkey PD-L1 cells respectively in Example 2.
FIG. 4 shows the blocking activity detection of Benchmark-ATE, 7B11, and 7B11_huGS_HM4 on human PD-L1 and human PD-1 respectively in Example 2.
FIG. 5 shows the activity detection of Benchmark-ATE, 7B11, and 7B11_huGS_HM4 in blocking the T cell PD-1/PD-L1 signaling pathway in Example 2.
FIG. 6 shows the detection results of Benchmark-ATE, 7B11, and 7B11_huGS_HM4 activating T cells to produce cytokines in Example 2; wherein FIG. 6a shows the detection results of Benchmark-ATE, 7B11, and 7B11_huGS_HM4 activating T cells to produce IL2; FIG. 6b shows the detection results of Benchmark-ATE, 7B11, and 7B11_huGS_HM4 activating T cells to produce IFNγ.
FIG. 7 shows the structural schematic diagram of the CD3-PDL1-MSLN trispecific antibody constructed in Example 3.
FIGs. 8A-8D respectively show the binding activity of the CD3-PDL1-MSLN trispecific antibody to human MC38/MSLN cells in Example 4.
FIGs. 9A-9C respectively show the binding activity of the CD3-PDL1-MSLN trispecific antibody to CHO-hPDL1 cells in Example 4.
FIGs. 10A-10C respectively show the blocking activity of the CD3-PDL1-MSLN trispecific antibody on the binding of human PD1 to human PD-L1 in Example 5.
FIGs. 11A-11C respectively show the activation of the CD3-PDL1-MSLN trispecific antibody on Jurkat-NFAT-luc cells in the tumor group in Example 7.
FIGs. 12A-12C respectively show the activation of Jurkat-NFAT-luc cells by the CD3-PDL1-MSLN trispecific antibody in the tumor-free group in Example 7.
FIGs. 13A-13C respectively show the TDCC activity of the CD3-PDL1-MSLN trispecific antibody on human tumor cells HCC1806 expressing MSLN in Example 8.
FIGs. 14A-14F respectively show the detection results of IL-2 release levels mediated by the CD3-PDL1-MSLN trispecific antibody in Example 9.
FIGs. 15A-15F respectively show the detection results of INF-γ release levels mediated by the CD3-PDL1-MSLN trispecific antibody in Example 9.
FIGs. 16A-16B respectively show the *in vivo* killing activity and safety of the 5Y3-16 antibody in Example 10.
FIGs. 17A-17B respectively shows the structural schematic diagram of the CD3-PDL1-MSLN trispecific antibody constructed in Example 11.
FIGs. 18A-18E respectively show the TDCC activity of CD3-PDL1-MSLN trispecific antibodies with different structures on tumor cells in Example 11.

### Sequence information

A description of the sequences involved in the present disclosure is provided in the following table.

**Table 1: Sequence information**

| SEQ ID NO: | Description | Sequence |
|---|---|---|
| 1 | VHH amino acid sequence of 3C6hu-1 | EVQLVESGGGLIQPGGSLRLSCAASGNIGSIRHMGWVRQA PGKGLEWVSTITTVGTTYYANFVKGRFTISRDNSKNTLYL QMNSLRAEDTAVYYCRATAHPTDYWGQGTLVTVSS |
| 2 | VHH amino acid sequence of 3C6hu-2 | EVQLVESGGGLIQPGGSLRLSCAASGNIGSIRHMGWHRQA PGKGLELVSTITTVGTTYYANFVKGRFTISRDNSKNTLYLQ MNSLRAEDTAVYYCRATAHPTDYWGQGTLVTVSS |
| 3 | VHH amino acid sequence of 3C6hu-3 | EVQLVESGGGLIQPGGSLRLSCAASGNIGSIRHMGWHRQA PGKELELVSTITTVGTTYYANFVKGRFTISRDNSKNTLYLQ MNSLRAEDTAVYYCRATAHPTDYWGQGTLVTVSS |
| 4 | VHH amino acid sequence of 3C6hu-4 | EVQLVESGGGLIQPGGSLRLSCAASGNIGSIRHMGWHRQA PGKGRELVSTITTVGTTYYANFVKGRFTISRDNSKNTLYLQ MNSLRAEDTAVYYCRATAHPTDYWGQGTLVTVSS |
| 5 | VHH amino acid sequence of 3C6hu-5 | EVQLVESGGGLIQPGGSLRLSCAASGNIGSIRHMGWHRQA PGKERELVSTITTVGTTYYANFVKGRFTISRDNSKNTLYLQ MNSLRAEDTAVYYCRATAHPTDYWGQGTLVTVSS |
| 6 | VHH amino acid sequence of 3C6hu-6 | EVQLVESGGGLIQPGGSLRLSCAASGNIGSIRHMGWHRQA PGKERELVATITTVGTTYYANFVKGRFTISRDNSKNTLYLQ MNSLRAEDTAVYYCRATAHPTDYWGQGTLVTVSS |
| 7 | VHH amino acid sequence of 3C6hu-7 | EVQLVESGGGLIQPGGSLRLSCAASGNIGSIRHMGWHRQA PGKEREWVATITTVGTTYYANFVKGRFTISRDNSKNTLYL QMNSLRAEDTAVYYCRATAHPTDYWGQGTLVTVSS |
| 8 | VHH amino acid sequence of 3C6hu-8 | EVQLVESGGGLIQPGGSLRLSCAASGNIGSIRHMGWVRQA PGKERELVATITTVGTTYYANFVKGRFTISRDNSKNTLYLQ MNSLRAEDTAVYYCRATAHPTDYWGQGTLVTVSS |
| 9 | 3C6 | EVQLVESGGGLVQAGESLRLSCAVSGNIGSIRHMGWHRQD PGKERELVATITTVGTTYYANFVKGRFTISRDNAKNTVYLQ MNSLKPEDTGVYYCRATAHPTDYWGQGTQVTVSS |
| 10 | 4A7 | EVQLVESGGGLVQAGDSLRLGCAASGRTFSTNVMGWFRQ APGKEREFVSAISRAGSTYSADSVKGRFTISRDNAENTVYL QMNSLKPEDTAVYYCAAGSSPTAIFGKSGMDYWGKGTLV TVSS |
| 11 | 4A7 CDR1-1(IMGT) | GRTFSTNV |
| 12 | 4A7 CDR2(IMGT) | ISRAGST |
| 13 | 4A7 CDR3(IMGT) | AAGSSPTAIFGKSGMDY |
| 14 | 4A7 CDR1(Kabat) | TNVMG |
| 15 | 4A7 CDR2(Kabat) | AISRAGSTYSADSVKG |
| 16 | 4A7 CDR3(Kabat/ AbM/Chothia) | GSSPTAIFGKSGMDY |
| 17 | 4A7 CDR1(AbM) | GRTFSTNVMG |
| 18 | 4A7 CDR2(AbM) | AISRAGSTY |
| 19 | 4A7 CDR1(Chothia) | GRTFSTN |
| 20 | 4A7 CDR2(Chothia) | SRAGS |
| 21 | 4A7 CDR1(Contact) | STNVMG |
| 22 | 4A7 CDR2(Contact) | FVSAISRAGSTY |
| 23 | 4A7 CDR3(Contact) | AAGSSPTAIFGKSGMD |
| 24 | VHH amino acid sequence of 4A7hu-1 | EVQLLESGGGLVQPGGSLRLSCAASGRTFSTNVMSWVRQ APGKGLEWVSAISRAGSTYYADSVKGRFTISRDNSKNTLY LQMNSLRAEDTAVYYCAAGSSPTAIFGKSGMDYWGQGTQ VTVSS |
| 25 | VHH amino acid sequence of 4A7hu-2 | EVQLLESGGGLVQPGGSLRLSCAASGRTFSTNVMGWVRQ APGKGLEWVSAISRAGSTYSADSVKGRFTISRDNSKNTLY LQMNSLRAEDTAVYYCAAGSSPTAIFGKSGMDYWGQGTQ VTVSS |
| 26 | VHH amino acid sequence of 4A7hu-3 | EVQLLESGGGLVQPGGSLRLSCAASGRTFSTNVMGWFRQ APGKGLEWVSAISRAGSTYSADSVKGRFTISRDNSKNTLY LQMNSLRAEDTAVYYCAAGSSPTAIFGKSGMDYWGQGTQ VTVSS |
| 27 | VHH amino acid sequence of 4A7hu-4 | EVQLLESGGGLVQPGGSLRLSCAASGRTFSTNVMGWVRQ APGKGLEFVSAISRAGSTYSADSVKGRFTISRDNSKNTLYL QMNSLRAEDTAVYYCAAGSSPTAIFGKSGMDYWGQGTQ VTVSS |
| 28 | VHH amino acid sequence of 4A7hu-5 | EVQLLESGGGLVQPGGSLRLSCAASGRTFSTNVMGWFRQ APGKGLEFVSAISRAGSTYSADSVKGRFTISRDNSKNTLYL QMNSLRAEDTAVYYCAAGSSPTAIFGKSGMDYWGQGTQ VTVSS |
| 29 | VHH amino acid sequence of 4A7hu-6 | EVQLLESGGGLVQAGGSLRLSCAASGRTFSTNVMGWFRQ APGKGLEFVSAISRAGSTYSADSVKGRFTISRDNSKNTLYL QMNSLRAEDTAVYYCAAGSSPTAIFGKSGMDYWGQGTQ VTVSS |
| 30 | VHH amino acid sequence of 4A7hu-7 | EVQLLESGGGLVQPGGSLRLSCAASGRTFSTNVMGWFRQ APGKGREFVSAISRAGSTYSADSVKGRFTISRDNSKNTLYL QMNSLRAEDTAVYYCAAGSSPTAIFGKSGMDYWGQGTQ VTVSS |
| 31 | VHH amino acid sequence of 4A7hu-8 | EVQLLESGGGLVQPGGSLRLSCAASGRTFSTNVMGWFRQ APGKEREFVSAISRAGSTYSADSVKGRFTISRDNSKNTLYL QMNSLRAEDTAVYYCAAGSSPTAIFGKSGMDYWGQGTQ VTVSS |
| 32 | VHH amino acid sequence of 4A7hu-9 | EVQLLESGGGLVQPGGSLRLSCAASGRTFSSYVMSWVRQ APGKGLEWVSAISRAGSTYYADSVKGRFTISRDNSKNTLY LQMNSLRAEDTAVYYCAAGSSPTAIFGKSGMDYWGQGTQ VTVSS |
| 33 | VHH amino acid sequence of 4A7hu-10 | EVQLLESGGGLVQPGGSLRLSCAASGRTFSSYVMGWVRQ APGKGLEWVSAISRAGSTYSADSVKGRFTISRDNSKNTLY LQMNSLRAEDTAVYYCAAGSSPTAIFGKSGMDYWGQGTQ VTVSS |
| 34 | VHH amino acid sequence of 4A7hu-11 | EVQLLESGGGLVQPGGSLRLSCAASGRTFSSYVMGWFRQ APGKGLEWVSAISRAGSTYSADSVKGRFTISRDNSKNTLY LQMNSLRAEDTAVYYCAAGSSPTAIFGKSGMDYWGQGTQ VTVSS |
| 35 | VHH amino acid sequence of 4A7hu-12 | EVQLLESGGGLVQPGGSLRLSCAASGRTFSSYVMGWVRQ APGKGLEFVSAISRAGSTYSADSVKGRFTISRDNSKNTLYL QMNSLRAEDTAVYYCAAGSSPTAIFGKSGMDYWGQGTQ VTVSS |
| 36 | VHH amino acid sequence of 4A7hu-13 | EVQLLESGGGLVQPGGSLRLSCAASGRTFSSYVMGWFRQ APGKGLEFVSAISRAGSTYSADSVKGRFTISRDNSKNTLYL QMNSLRAEDTAVYYCAAGSSPTAIFGKSGMDYWGQGTQ VTVSS |
| 37 | VHH amino acid sequence of 4A7hu-14 | EVQLLESGGGLVQAGGSLRLSCAASGRTFSSYVMGWFRQ APGKGLEFVSAISRAGSTYSADSVKGRFTISRDNSKNTLYL QMNSLRAEDTAVYYCAAGSSPTAIFGKSGMDYWGQGTQ VTVSS |
| 38 | VHH amino acid sequence of 4A7hu-15 | EVQLLESGGGLVQPGGSLRLSCAASGRTFSSYVMGWFRQ APGKGREFVSAISRAGSTYSADSVKGRFTISRDNSKNTLYL QMNSLRAEDTAVYYCAAGSSPTAIFGKSGMDYWGQGTQ VTVSS |
| 39 | VHH amino acid sequence of 4A7hu-16 | EVQLLESGGGLVQPGGSLRLSCAASGRTFSSYVMGWFRQ APGKEREFVSAISRAGSTYSADSVKGRFTISRDNSKNTLYL QMNSLRAEDTAVYYCAAGSSPTAIFGKSGMDYWGQGTQ VTVSS |
| 40 | CD3-VH1 | EVQLVESGGGLVQPGGSLRLSCAASGFTFNTYAMNWVRQ APGKGLEWVARIRSKYNNYATYYADSVKGRFTISRDDSKS SLYLQMNSLKTEDTAVYYCVRHGNFGNNYVSWFASWGQ GTLVTVSS |
| 41 | CD3-VH2 | EVQLVESGGGLVQPGGSLRLSCAASGFTFNTYAMNWVRQ APGKGLEWVARIRSKYNNYATYYADSVKGRFTISRDDSKS |
| | | ILYLQMNSLKTEDTAMYYCVRHERFGNSYVSWFAYWGQ GTLVTVSS |
| 42 | CD3-VH3 | EVQLVESGGGLVQPGGSLRLSCAASGFTFNTYAMNWVRQ APGKGLEWVARIRSKYNNYATYYADSVKDRFTISRDDSKS SLYLQMNNLKTEDTAMYYCVRHGNFGNTYVSWFAMWG QGTLVTVSS |
| 43 | CD3-VL | QAVVTQEPSFSVSPGGTVTLTCRSSTGAVTTSNYANWVQQ TPGQAFRGLIGGTNKRAPGVPARFSGSLIGDKAALTITGAQ ADDESIYFCALWYSNLWVFGGGTKLTVL |
| 44 | Light chain constant region | GQPKAAPSVTLFPPSSEELQANKATLVCLISDFYPGAVTVA WKADSSPVKAGVETTTPSKQSNNKYAASSYLSLTPEQWK SHRSYSCQVTHEGSTVEKTVAPTECS |
| 45 | Mutant 1 of IgG1 | ASTKGPSVFPLAPSSKSTSGGTAALGCLVKDYFPEPVTVS WNSGALTSGVHTFPAVLQSSGLYSLSSVVTVPSSSLGTQTY ICNVNHKPSNTKVDKKVEPKSCDKTHTCPPCPAPEAAGAP SVFLFPPKPKDTLMISRTPEVTCVVVDVSHEDPEVKFNWY VDGVEVHNAKTKPREEQYNSTYRVVSVLTVLHQDWLNG KEYKCAVSNKALPAPIEKTISKAKGQPREPQVYTLPPSRDE LTKNQVSLWCLVKGFYPSDIAVEWESNGQPENNYKTTPPV LDSDGSFFLYSKLTVDKSRWQQGNVFSCSVMHEALHNHY TQKSLSLSPG |
| 46 | Mutant 1-Fc of IgG1 | DKTHTCPPCPAPEAAGAPSVFLFPPKPKDTLMISRTPEVTC VVVDVSHEDPEVKFNWYVDGVEVHNAKTKPREEQYNST YRVVSVLTVLHQDWLNGKEYKCAVSNKALPAPIEKTISKA KGQPREPQVYTLPPSRDELTKNQVSLWCLVKGFYPSDIAV EWESNGQPENNYKTTPPVLDSDGSFFLYSKLTVDKSRWQ QGNVFSCSVMHEALHNHYTQKSLSLSPG |
| 47 | Mutant 2-Fc of IgG1 | DKTHTCPPCPAPEAAGAPSVFLFPPKPKDTLMISRTPEVTC VVVDVSHEDPEVKFNWYVDGVEVHNAKTKPREEQYNST YRVVSVLTVLHQDWLNGKEYKCAVSNKALPAPIEKTISKA KGQPREPQVYTLPPSRDELTKNQVSLSCAVKGFYPSDIAVE WESNGQPENNYKTTPPVLDSDGSFFLVSKLTVDKSRWQQ GNVFSCSVMHEALHNHYTQKSLSLSPG |
| 48 | Human IgG1 Fc | DKTHTCPPCPAPEAAGAPSVFLFPPKPKDTLMISRTPEVTC VVVDVSHEDPEVKFNWYVDGVEVHNAKTKPREEQYNST YRVVSVLTVLHQDWLNGKEYKCAVSNKALPAPIEKTISKA KGQPREPQVYTLPPSRDELTKNQVSLTCLVKGFYPSDIAVE WESNGQPENNYKTTPPVLDSDGSFFLYSKLTVDKSRWQQ GNVFSCSVMHEALHNHYTQKSLSLSP |
| 49 | Linker-1 | GGGGS |
| 50 | Linker-2 | GGGGSGGGGS |
| 51 | Linker-3 | GGGGSGGGGSGGGGS |
| 52 | 3C6 CDR1(IMGT) | GNIGSIRH |
| 53 | 3C6 CDR2(IMGT) | ITTVGTT |
| 54 | 3C6 CDR3(IMGT) | RATAHPTDY |
| 55 | CD3 HCDR1(Kabat) | TYAMN |
| 56 | CD3 HCDR2(Kabat) | IRSKYNNYATYYADS |
| 57 | CD3 HCDR3-1(Kabat) | HGNFGNNYVSWFAS |
| 58 | CD3 HCDR3-2(Kabat) | HERFGNSYVSWFAY |
| 59 | CD3 HCDR3-3(Kabat) | HGNFGNTYVSWFAM |
| 60 | CD3 LCDR1(Kabat) | SSTGAVTTSNYAN |
| 61 | CD3 LCDR2(Kabat) | GTNKRAP |
| 62 | CD3 LCDR3(Kabat) | LWYSNLWV |
| 63 | 4A7 CDR1-2(IMGT) | GRTFSSYV |
| 64 | CD3-VH4 | EVQLVESGGGLVQPGGSLRLSCAASGFTFNTYAMNWVRQ APGKGLEWVARIRSKYNNYATYYADSVKGRFTISRDDSKS SLYLQMNSLKTEDTAVYYCVRHGNFKNSYGSWFAYWGQ GTLVTVSS |
| 65 | CD3 HCDR3-4(Kabat) | HGNFKNSYGSWFAY |
| 66 | Control antibody | QVQLVESGGGVVQAGGSLTLSCAASGSTFSIRAMRWYRQ APGTERDLVAVIYGSSTYYADAVKGRFTISRDNSKNTLYLQ MNSLRAEDTAVYYCNADTIGTARDYWGQGTLVTVSSGGG GSGGGSEVQLVESGGGLVQPGNSLRLSCAASGFTFSKFGM SWVRQAPGKGLEWVSSISGSGRDTLYADSVKGRFTISRDN AKTTLYLQMNSLRPEDTAVYYCTIGGSLSVSSQGTLVTVSS GGGGSGGGSEVQLVESGGGLVQPGGSLKLSCAASGFTFN KYAINWVRQAPGKGLEWVARIRSKYNNYATYYADQVKD RFTISRDDSKNTAYLQMNNLKTEDTAVYYCVRHANFGNS YISYWAYWGQGTLVTVSSGGGGSGGGGSGGGGSQTVVT QEPSLTVSPGGTVTLTCASSTGAVTSGNYPNWVQQKPGQA PRGLIGGTKFLVPGTPARFSGSLLGGKAALTLSGVQPEDEA EYYCTLWYSNRWVFGGGTKLTVLGGSHHHHHHHH |
| 67 | 260-7-B11 | EVQLVESGGGLVQAGGSLRLSCAASGGTFLTYGMGWFRQ APGKEREFVAAINWSGSMTNYADSVKGRSTISRDNARNTV |
| | | YLQMISLKPEDTAVYYCAAKTGAVAYIQKNEYDYWGQGT QVTVSS |
| 68 | IMGT 260-7-B11 CDR1 | GGTFLTYG |
| 69 | IMGT 260-7-B11 CDR2 | INWSGSMT |
| 70 | IMGT 260-7-B11 CDR3 | AAKTGAVAYIQKNEYDY |
| 71 | Kabat 260-7-B11 CDR1 | TYGMG |
| 72 | Kabat 260-7-B11 CDR2 | AINWSGSMTNYADSVKG |
| 73 | Kabat/ AbM/ Chothia 260-7-B11 CDR3 | KTGAVAYIQKNEYDY |
| 74 | AbM 260-7-B11 CDR1 | GGTFLTYGMG |
| 75 | AbM 260-7-B11 CDR2 | AINWSGSMTN |
| 76 | Chothia 260-7-B11 CDR1 | GGTFLTY |
| 77 | Chothia 260-7-B11 CDR2 | NWSGSM |
| 78 | Contact 260-7-B11 CDR1 | LTYGMG |
| 79 | Contact 260-7-B11 CDR2 | FVAAINWSGSMTN |
| 80 | Contact 260-7-B11 CDR3 | AAKTGAVAYIQKNEYD |
| 81 | VHH amino acid sequence of 7B_11_huGS_HM4 | EVQLVESGGGLVQPGGSLRLSCAASGGTFLTYGMGWFRQ APGKGLEFVAAINWSGSMTNYADSVKGRFTISRDNAKNT VYLQMNSLRAEDTAVYYCAAKTGAVAYIQKNEYDYWGQ GTQVTVSS |
| 82 | VHH amino acid sequence of 7B11_huBSM1 | EVQLVESGGGLVQAGGSLRLSCAASGGTFLTYGMGWFRQ APGKERVFVAAINWSGSMTNYADSVKGRFTISRDNAKNT VYLQMRSLTSEDTAVYYCAAKTGAVAYIQKNEYDYWGQG TQVTVSS |
| 83 | VHH amino acid sequence of 7B11_huBSM_ HM 1 | EVQLVESGGGLVQAGGSLRLSCAASGGTFLTYGMGWFRQ APGKGLVFVAAINWSGSMTNYADSVKGRFTISRDNAKNT VYLQMRSLTSEDTAVYYCAAKTGAVAYIQKNEYDYWGQG TQVTVSS |
| 84 | VHH amino acid sequence of 7B11_huBSM_HM 2 | EVQLVESGGGLVQAGGSLRLSCAASGGTFLTYGMGWFRQ APGKEREFVAAINWSGSMTNYADSVKGRFTISRDNAKNT VYLQMRSLTSEDTAVYYCAAKTGAVAYIQKNEYDYWGQG TLVTVSS |
| 85 | VHH amino acid sequence of 7B11_huGS1 | EVQLVESGGGLVQPGGSLRLSCAASGGTFLTYGMGWVRQ APGKGLEWVAAINWSGSMTNYADSVKGRFTISRDNAKNT VYLQMNSLRAEDTAVYYCAAKTGAVAYIQKNEYDYWGQ GTLVTVSS |
| 86 | VHH amino acid sequence of 7B11_huGS_HM1 | EVQLVESGGGLVQPGGSLRLSCAASGGTFLTYGMGWFRQ APGKGLEFVAAINWSGSMTNYADSVKGRFTISRDNAKNT VYLQMNSLRAEDTAVYYCAAKTGAVAYIQKNEYDYWGQ GTLVTVSS |
| 87 | VHH amino acid sequence of 7B11_huGS_HM2 | EVQLVESGGGLVQAGGSLRLSCAASGGTFLTYGMGWFRQ APGKGLEFVAAINWSGSMTNYADSVKGRFTISRDNAKNT VYLQMNSLRAEDTAVYYCAAKTGAVAYIQKNEYDYWGQ GTLVTVSS |
| 88 | VHH amino acid sequence of 7B11_huGS_HM3 | EVQLVESGGGLVQPGGSLRLSCAASGGTFLTYGMGWFRQ APGKEREFVAAINWSGSMTNYADSVKGRFTISRDNAKNT VYLQMNSLRAEDTAVYYCAAKTGAVAYIQKNEYDYWGQ GTLVTVSS |

The present disclosure is now described with reference to the following examples which are intended to illustrate, but not to limit, the present disclosure.

Those skilled in the art are aware that examples describe the present disclosure by way of example and are not intended to limit the scope of protection claimed by the present disclosure. The experimental methods in the examples, unless otherwise specified, are all routine methods. If the specific conditions are not specified in the examples, the conditions should be carried out according to the conventional conditions or the conditions recommended by the manufacturer. If the manufacturer of the reagents or instruments used is not indicated, they are all conventional products that can be purchased commercially.

### Example 1: Humanization of MSLN antibody molecules, expression of humanized antibody, and dynamic affinity detection

The hFc-tagged human MSLN recombinant protein (huMSLN-hFc, Kactus Biosystems, MSL-HM280) was used as an immune antigen to immunize alpacas, and alpaca nanobody 3C6 having good cross-binding activity for humans, monkeys, and mice, was finally obtained by screening, with the VHH sequence as set forth in SEQ ID NO: 9, and the CDR1-CDR3 defined by IMGT are as set forth in SEQ ID NOs: 52-54 respectively. A humanized antibody of 3C6 was further constructed.

### 1.1 Humanization of MSLN antibody molecules

Anti-MSLN nanobody 3C6 was humanized. The 3C6 sequence was aligned with the IMGT offline database to obtain the germline gene sequence with the highest homology. Using the germline gene sequence as a template, CDR grafting of the target sequence was performed. After grafting was completed, the Vernier region residue of the framework region of the target sequence was subjected to reverse mutation to ensure that the antibody affinity was not affected after humanization. Using the Modeller program, the 3D structure of the humanized sequence was conformed, and the energy contribution of each amino acid residue to the conformational stability of the protein was calculated with the Chimera software. The amino acid residues of high energy were subjected to reverse mutation to enhance the thermal stability of the humanized sequence. After completing the above steps, 8 sequences with different levels of humanization were obtained.

### 1.2 MSLN humanized antibody expression

The 8 sequences with different levels of humanization were respectively constructed at the N-terminus of human IgG1 Fc (SEQ ID NO: 48) by the Linker (SEQ ID NO: 50), then transferred into a PTT5 expression vector (Unibio, lot: VT2202), and transiently expressed by the Expicho-s expression system (Gibco) to obtain a VHH-Fc antibody. The supernatant of expression cells were collected and multiple humanized VHH-Fc antibodies were obtained by protein A chromatographic column purification, in which the humanized VHH-Fc antibodies are named 3C6hu-1, 3C6hu-2, 3C6hu-3, 3C6hu-4, 3C6hu-5, 3C6hu-6, 3C6hu-7, and 3C6hu-8. The variable regions of humanized anti-MSLN nanobodies are as set forth in SEQ ID NOs: 1-8.

### 1.3 Dynamic affinity detection of MSLN humanized antibody molecules

The anti-DYKDDDDK antibody was bound to the HC30M chip via amino coupling, and then 2 µg/mL of MSLN single-domain antibody was captured. After the baseline was stabilized, gradient diluted huMSLN-Fc (Kactus Biosystems, MSL-HM280) and cynoMSLN-Fc (Kactus Biosystems, MSL-CM280) (starting from 100 nM, gradient dilution) were flowed through the chip from low to high concentrations, respectively, with a binding time of 8 minutes and a dissociation time of 20 minutes. Kinetic constants were obtained by fitting with Carteria software. The results of the dynamic affinity of the MSLN single-domain antibody to the MSLN protein are shown in Table 2 below. The results show that the obtained humanized MSLN single-domain antibody has good binding activity to both human and monkey MSLN.

**Table 2. Dynamic affinity of humanized MSLN single-domain antibody to MSLN protein**

| Name of antibody | huM SLN-F c | | | cynoMSLN-Fc | | |
|---|---|---|---|---|---|---|
| | ka (M-1 s-1) | kd (s-1) | KD (M) | ka (M-1 s-1) | kd (s-1) | KD (M) |
| 3C6 | 5.36E+04 | 3.04E-04 | 5.66E-09 | 4.53E+04 | 4.69E-05 | 1.04E-09 |
| 3C6hu-1 | 6.95E+05 | 1.87E-03 | 2.69E-09 | 4.12E+05 | 1.51E-03 | 3.67E-09 |
| 3C6hu-2 | 5.40E+05 | 7.32E-03 | 1.35E-08 | 3.74E+05 | 4.26E-03 | 1.14E-08 |
| 3C6hu-3 | 6.11E+05 | 2.18E-03 | 3.56E-09 | 4.55E+05 | 1.25E-03 | 2.74E-09 |
| 3C6hu-4 | 9.63E+05 | 4.71E-03 | 4.89E-09 | 5.11E+05 | 2.85E-03 | 5.58E-09 |
| 3C6hu-5 | 7.72E+05 | 1.75E-03 | 2.26E-09 | 5.51E+05 | 9.14E-04 | 1.66E-09 |
| 3C6hu-6 | 8.20E+05 | 1.85E-03 | 2.25E-09 | 6.44E+05 | 1.15E-03 | 1.78E-09 |
| 3C6hu-7 | 5.60E+05 | 2.77E-03 | 4.94E-09 | 5.10E+05 | 2.44E-03 | 4.79E-09 |
| 3C6hu-8 | 2.63E+06 | 3.15E-02 | 1.20E-08 | 9.56E+05 | 1.65E-02 | 1.73E-08 |

### Example 2: Preparation of PD-L1 antibodies

### 2.1 PD-L1 immunization in alpacas in vivo

In order to generate a humoral immune response against PD-L1 in alpacas *in vivo,* the alpacas were first immunized by subcutaneous injection with a mixture of complete Freund's adjuvant (CFA) and 0.5 mg of PD-L1-Fc protein (Kactus Biosystems, PDL-HM210), followed by subsequent subcutaneous injection with a mixture of incomplete Freund's adjuvant (IFA) and 0.25 mg of PD-L1-Fc protein. The immunization is conducted for 4 times in total with an interval of every 3 weeks, resulting in the generation of antigen-specific nanobodies in alpacas.

From the start of the second immunization, alpaca serum was taken every 7 days for serum titer monitoring. 2 µg/mL antigen was coated overnight with CBS (carbonate buffer), washed with PBST (phosphate buffered saline), then added with 5% skim milk, and blocked for 1 hour at 37°C. After washing with PBST, serum dilutions (double dilution starting from 1:2000) were added and blocked for 1 hour at 37°C. After washing with PBST, goat anti-Alpaca secondary antibodies labeled with horseradish peroxidase (alpaca S001H, diluted with PBS at 1:1 W) were added and incubated at 37°C for 45 min. After washing with PBST, TMB coloring solution (Thermo, 34029) was added to develop color at 37°C for 5 minutes. The reaction was terminated by adding a stop solution, and the serum titers were determined by measuring the optical density at 450 nm.

### 2.2 Establishment of PD-L1 antibody phage library

50 mL of peripheral blood was collected after the third and fourth immunizations, and PBMCs were separated according to the instructions of sample density separation solution (TBD Science, HY2015) and total RNA was extracted. Total RNAs from peripheral blood after the third and fourth immunizations were extracted using RNAiso Plus reagent (Takara, 9109). cDNA was synthesized and VHH sequences were amplified by slot PCR. The vector pComb3XSS (EditGene, 63890) and the target fragment were respectively digested with SfiI and recovered. The ligated product was electroporated into TG1 competent cells to construct the PD-L1 nanobody library and determine the library capacity, which was 2.15 × 10⁹ CFM. 48 clones were randomly picked from the plate for titer measurement of library transformants for identification, and the results showed an insertion rate of 100%. Library packaging was performed using the helper phage M13KO7 (NEB, N0315S) and phage library titer was determined to be 1.02 × 10¹³ cfu/mL.

### 2.3 Anti-PD-L1 nanobody screening

### 2.3.1 Phage panning

Using a protein magnetic bead panning method, streptavidin magnetic beads (Dynabeads^{™} M-280 Streptavidin) were added with 3% skim milk powder, rotated, and blocked for 1 hour at room temperature. 5 µg of biotinylated PD-L1 (ACRO, PD1-H82E5) antigen was added thereto, and the mixture was rotated and incubated for 1 hour at room temperature to form a magnetic bead protein complex. Then, 60 µL of the VHH phage library was added to the magnetic bead protein complex, and the mixture was rotated and incubated at room temperature for 1 hour, washed 10 times with 0.05% PBST to remove non-specifically bound phages, then TG1 bacteria were infected at 37°C for 1 hour before plating onto 2YT medium plates and cultured overnight. The same screening was operated for 2 rounds to complete the enrichment of positive phages.

### 2.3.2 Specific clone screening

### (1) Periplasmic protein preparation

After the above two rounds of panning, 96 single colonies were picked from the phage-containing bacterial medium and cultured in 2YT medium. IPTG (Sangon, B541007-0001) was added to a final concentration of 1 mM, and cultured at 30°C overnight to induce periplasmic protein expression. The bacterial wall was broken using chicken lysozyme protein, and the periplasmic protein was extracted and used for specific clone screening.

### (2) Enzyme-linked immunosorbent assay (ELISA) screening of positive monoclones

The CBS coating solution was used to respectively coat 2 µg/mL of human PD-L1 protein (ACRO, PD1-H5258), monkey PD-L1 protein (Kactus Biosystems, PDL-CM210), and/or mouse PD-L1 protein (ACRO, PD1-M5251). 50 µL of the periplasmic protein prepared above was added, incubated for 1 hour, washed with 0.05% PBST to remove unbound non-specific clones, added with anti-mouse HA-HRP secondary antibody (GenScript, A01296) and incubated for 1 hour, washed with 0.05% PBST to remove unbound non-specific clones, added with TMB coloring solution and incubated for 1 hour. The microplate reader was used to read at 450 nm.

### (3) T cell signaling pathway blocking assay for screening of positive monoclones

One day in advance, GS-C2 cells (cells overexpressing human PD-L1, GenScript, M00613) were plated at 4 × 10⁴ cells per well onto a white 96-well flat-bottom plate (Corning) and cultured overnight at 37°C, 5% CO₂. 5 µL/well of the above periplasmic protein was taken and added to 45 µL/well of 1640 medium for dilution. The plate plated with GS-C2 cells one day in advance was taken out. The upper layer of culture medium was discarded, and the diluted periplasmic protein was added to the cell plate. Then Jurket-NFAT-PD1 cells (GenScript, M00613) were taken and suspended at 8 × 10⁴ cells per well in 5% 1640 culture medium, added to a white cell plate, mixed well, and cultured at 37°C, 5% CO₂ for 6 hours. After the culture was finished, 100 µL of the detection reagent was added to each well according to the instructions of the Bio-Lite Luciferase Assay System (Vazyme, DD1201), stood at room temperature for 3 min, and the fluorescence signal was detected using an instrument (BMG). Two positive monoclones were obtained, named as 4A7 and 260-7-B11, respectively.

### 2.4 Anti-PD-L1 nanobody expression

Positive clones 4A7 and 260-7-B11 obtained from the above screening were respectively cloned into prokaryotic expression vectors, then the expression of nanobodies (VHH antibodies) was induced by IPTG, and the nanobodies were obtained after purification. The nanobodies were then subjected to DNA sequencing. The sequencing results were analyzed and the CDR regions were defined. The variable region sequences of the anti-PD-L1 nanobodies were obtained as set forth in SEQ ID NOs: 10 and 67, and the CDR sequences are as set forth in the table below.

**Table 3. CDR sequences of PD-L1 nanobodies 4A7 and 260-7-B11**

| Name of antibody | CDRs coding system | CDR1 | CDR2 | CDR3 |
|---|---|---|---|---|
| | | SEQ ID NO: | | |
| 4A7 | IMGT | 11 | 12 | 13 |
| | Kabat | 14 | 15 | 16 |
| | AbM | 17 | 18 | 16 |
| | Chothia | 19 | 20 | 16 |
| | Contact | 21 | 22 | 23 |
| 260-7-B11 | IMGT | 68 | 69 | 70 |
| | Kabat | 71 | 72 | 73 |
| | AbM | 74 | 75 | 73 |
| | Chothia | 76 | 77 | 73 |
| | Contact | 78 | 79 | 80 |

### 2.5 Dynamic affinity detection of anti-PD-L1 nanobodies

### 2.5.1 Dynamic affinity detection of 4A7 nanobody

The anti-DYKDDDDK antibody was bound to the HC30M chip via amino coupling, then 2 µg/mL of 4A7 nanobody was captured. After the baseline was stabilized, gradient diluted human PD-L1 protein (ACRO, PD1-H5258), monkey PD-L1 protein (Kactus Biosystems, PDL-CM210), and mouse PD-L1 protein (ACRO, PD1-M5251) (starting from 100 nM, gradient dilution) were flowed over the chip from low to high concentrations, with a binding time of 8 minutes and a dissociation time of 20 minutes. Kinetic constants were obtained by fitting with Carteria software. The results of the dynamic affinity of 4A7 nanobody to human, mouse, and monkey PD-L1 proteins are shown in the table below.

**Table 4. Dynamic affinity of 4A7 nanobody to human, mouse, and monkey PD-L1 proteins**

| Name of antibody | Human PDL1-Fc | Monkey PDL1-Fc | Mouse PDL1-Fc |
|---|---|---|---|
| | K_{D} (M) | K_{D} (M) | K_{D} (M) |
| 4A7 | 1.47E-07 | 2.21E-07 | 9.91E-08 |

### 2.5.2 Dynamic affinity detection of 260-7-B11 nanobody

The affinity and kinetic data of the 260-7-B11 nanobody to the human PD-L1 protein (ACRO, PD1-H5258) were determined by plasma surface resonance (SPR) technology on a Carterra LSA platform. The measurement was conducted using a capture kinetics process. The test antibody was immobilized on the surface of the NiHC200M chip via His-Tag. After several rounds of blank buffer injection and stabilizing the detection baseline, human PD-L1 protein was injected as the analyte. Each round of injection consisted of 2 minutes of baseline, 8 minutes of binding, and 20 minutes of dissociation. After subtracting the reference point and zero concentration signal (Double Reference) from the original measurement data, the binding curve was fitted by Carterra LSA Kinetics software and the kinetic parameters were calculated. The results of the dynamic affinity of 260-7-B11 nanobody to human PD-L1 protein are shown in the table below.

**Table 5. Dynamic affinity of 260-7-B11 nanobody to human PD-L1 protein**

| Name of antibody | KD (M) |
|---|---|
| 260-7-B11 | 2.43E-11 |

The results in Table 4 and Table 5 show that the 4A7 nanobody can bind to human, mouse, and monkey PD-L1 proteins; the 260-7-B11 nanobody can bind to human PD-L1.

### 2.6 Detection of cell binding ability of anti-PD-L1 nanobody

GS-C2 cells (GenScript, M00613) were taken, washed with PBS, resuspended in 1% BSA, and then added to a 96-well V-bottom plate at a concentration of 1 × 10⁵ cells per well, then added with the gradient diluted 260-7-B11 nanobody (gradient diluted according to the starting concentration of 33.33 nM), incubated on ice for 1 hour, and washed with PBS. Then PE-labeled goat anti-Flag fluorescent secondary antibody was added, and incubated on ice for 30 min. After washing with PBS, a flow cytometer was used for detection. EC₅₀ was fitted with the experimental data by GraphPad Prism. The binding affinity results of 260-7-B11 nanobody to GS-C2 cells expressing human PD-L1 are shown in the table below.

**Table 6. Binding affinity of 260-7-B11 nanobody to GS-C2 cells over-expressing human PD-L1**

| Name of antibody | EC₅₀ (nM) |
|---|---|
| 260-7-B11 | 0.7391 |

It can be seen from the results in Table 6 that the 260-7-B11 nanobody can bind to GS-C2 cells over-expressing human PD-L1.

### 2.7 Detection of the ability of anti-PD-L1 nanobodies to block the binding of PD-L1 to PD1

### 2.7.1 Detection of the ability of 4A7 nanobody to block the binding of PD-L1 to PD1

The huPDL1 sequence (NCBI: Accession # NP_054862.1), mPDL1 (NCBI: Accession # NP_068693), and cynoPDL1 (NCBI: Accession # F6VEW6-1) were respectively cloned into the lentiviral vectors (plvx-IRES-puro, psPAX2, pMD2G). The constructed lentiviral plasmids and packaging plasmids were co-transfected into HEK293T (thermo K1649B) cells, and the cell supernatants were collected at 48 hours. The cell supernatants were respectively added to CHO cells (Thermo, R80007), and 4 µg/mL puromycin was added for screening at 24 hours. Single cells expressing huPDL1, mPDL1, and cynoPDL1 were sorted by a cell sorter (Sony, LESH800SBP), and finally stable CHO-huPDL1, CHO-mPDL1, and CHO-cynoPDL1 cells were obtained.

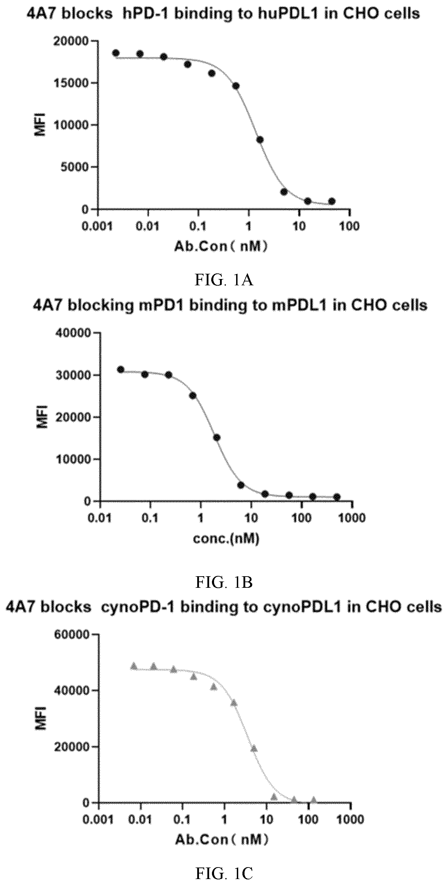

CHO-huPDL1, CHO-mPDL1, and CHO-cynoPDL1 cells were respectively taken, washed with PBS, and resuspended with 1% BSA. Then 1 × 10⁵ cells per well were added to a 96-well V-bottom plate. The gradient diluted anti-PD-L1 nanobody (gradient diluted according to the starting concentration of 33.33 nM) and a constant concentration of 1.25 µg/mL of human PD-1-hFc (ACRO, PD1-H5257), mouse PD-1-hFc (ACRO, PD1-M5251), and monkey PD-1-hFc (ACRO, PD1-C5254) protein premixes were added, followed by incubation on ice for 1 hour. After washing with PBS, PE anti-human Fc (Biolegend, 410708) was added and incubated on ice for 30 min. After washing with PBS, a flow cytometer was used for detection. IC₅₀ was fitted with experimental data by GraphPad Prism. The activity of 4A7 nanobody in blocking the binding of PD-L1 to PD-1 is shown in FIGs. 1A-1C and the table below.

**Table 7. Activity of 4A7 nanobody in blocking the binding of PD-L1 to PD-1**

| Name of antibody | IC₅₀ (nM) | | |
|---|---|---|---|
| | Activity blocking the binding of human PD-L1 to human PD-1 | Activity blocking the binding of mouse PD-L1 to mouse PD-1 | Activity blocking the binding of monkey PD-L1 to monkey PD-1 |
| 4A7 | 1.354 | 1.829 | 3.552 |

It can be seen from the results that the 4A7 nanobody can block the binding of human PD-L1 to human PD-1, can block the binding of mouse PD-L1 to mouse PD-1, and can block the binding of monkey PD-L1 to monkey PD-1.

### 2.7.2 Detection of the ability of 260-7-B11 nanobody to block the binding of PD-L1 to PD1

The monkey PD-L1 (Cyno-PD-L1) sequence (NCBI: Accession # F6VEW6-1) was cloned into the lentiviral vector (plvx-IRES-puro, psPAX2, pMD2G). The constructed lentiviral plasmids and packaging plasmids were co-transfected into HEK293T cells, and the cell supernatants were collected at 48 hours. The cell supernatants were added to 293F cells (Nanjing Cobioer, CBP60437), and 4 µg/mL puromycin was added for screening at 24 hours. Single cells expressing Cyno-PD-L1 were sorted by a cell sorter (Sony, LESH800SBP), and finally stable 293F-cyno PD-L1 cells over-expressing monkey PD-L1 were obtained.

GS-C2 cells (GenScript, M00613) and 293F-cyno PD-L1 cells were respectively taken, washed with PBS, resuspended in 1% BSA, and then added to a 96-well V-bottom plate at a concentration of 1 × 10⁵ cells per well, then respectively added with the gradient diluted anti-PD-L1 nanobody (gradient diluted according to the starting concentration of 33.33 nM) and a constant concentration of 1.25 µg/mL of human PD-1-mFc (ACRO, PD1-H5255) and monkey PD-1-hFc (ACRO, PD1-C5254) protein premixes, incubated on ice for 1 hour, washed with PBS, then added with PE-labeled goat anti-mouse IgG Fc fluorescent secondary antibody, and incubated on ice for 30 min. After washing with PBS, a flow cytometer was used for detection. IC₅₀ was fitted with experimental data by GraphPad Prism. The activity of 260-7-B11 nanobody in blocking the binding of PD-L1 to PD-1 is shown in the table below.

**Table 8. Activity of 260-7-B11 nanobody in blocking the binding of PD-L1 to PD-1**

| Name of antibody | IC₅₀ (nM) | |
|---|---|---|
| | Activity blocking the binding of human PD-L1 to human PD-1 | Activity blocking the binding of monkey PD-L1 to monkey PD-1 |
| 260-7-B11 | 0.3962 | 0.6266 |

It can be seen from the results in Table 8 that the 260-7-B11 nanobody can block the binding of human PD-L1 to human PD-1 and can block the binding of monkey PD-L1 to monkey PD-1.

### 2.8 T cell PD-1/PD-L1 signaling pathway blocking activity screening detection

One day in advance, GS-C2 cells (GenScript, M00613) were plated at 4 × 10⁴ cells per well onto a white 96-well flat-bottom plate (Corning) and cultured overnight at 37°C, 5% CO₂. The plate plated with GS-C2 cells one day in advance was taken out, then the upper layer of the culture medium was discarded, and the gradient diluted anti-PD-L1 nanobody (gradient diluted according to the starting concentration of 266.67 nM) was added. Then Jurket-NFAT-PD1 cells (GenScript, M00613) were suspended at 8 × 10⁴ cells per well in 5% 1640 culture medium, added to a white cell plate, mixed well, and cultured at 37°C, 5% CO₂ for 6 hours. After the culture was finished, 100 µL of the detection reagent was added to each well according to the instructions of the Bio-Lite Luciferase Assay System (Vazyme, DD 1201), left at room temperature for 3 min, and the fluorescence signal was detected using an instrument (BMG). The results of 260-7-B11 nanobody blocking T cell PD-1/PD-L1 signaling pathway activity are shown in the table below.

**Table 9. Activity of 260-7-B11 nanobody in blocking T cell PD-1/PD-L1 signaling pathway**

| Name of antibody | EC₅₀ (nM) |
|---|---|
| 260-7-B11 | 15.32 |

It can be seen from the results in Table 9 that the 260-7-B11 nanobody can block the activity of the PD-1/PD-L1 signaling pathway of T cells.

### 2.9 Humanization of anti-PD-L1 nanobodies

Anti-PD-L1 nanobodies 4A7 and 260-7-B11 were humanized. The 4A7 and 260-7-B11 sequences were aligned with the IMGT offline database to obtain the germline gene sequence with the highest homology. Using the germline gene sequence as a template, CDR grafting of the target sequence was performed. After grafting was completed, the Vernier region residue of the framework region of the target sequence was subjected to reverse mutation to ensure that the antibody affinity was not affected after humanization. Using the Modeller program, the 3D structure of the humanized sequence was conformed, and the energy contribution of each amino acid residue to the conformational stability of the protein was calculated with the Chimera software. The amino acid residues of high energy were subjected to reverse mutation to enhance the thermal stability of the humanized sequence. After completing the above steps, several sequences with different levels of humanization could be obtained. Among them, 4A7 obtained 16 sequences with different levels of humanization; 260-7-B11 obtained 8 sequences with different levels of humanization.

### 2.10 Expression of humanized PDL1 antibody

The 16 sequences of the above 4A7 with different levels of humanization were respectively constructed into the N-terminus of human IgG1 Fc (SEQ ID NO: 48) by the Linker (SEQ ID NO: 50), and then transferred into the PTT5 expression vector (Unibio, lot: VT2202); 8 sequences of 260-7-B11 with different levels of humanization were respectively constructed into the N-terminus of human IgG1 Fc (SEQ ID NO: 48) by the Linker (SEQ ID NO: 50), and then transferred into PTT5 expression vector (Unibio, lot: VT2202), and transiently expressed by Expicho-s expression system (Gibco) to obtain the VHH-Fc antibody. The supernatant of the expressing cells was collected multiple humanized VHH-Fc antibodies were obtained through protein A chromatography column purification. Among them, 16 of the 4A7 humanized VHH-Fc antibodies, named 4A7hu-1, 4A7hu-2, 4A7hu-3, 4A7hu-4, 4A7hu-5, 4A7hu-6, 4A7hu-7, 4A7hu-8, 4A7hu-9, 4A7hu-10, 4A7hu-11, 4A7hu-12, 4A7hu-13, 4A7hu-14, 4A7hu-15 and 4A7hu-16, having variable region (VHH) sequences as set forth in SEQ ID NOs: 24-39. 8 of 260-7-B11 humanized VHH-Fc antibodies, named 7B11_huGS_HM4, 7B11_huBSM1, 7B11_huBSM_HM1, 7B11_huBSM_HM2, 7B11_huGS1, 7B11_huGS_HM1, 7B11_huGS_HM2, and 7B11_huGS_HM3, having variable region (VHH) sequences as set forth in SEQ ID NOs: 81-88. Among them, 7B11 represents a VHH-Fc antibody obtained by fusing the sequence of 260-7-B11 and human IgG1 Fc (SEQ ID NO: 48) according to the method of this example.

### 2.11 Dynamic affinity detection of humanized PD-L1 nanobodies

### 2.11.1 Dynamic affinity detection of 4A7 humanized nanobody

The anti-DYKDDDDK antibody was bound to the HC30M chip via amino coupling, then 2 µg/mL of humanized PD-L1 single-domain antibody was captured. After the baseline was stabilized, gradient diluted human PD-L1 protein (ACRO, PD1-H5258), monkey PD-L1 protein (Kactus Biosystems, PDL-CM210), and mouse PD-L1 protein (ACRO, PD1-M5251) (starting from 100 nM, gradient dilution) were flowed over the chip from low to high concentrations, with a binding time of 8 minutes and a dissociation time of 20 minutes. Kinetic constants were obtained by fitting with Carteria software. The results are shown in the table below. These humanized single-domain antibodies have good cross-activity with human, mouse, and monkey PD-L1 proteins.

**Table 10. Dynamic affinity of humanized single-domain antibodies to human, mouse, and monkey PD-L1 proteins**

| Name of antibody | Human PDL1-Fc | Monkey PDL1-Fc | Mouse PDL1-Fc |
|---|---|---|---|
| | KD (M) | KD (M) | KD (M) |
| 4A7hu-1 | NA | 1.32E-06 | 1.43E-07 |
| 4A7hu-2 | NA | 3.67E-07 | 4.08E-07 |
| 4A7hu-3 | 1.63E-06 | 5.99E-07 | 1.22E-07 |
| 4A7hu-4 | 1.75E-07 | 1.12E-07 | 2.10E-08 |
| 4A7hu-5 | 1.41E-07 | 9.43E-08 | 9.72E-09 |
| 4A7hu-6 | 1.36E-07 | 9.90E-08 | 9.59E-09 |
| 4A7hu-7 | 1.27E-07 | 6.17E-08 | 9.76E-09 |
| 4A7hu-8 | 9.27E-08 | 5.75E-08 | 1.04E-08 |
| 4A7hu-9 | 6.52E-07 | 6.09E-07 | 1.46E-07 |
| 4A7hu-10 | 8.05E-07 | 6.22E-07 | 1.97E-07 |
| 4A7hu-11 | 7.96E-07 | 8.28E-07 | 1.54E-07 |
| 4A7hu-12 | 7.90E-08 | 9.45E-08 | 1.95E-08 |
| 4A7hu-13 | 5.87E-08 | 7.28E-08 | 1.07E-08 |
| 4A7hu-14 | 6.09E-08 | 7.65E-08 | 9.70E-09 |
| 4A7hu-15 | 5.49E-08 | 6.07E-08 | 9.97E-09 |
| 4A7hu-16 | 4.51E-08 | 7.53E-08 | 9.82E-09 |

### 2.11.2 Dynamic affinity detection of 260-7-B11 humanized nanobodies

The anti-DYKDDDDK antibody was bound to the HC30M chip via amino coupling, then 2 µg/mL of humanized PD-L1 single-domain antibody was captured. After the baseline was stabilized, gradient diluted human PD-L1 protein (ACRO, PD1-H5258) and monkey PD-L1 protein (Kactus Biosystems, PDL-CM210) (starting from 100 nM, gradient dilution) were flowed over the chip from low to high concentrations, with a binding time of 8 minutes and a dissociation time of 20 minutes. Kinetic constants were obtained by fitting with Carteria software. The results are shown in the table below. These humanized single-domain antibodies have good cross-activity with human and monkey PD-L1 proteins.

**Table 11. Dynamic affinity detection of humanized anti-PD-L1 nanobodies**

| Name of antibody | Human PDL1-Fc | Monkey PDL1-Fc |
|---|---|---|
| | KD (M) | KD (M) |
| 7B11_huBSM1 | 3.72E-11 | 2.94E-11 |
| 7B11_huBSM_HM1 | 3.82E-11 | 3.47E-11 |
| 7B11_huBSM_HM2 | 3.62E-11 | 3.07E-11 |
| 7B11_huGS1 | 8.15E-11 | 5.88E-11 |
| 7B11_huGS_HM1 | 2.94E-11 | 2.52E-11 |
| 7B11_huGS_HM4 | 3.50E-11 | 3.26E-11 |

It can be seen from Table 11 that the humanized single-domain antibodies in the table can bind to human and monkey PD-L1 proteins well.

### 2.12 Cell binding activity detection of humanized anti-PD-L1 nanobodies

### 2.12.1 Binding activity with human PD-L1 cells

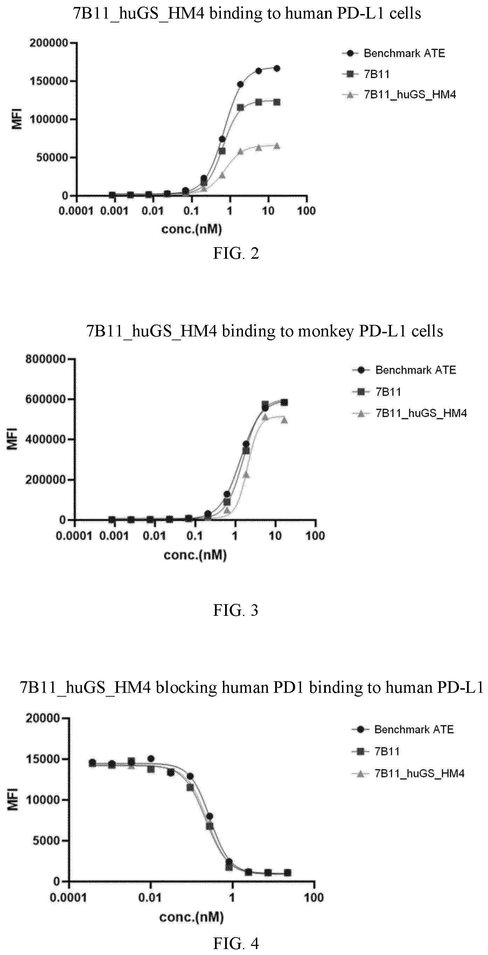

GS-C2 cells (GenScript, M00613) were taken, washed with PBS, resuspended in 1% BSA, and then added to a 96-well V-bottom plate at a concentration of 1 × 10⁵ cells per well, then added with the gradient diluted 7B11_huGS_HM4, 7B11, and control antibody Benchmark-ATE (monoclonal antibody, Atezolizumab) (gradient diluted according to the starting concentration of 16.67 nM), incubated on ice for 1 hour, washed with PBS, then added with PE-labeled goat anti-human IgG Fc fluorescent secondary antibody, and incubated on ice for 30 min. After washing with PBS, a flow cytometer was used for detection. The data were fitted by GraphPad Prism and the results are shown in FIG. 2.

### 2.12.2 Cross-binding activity with monkey PD-L1 cells

293F-cyno PD-L1 cells (293F, over-expressing monkey PD-L1) were taken, washed with PBS, resuspended in 1% BSA, and then added to a 96-well V-bottom plate at a concentration of 1 × 10⁵ cells per well, then added with the gradient diluted 7B11_huGS_HM4, 7B11, and Benchmark-ATE (gradient diluted according to the starting concentration of 16.67 nM), incubated on ice for 1 hour, washed with PBS, then added with PE-labeled goat anti-human IgG Fc fluorescent secondary antibody, and incubated on ice for 30 min. After washing with PBS, a flow cytometer was used for detection. The data were fitted by GraphPad Prism and the results are shown in FIG. 3. The cell binding activity results of 7B11_huGS_HM4 and 7B11 nanobodies are shown in Table 12 and FIGs. 2-3.

**Table 12. Cell binding activity of humanized anti-PD-L1 nanobodies**

| Name of antibody | Binding activity EC₅₀ (nM) to cells over-expressing human PD-L1 | Binding activity EC₅₀ (nM) to cells over-expressing monkey PD-L1 |
|---|---|---|
| Benchmark-ATE | 0.694 | 1.344 |
| 7B11 | 0.6418 | 1.578 |
| 7B11_huGS_HM4 | 0.7289 | 1.998 |

As can be seen from FIGs. 2-3 and Table 12, 7B11 and 7B11_huGS_HM4 have excellent binding activity to cells over-expressing human and monkey PD-L1.

### 2.13 Blocking activity of humanized anti-PD-L1 nanobodies against human PD-L1 and PD-1

GS-C2 cells (GenScript, M00613) were taken, washed with PBS, resuspended in 1% BSA, and then added to a 96-well V-bottom plate at a concentration of 1 × 10⁵ cells per well, then added with the gradient diluted 7B11_huGS_HM4, 7B11, and Benchmark-ATE (gradient diluted according to the starting concentration of 22.22 nM) and a constant concentration of 1.25 µg/mL of human PD-1-mFc protein premixes, incubated on ice for 1 hour, washed with PBS, then added with PE-labeled goat anti-mouse IgG Fc fluorescent secondary antibody, and incubated on ice for 30 min. After washing with PBS, a flow cytometer was used for detection. The data were fitted by GraphPad Prism, and the results are shown in FIG. 4 and Table 13.

**Table 13. Blocking activity of humanized anti-PD-L1 nanobodies**

| Name of antibody | Blocking activity IC₅₀ (nM) against human PD-L1 and PD-1 |
|---|---|
| Benchmark-ATE | 0.2799 |
| 7B11 | 0.2206 |
| 7B11_huGS_HM4 | 0.2397 |

It can be seen from FIG. 4 and Table 13 that 7B11 and 7B11_huGS_HM4 can block the binding of PD-L1 and PD-1 better.

### 2.14 Detection of blocking activity of humanized anti-PD-L1 nanobodies in T cell PD-1/PD-L1 signaling pathway

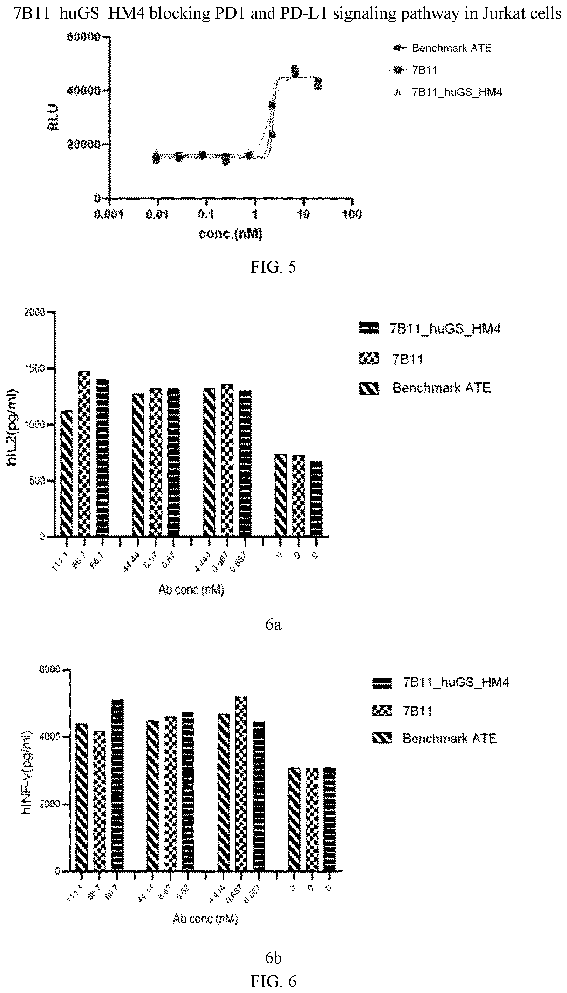

One day in advance, GS-C2 cells (GenScript, M00613) were plated at 4 × 10⁴ cells per well in a white 96-well flat-bottom plate (Corning) and cultured overnight at 37°C with 5% CO₂. The plate plated with GS-C2 cells one day in advance was taken out, then the upper layer of the culture medium was discarded, and the gradient diluted 7B11_huGS_HM4, 7B11, and Benchmark-ATE (gradient diluted according to the starting concentration of 20 nM) was added. Then Jurket-NFAT-PD1 cells (GenScript, M00613) were suspended at 8 × 10⁴ cells per well in 5% 1640 culture medium, added to a white cell plate, mixed well, and cultured at 37°C and 5% CO₂ for 6 hours. After the culture was finished, 100 µL of the detection reagent was added to each well according to the instructions of the Bio-Lite Luciferase Assay System (Vazyme), left at room temperature for 3 min, and the fluorescence signal was detected using an instrument (BMG). The results are shown in Table 14 and FIG. 5.

**Table 14. Activity of humanized anti-PD-L1 nanobody in blocking T cell PD-1/PD-L1 signaling pathway**

| Name of antibody | EC₅₀ (nM) |
|---|---|
| Benchmark-ATE | 2.376 |
| 7B11 | 2.11 |
| 7B11_huGS_HM4 | 1.973 |

The results in Table 14 and FIG. 5 show that 7B11_huGS_HM4 and 7B11 can block the PD-1/PD-L1 signaling pathway of T cells well.

### 2.15 Effect of humanized anti-PD-L1 nanobodies on T cell activation in lymphocyte response

DC cells (OriBiotech, FPB-DC002F-C) and PBMC cells (OriBiotech, FPB004F-C-MLR) were resuscitated. DC cells (according to 1 × 10⁴ cells per well) were mixed with PBMC cells (according to 1 × 10⁵ cells per well), with a total volume of 200 µL per well, and added to a 96-well flat-bottom plate. The 96-well plate was divided into three groups, with Benchmark-ATE, 7B11, and 7B11_huGS_HM4 added to each group respectively. Different concentrations of Benchmark-ATE (111.1 nM, 44.44 nM, 4.444 nM, and 0 nM) were added to each well of the Benchmark-ATE group. Different concentrations of 7B11 (66.7 nM, 6.67 nM, 0.667 nM, and 0 nM) were added to each well of the 7B11 group. Different concentrations of 7B11_huGS_HM4 (66.7 nM, 6.67 nM, 0.667 nM, and 0 nM) were added to each well of the 7B11_huGS_HM4 group. The 96-well plate was placed at 37°C, 5% CO₂ for 5 days, and the concentrations of IL2 and IFNγ were detected using HUMAN IL2 KITS (Cisbio Bioassays, 62HIL02PEG) and HUMAN IFNγ KITS (Cisbio Bioassays, 62HIFNGPEG).

The results are shown in FIG. 6a and FIG. 6b. FIG. 6a shows the detection results of Benchmark-ATE, 7B11, and 7B11_huGS_HM4 activating T cells to produce IL2; FIG. 6b shows the detection results of Benchmark-ATE, 7B11, and 7B11_huGS_HM4 activating T cells to produce IFNγ. It can be seen from the results that 7B11 and 7B11_huGS_HM4 can stimulate the donor to produce cytokines, indicating that 7B11 and 7B11_huGS_HM4 can effectively activate T cells.

### Example 3: Preparation of CD3-PDL1-MSLN trispecific antibody

### 3.1 Construction of CD3-PDL1-MSLN trispecific antibody

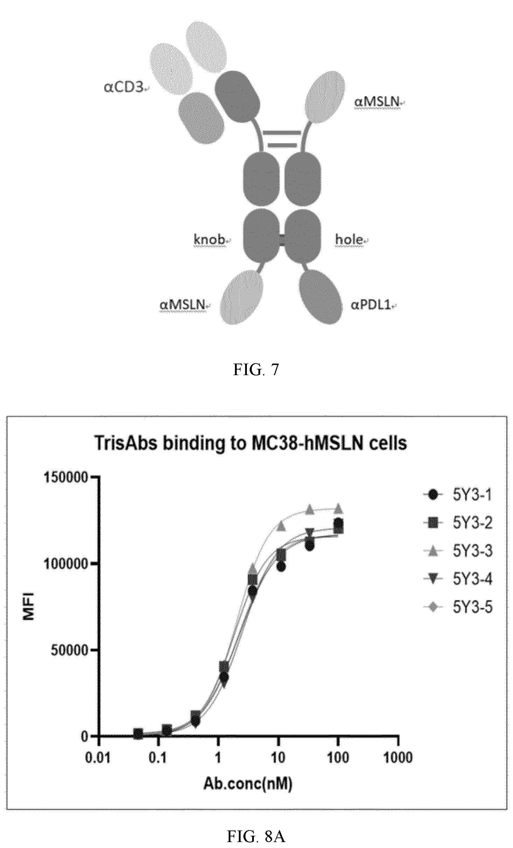

The structure of the CD3-PDL1-MSLN trispecific antibody in this example is shown in FIG. 7. It consists of three chains: 1. light chain, consisting of a CD3 antibody light chain variable region and a light chain CL1 constant region (SEQ ID NO: 44); 2. heavy chain 1, having a humanized CD3 heavy chain variable region, mutant 1 of IgG1 (SEQ ID NO: 45) linked at C-terminus to a humanized MSLN single-domain antibody by (GGGGS)3 in order; 3. heavy chain 2, having a humanized MSLN single-domain antibody linked to mutant 2 of IgG1 (SEQ ID NO: 47) at N-terminus by (GGGGS)1, and the C-terminus of mutant 2 of IgG1 linked to a PD-L1 single-domain antibody by (GGGGS)3 in order; wherein the CD3 heavy chain variable region is selected from VH1 (SEQ ID NO: 40), VH2 (SEQ ID NO: 41), and VH3 (SEQ ID NO: 42); and the CD3 light chain variable region is VL (SEQ ID NO: 43). Specifically, a total of 16 trispecific antibodies (5Y3-1 to 5Y3-15, 5Y3-16) were obtained, and their structures are shown in the table below; the nucleic acid sequences encoding the above 16 polypeptide chains were constructed into the PTT5 plasmid vectors (Unibio, lot: VT2202), and enough plasmids were respectively extracted for later use.

**Table 15. Structure of trispecific antibodies**

| | Trispecific antibody sequence combination | | | | | | | | | | | | | | | |
|---|---|---|---|---|---|---|---|---|---|---|---|---|---|---|---|---|
| Na me | 5Y 3-1 | 5Y 3-2 | 5Y 3-3 | 5Y 3-4 | 5Y 3-5 | 5Y 3-6 | 5Y 3-7 | 5Y 3-8 | 5Y 3-9 | 5Y 3-10 | 5Y 3-11 | 5Y 3-12 | 5Y 3-13 | 5Y 3-14 | 5Y 3-15 | 5Y3-16 |
| CD 3-V H | VH 1 | VH 2 | VH 3 | VH 1 | VH 2 | VH 3 | VH 1 | VH 2 | VH 3 | VH 1 | VH 2 | VH 3 | VH 1 | VH 2 | VH 3 | VH1 |
| PD L1 | 4A7hu-6 (SEQ ID NO: 29) | | | 4A7hu-7 (SEQ ID NO: 30) | | | 4A7hu-15 (SEQ ID NO: 38) | | | 4A7hu-8 (SEQ ID NO: 31) | | | 4A7hu-16 (SEQ ID NO: 39) | | | 7B11 _huG S_H M4 (SEQ ID NO: 81) |
| M SL N | 3C6hu-8 (SEQ ID NO: 8) | | | | | | | | | | | | | | | |
| CD 3-VL | VL (SEQ ID NO: 43) | | | | | | | | | | | | | | | |

### 3.2 Expression and purification of CD3-PDL1-MSLN trispecific antibody

The day before transfection (D1), the cell density was diluted to 2 × 10⁶ cells/mL with culture medium; the day of transfection (D₀), the cells were counted (cell viability should be ≥ 95%), and the cell density was adjusted to 4.0 × 10⁶ cells/mL, and the plasmids of the antibody fragments were mixed at a ratio of heavy chain 1: heavy chain 2: light chain = 1:1:1, then mixed with PEI, co-transfected with CHO-S cells (Gibco A29127), after which cells were transferred to a 37°C, 120 rpm, 8% CO₂ incubator for culturing. The day after transfection (D1), prewarmed CHO gro (Mirus 6200A) complete medium was supplemented in an amount of 1/5 of the expression volume, cooled to 32°C and the culture was continued. Advanced CHO Feed1 (Sigma 24367C) was supplemented at 8%, 5%, and 5% on the 2nd, 4th, and 6th day after transfection, and the cell viability was monitored every day from D7, and the cells were harvested when the viability was < 80%. The mixture was centrifuged to obtain the supernatant and purified with protein A column for later use.

### Example 4: Verification of binding activity of CD3-PDL1-MSLN trispecific antibody

### 4.1 Binding of CD3-PDL1-MSLN trispecific antibody to human Jurkat cells

(1) Jurkat cells (Company: ATCC; catalog number: TIB-152) were adjusted to 1 × 10⁷ cells/mL with PBS, and 50 µL of cell suspension was prepared for each sample.
(2) For 5Y3-1 to 5Y3-15, the final concentration was adjusted to 2 µg/mL with PBS, and then a three-fold gradient dilution was performed, resulting in 8 gradients; for 5Y3-16, the final concentration was adjusted to 2000 nM with PBS, and then a three-fold gradient dilution was performed, with 7 gradients. Each well was filled with 50 µL of antibody.
(3) 50 µL of cell suspension was taken, and the total number of cells was 5 × 10⁵ cells/well. For 5Y3-1 to 5Y3-15, the final concentration of the antibody was the starting concentration of 2 µg/mL, and a three-fold gradient dilution was performed across 8 wells, incubated at 4°C for 1 hour. For 5Y3-16, the final concentration of the antibody was the starting concentration of 2000 nM, and a three-fold gradient dilution was performed across 7 wells, incubated at 4°C for 1 hour.
(4) 500 g, 5 minutes, the supernatant was gently removed, and the residue was washed twice with 200 µL of PBS.
(5) Addition of secondary antibody PE anti-human Fc (Biolegend 410708): The fluorescent secondary antibody, diluted with PBS (1: 100), was added and incubated at 4°C for 30 minutes.
(6) The fluorescent secondary antibody was washed, 500 g for 5 minutes, then the supernatant was gently removed, and the residue was washed once with 200 µL of PBS. Flow cytometry was then performed.

The results are shown in the table below, and the constructed trispecific antibody can bind to Jurkat cells expressing CD3.

**Table 16. Binding results of trispecific antibodies to human Jurkat cells**

| Name of antibody | 5Y3-1 | 5Y3-2 | 5Y3-3 | 5Y3-4 | 5Y3-5 | 5Y3-6 | 5Y3-7 | 5Y3-8 |
|---|---|---|---|---|---|---|---|---|
| EC₅₀ (nM) | 24.87 | 52.91 | 15.48 | 38.29 | 13.44 | 38.25 | 47.81 | 26.22 |
| Name of antibody | 5Y3-9 | 5Y3-10 | 5Y3-11 | 5Y3-12 | 5Y3-13 | 5Y3-14 | 5Y3-15 | 5Y3-16 |
| EC₅₀ (nM) | 23.38 | 50.89 | 12.74 | 12.42 | 35.97 | 9.314 | 11.87 | 31.01 |

### 4.2 Binding of CD3-PDL1-MSLN trispecific antibody to human MC38/MSLN cells

Human MC38/MSLN cell construction: The human MSLN (huMSLN) full-length sequence (cDNA purchased from Sino Biological, HG13128-UT) was cloned into a lentiviral vector. The constructed lentiviral plasmids and packaging plasmids were co-transfected into HEK293T cells. The cell supernatants were respectively collected at 48 hours and 72 hours, and then successively added to MC38 cells (purchased from Nanjing Cobioer, Catalog number: CBP60825). 4 µg/mL puromycin was added 24 hours later for screening. Single cells highly expressing huMSLN were sorted using a cell sorter (Sony, LE-SH800SBP), and finally the stable-transformed cell monoclonal MC38/MSLN highly expressing huMSLN was obtained.

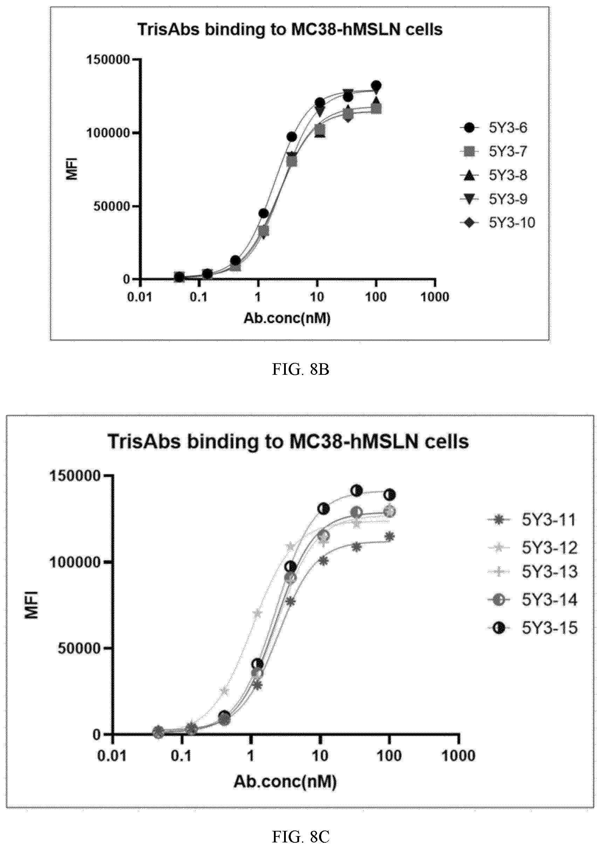

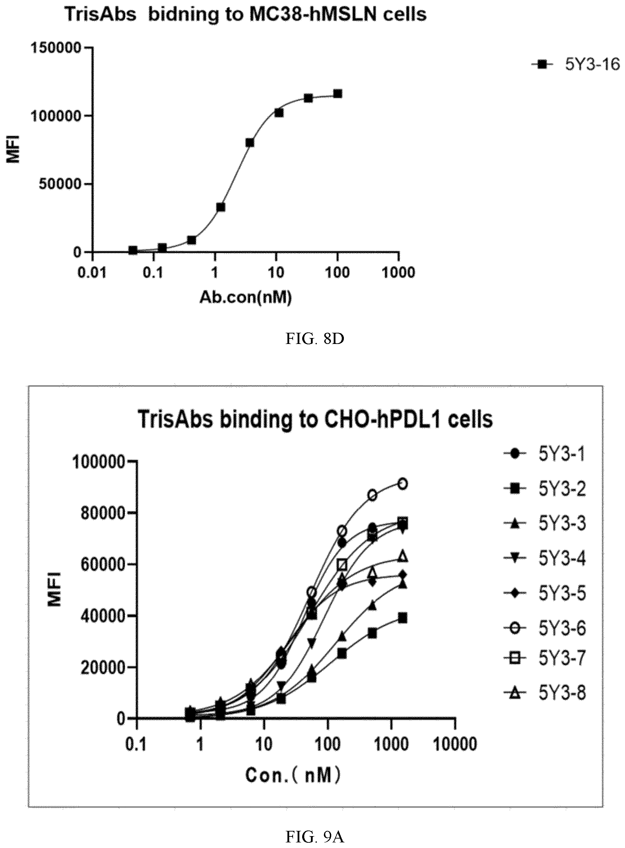

50 µL of MC38/MSLN cells (2 × 10⁵ cells) were added to a 96-well V-shaped plate. Then, 50 µL of gradient-diluted antibody was added to each well (antibody gradient diluted starting from 100 nM), and incubated on ice for 1 hour. After washing away the primary antibody, PE-labeled goat anti-mouse IgG Fc fluorescent secondary antibody was added, incubated on ice for 1 hour, washed, and then resuspended in 200 µL of PBS per well, and a flow cytometer was used for detection. EC₅₀ values were calculated using GraphPad Prism software, and the results are shown in FIGs. 8A-8D. The results show that the constructed trispecific antibody can bind to cells expressing MSLN.

### 4.3 Binding of CD3-PDL1-MSLN trispecific antibody to CHO-hPDL1 cells

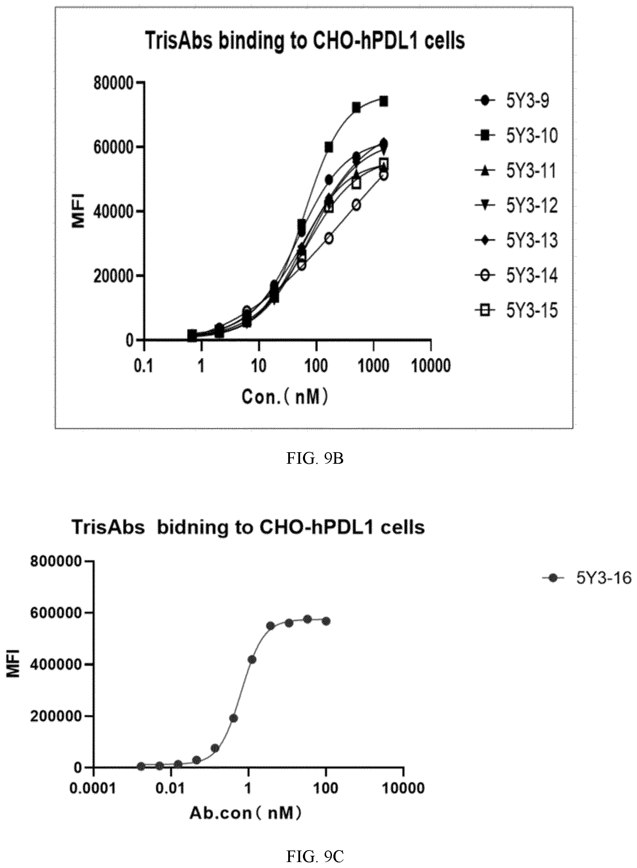

CHO-huPDL1 cells were taken, washed with PBS, and resuspended in 1% BSA. Then, 1 × 10⁵ cells per well were added to a 96-well V-bottom plate. Then, gradient diluted anti-CD3-PDL1-MSLN trispecific antibodies (for 5Y3-1 to 5Y3-15, starting at 1500 nM with a 3-fold gradient dilution for a total of 8 gradients; for 5Y3-16, starting at 100 nM with a 3-fold gradient dilution for a total of 11 gradients) were added and incubated on ice for 1 hour. After washing with PBS, PE-labeled goat anti-mouse IgG Fc secondary antibodies were added and incubated on ice for 30 minutes. After washing with PBS, a flow cytometer was used for detection. EC₅₀ was fitted with experimental data by GraphPad Prism, and the results are shown in FIGs. 9A-9C. The results show that the constructed trispecific antibody can bind to cells expressing PD-L1.

### Example 5: CD3-PDL1-MSLN trispecific antibody blocking the binding of human PD1 to human PD-L1

CHO-huPDL1 cells were taken, washed with PBS, resuspended in 1% BSA, and then added to a 96-well V-bottom plate at a concentration of 1 × 10⁵ cells per well, then added with the gradient diluted CD3-PDL1-MSLN trispecific antibody (for 5Y3-1 to 5Y3-15, starting at 1500 nM with a 3-fold gradient dilution for a total of 8 gradients; for 5Y3-16, starting at 50 nM for a gradient dilution) and a constant concentration of 1.25 µg/mL of human PD-1-mFc (ACRO, PD1-H5255) protein premixes, incubated on ice for 1 hour, washed with PBS, then added with PE-labeled goat anti-mouse IgG Fc fluorescent secondary antibody, and incubated on ice for 30 min. After washing with PBS, a flow cytometer was used for detection. EC₅₀ was fitted with the experimental data by GraphPad Prism.

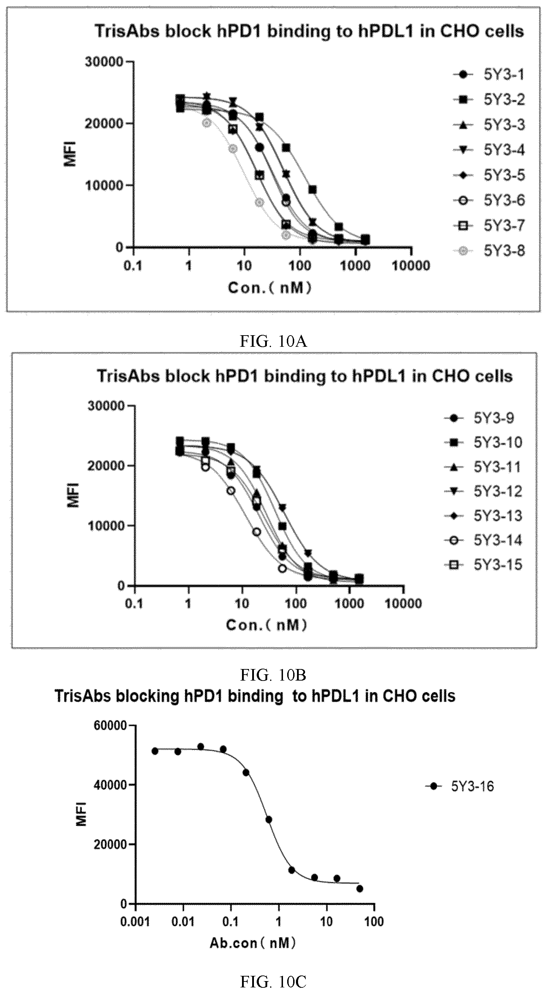

The results are shown in FIGs. 10A-10C. The constructed trispecific antibody can effectively block the binding of human PD1 to human PD-L1.

### Example 6: Dynamic affinity detection of CD3-PDL1-MSLN trispecific antibody

Antibodies at three concentrations of 4, 2, and 1 µg/mL were immobilized on the HC200M chip containing anti-human IgG Fc secondary antibody, and the antibody binding amount was controlled to approximately 1000 RU. After the baseline was stabilized, the gradient diluted human CD3εγ (Company: acrobiosystems, catalog number: CDG-H52W5), human MSLN-Fc (Kactus Biosystems, MSL-HM280), and human PD-L1 protein (ACRO, PD1-H5258) (starting from 592.5 nM, 3-fold dilution to 8 gradients) were flowed over the chip at a rate of 1000 µL/min, with a binding time of 8 min and a dissociation time of 20 min. Using Carterra's built-in Kinetics software, the kinetic constants were fitted with a 1:1 binding model. The results are shown in the table below. The constructed trispecific antibody has good affinity to human CD3εγ, human MSLN, and human PD-L1.

**Table 17. Affinity determination of trispecific antibodies to human CD3εγ, human MSLN, and human PD-L1**

| Name of antibody | Human CD3εγ | Human MSLN | Human PD-L 1 |
|---|---|---|---|
| | KD (M) | | |
| 5Y3-1 | 4.74E-07 | 4.58E-09 | 3.93E-07 |
| 5Y3-2 | 1.95E-07 | 4.66E-09 | 3.99E-07 |
| 5Y3-3 | 2.60E-07 | 4.65E-09 | 3.96E-07 |
| 5Y3-4 | 6.21E-07 | 4.60E-09 | 4.33E-07 |
| 5Y3-5 | 2.19E-07 | 4.66E-09 | 3.66E-07 |
| 5Y3-6 | 2.77E-07 | 4.52E-09 | 3.44E-07 |
| 5Y3-7 | 5.52E-07 | 4.53E-09 | 1.33E-07 |
| 5Y3-8 | 2.28E-07 | 4.30E-09 | 1.40E-07 |
| 5Y3-9 | 2.68E-07 | 4.18E-09 | 1.19E-07 |
| 5Y3-10 | 5.95E-07 | 4.45E-09 | 3.70E-07 |
| 5Y3-11 | 2.45E-07 | 4.38E-09 | 3.62E-07 |
| 5Y3-12 | 3.42E-07 | 4.24E-09 | 3.80E-07 |
| 5Y3-13 | 7.59E-07 | 4.38E-09 | 1.46E-07 |
| 5Y3-14 | 2.57E-07 | 8.38E-09 | 1.28E-07 |
| 5Y3-15 | 2.76E-07 | 8.21E-09 | 1.33E-07 |
| 5Y3-16 | 4.64E-07 | 7.98E-09 | 1.21E-10 |

### Example 7: Activation of T cell activation signaling pathway by CD3-PDL1-MSLN trispecific antibody

### (1) Tumor-free group

For 5Y3-1 to 5Y3-15, the antibody was diluted to 2 µg/mL with 1640 + 10% FBS, gradient diluted, and 50 µL of the diluted antibody was added per well. Jurkat-NFAT-luc cells (Nanjing Cobioer, Catalog number: CBP74020) in logarithmic growth phase were adjusted to 6 × 10⁵ cells/mL with 1640 + 10% FBS, and 50 µL of cells (3 × 10⁴ cells/well) were added per well. After gentle mixing, the plate was incubated at 37°C, 5% CO₂ for 5-6 hours. 20 µL/well of chemiluminescent substrate was added for detection using an instrument.

For 5Y3-16, the antibody was diluted to 5 nM with 1640 + 10% FBS, gradient diluted, and 50 µL of the diluted antibody was added per well. Jurkat-NFAT-luc cells (Nanjing Cobioer, Catalog number: CBP74020) in logarithmic growth phase were adjusted to 1.6 × 10⁶ cells/mL with 1640 + 10% FBS, and 50 µL of cells (8 × 10⁴ cells/well) were added per well. After gentle mixing, the plate was incubated at 37°C, 5% CO₂ for 5-6 hours. 20 µL/well of chemiluminescent substrate was added for detection using an instrument.

### (2) Tumor group

For 5Y3-1 to 5Y3-15, the antibody was diluted to 2 µg/mL with 1640 + 10% FBS, gradient diluted, and 50 µL of diluted antibody was added to each well. Jurkat-NFAT-luc cells in logarithmic growth phase were adjusted to 1.2 × 10⁶ cells/mL with 1640 + 10% FBS, and 25 µL of cells (3 × 10⁴ cells/well) were added to each well. MC38/MSLN cells constructed in the Example 4 were digested and adjusted to 1.2 × 10⁶ cells/mL with 1640 + 10% FBS after digestion. 25 µL of cells (3 × 10⁴ cells/well) were added to each well, gently mixed, and then incubated at 37°C, 5% CO₂ for 5-6 hours. 20 µL/well of chemiluminescent substrate was added for detection using an instrument.

For 5Y3-16, the antibody was diluted to 1 nM with 1640 + 10% FBS, gradient diluted, and 50 µL of diluted antibody was added to each well. Jurkat-NFAT-luc cells in logarithmic growth phase were adjusted to 3.2 × 106 cells/mL with 1640 + 10% FBS, and 25 µL of cells (8 × 10⁴ cells/well) were added to each well. MC38/MSLN cells constructed in the Example 4 were digested and adjusted to 1.6 × 106 cells/mL with 1640 + 10% FBS after digestion. 25 µL of cells (4 × 10⁴ cells/well) were added to each well, gently mixed, and then incubated at 37°C, 5% CO₂ for 5-6 hours. 20 µL/well of chemiluminescent substrate was added for detection using an instrument.

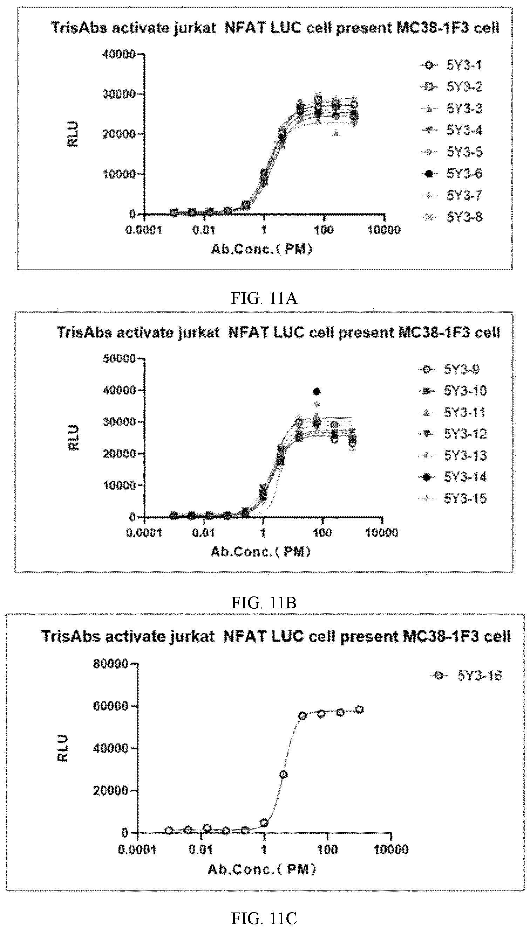

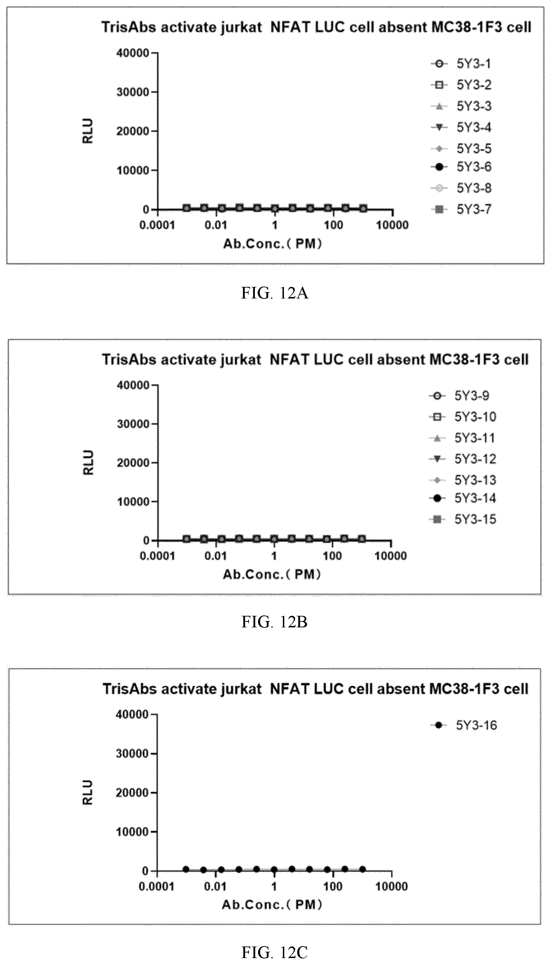

The determination results of the tumor group are shown in FIGs. 11A-11C, and the determination results of the tumor-free group are shown in FIGs. 12A-12C. The results show that the CD3-PDL1-MSLN trispecific antibody of the present disclosure could not activate Jurkat-NFAT-luc cells without adding tumor cells, but it could moderately activate Jurkat-NFAT-luc cells after adding tumor cells.

### Example 8: CD3-PDL1-MSLN trispecific antibody-mediated TDCC

### 8.1 Preparation of HCC1806-luc cells

The Luc sequence (NCBI: GenBank: MF062157.1) was cloned into the lentiviral vector (plvx-IRES-puro, psPAX2, pMD2G). The constructed lentiviral plasmids and packaging plasmids were co-transfected into HEK293T cells, and the cell supernatants were collected at 48 hours. The cell supernatants were added to HCC1806 cells (ATCC, CRL-2335, human breast cancer cells), and 4 µg/mL puromycin was added for screening at 24 hours. Single cells expressing Luc were sorted by a cell sorter (Sony, LESH800SBP), and finally stable HCC1806-luc cells were obtained.

### 8.2 Preparation of PBMC

Frozen PBMC cells (Shanghai Sailybio) were resuscitated and centrifuged at 500 g for 3 minutes to remove the supernatant. The cells were then washed once with 1640 + 10% FBS (inactivated) and centrifuged at 500 g for 3 minutes to remove the supernatant. The cells were resuspended in an appropriate amount of 1640 + 10% FBS (inactivated), counted, diluted to the corresponding density, and added to the well plate with 25 µL per well, resulting in the total number of PBMC cells to be 1.25×10⁵ cells/well.

### 8.3 HCC1806-luc cell plating

25 µL of HCC1806-luc cells were taken and added to the above PBMC well plate at 1.25 × 10⁴ cells/well.

### 8.4 Dilution of CD3-PDL1-MSLN trispecific antibody

For 5Y3-1 to 5Y3-15, the concentration was adjusted to 2 nM with culture medium and then gradient-diluted; and 50 µL of the diluted antibodies were added to PBMC and HCC1806-luc cells.

For 5Y3-16, the concentration was adjusted to 20 nM with culture medium and then gradient-diluted; and 100 µL of the diluted antibodies were added to PBMC and HCC1806-luc cells.

### 8.5 Incubation

Incubation was carried out for 2 days in an incubator at 37°C, 5% CO₂.

### 8.6 Readings

The well plate was taken out, and 50 µL of luciferase substrate was added. The data were then read on the instrument and analyzed.

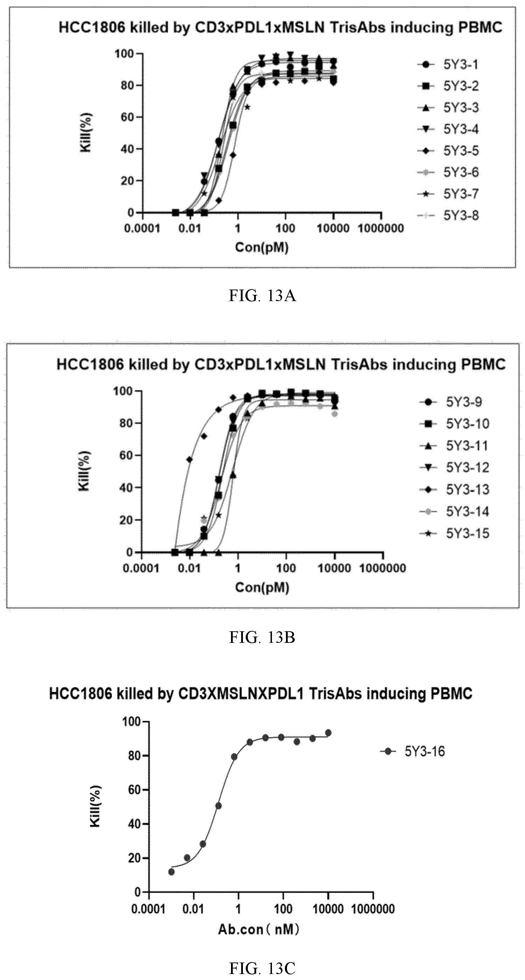

The experimental results are shown in FIGs. 13A-13C. The CD3-PDL1-MSLN trispecific antibody can exert T-cell dependent cellular cytotoxicity (TDCC) effect on human tumor cells HCC1806 expressing MSLN.

### Example 9: CD3-PDL1-MSLN trispecific antibody cytokine assay

For 5Y3-1 to 5Y3-16, the CD3-PDL1-MSLN trispecific antibodies and the positive control antibody HPN536 (HPN536 is a trispecific antibody targeting CD3/MSLN/HAS developed by Harpoon Therapeutics, the sequence of which is as set forth in SEQ ID NO: 66, and the above HPN536 sequence was constructed into a PTT5 expression vector (Unibio, lot: VT2202), and transiently expressed by an Expicho-s expression system (Gibco), and subsequently purified by a protein A chromatography column to obtain the HPN536 antibody) were adjusted to concentrations of 200 nM, 100 nM, 50 nM, 25 nM, and 12.5 nM in five gradients. Diluted trispecific antibody (50 µL/well) or control antibody was added, with the final well not added with trispecific antibody or control antibody. The PBMC cell density was adjusted to 2 × 10⁶ cells/mL at 50 µL/well. The cells were cultured at 37°C, 5% CO₂ for 48 hours. 30 µL of supernatant was respectively taken at 24 hours and 48 hours, and the HTRF kit (cisbio 62HIL02PEH, 62HIFNGPEH) was used to detect the IL2 and INF-γ contents in the supernatant respectively.

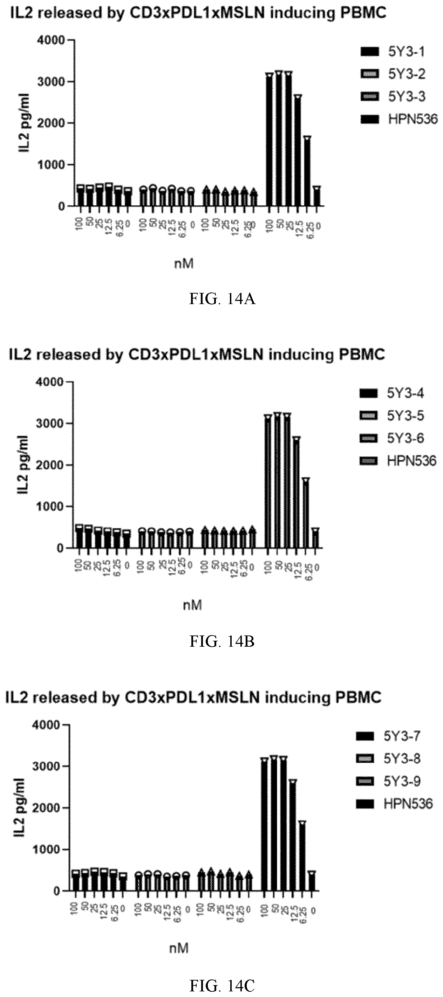

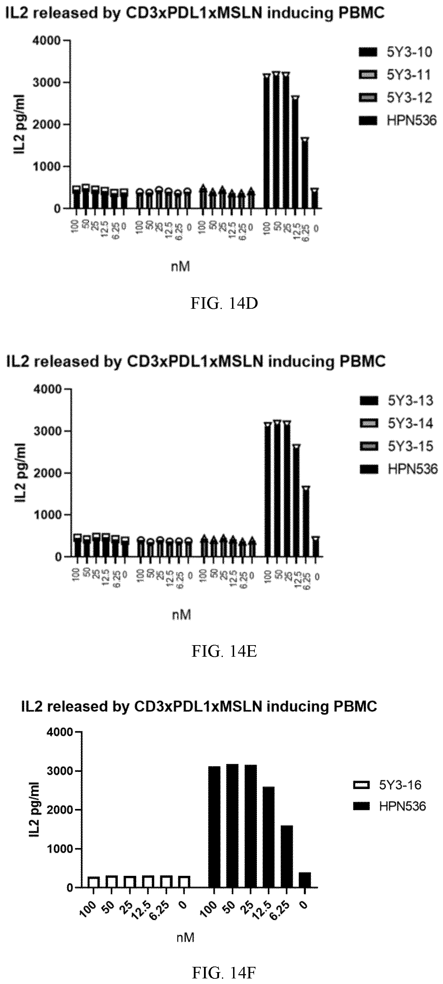

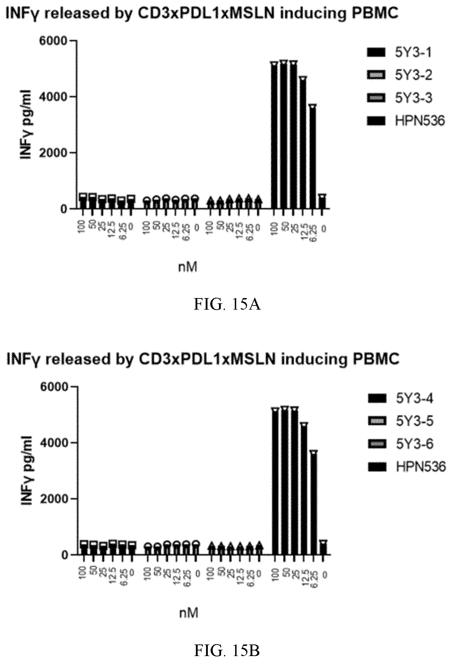

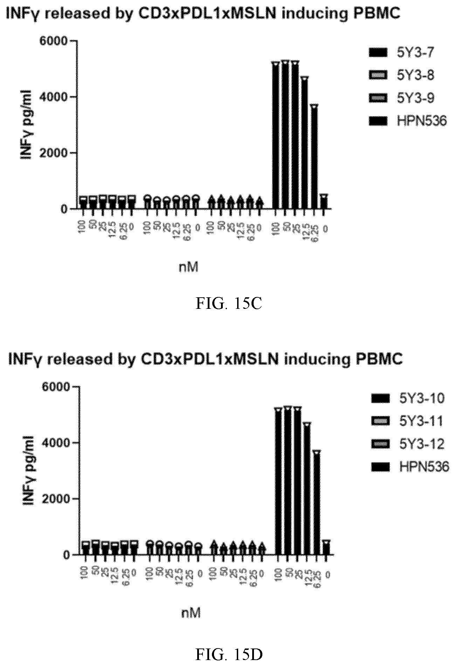

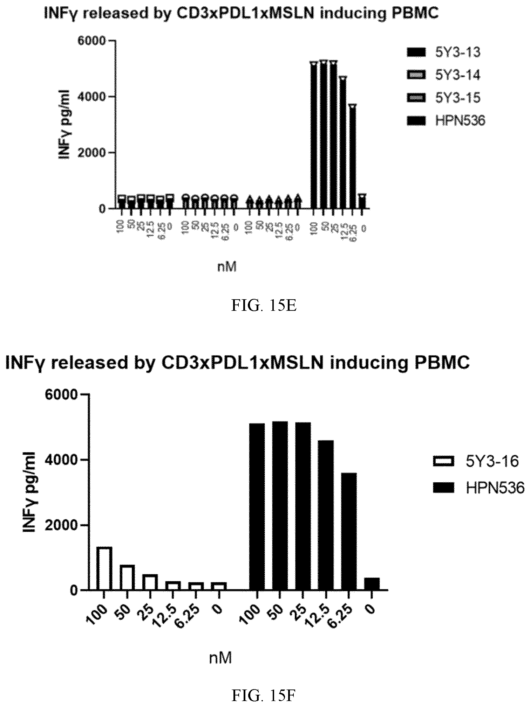

The IL2 detection results are shown in FIGs. 14A-14F, and the INF-γ detection results are shown in FIGs. 15A-15F. The results show that the CD3-PDL1-MSLN trispecific antibody of the present disclosure is co-incubated with PBMC. Compared with the antibody of control group which is stronger in promoting the secretion of IL2 and INF-γ, the trispecific antibody of the present disclosure is much weaker in promoting the secretion of IL2 and INF-γ, indicating that the trispecific antibody of the present disclosure has a low risk for the side effect of inducing PBMC to produce IL2 and INF-γ and has good safety.

### Example 10: PBMC immune reconstitution mouse subcutaneous HCC1806 xenograft tumor model

6 to 8-week female NOG-dKO (NOG-MHC I/II-2 KO Mice) mice (Beijing Vital River Laboratory Animal Technology Co., Ltd.) were selected; 5 × 10^{^7} PBMC cells were injected intraperitoneally into each mouse, and after 7 days, 3 × 10^{^6} HCC1806 cells were inoculated subcutaneously into each mouse. When tumors grew to a mean value of about 80 mm³, the mice were randomly grouped, with 8 mice in each administration group. The administration group was respectively set as 0.05 mg/kg administration group, 0.25 mg/kg administration group, and 1 mg/kg administration group, and the administration was carried out twice a week for a total of 4 doses; the 8 mice in the control group were not administered. The tumor volume and body weight of the mice were monitored. After the experiment, the mice were killed by cervical dislocation, and the tumors were weighed and recorded.

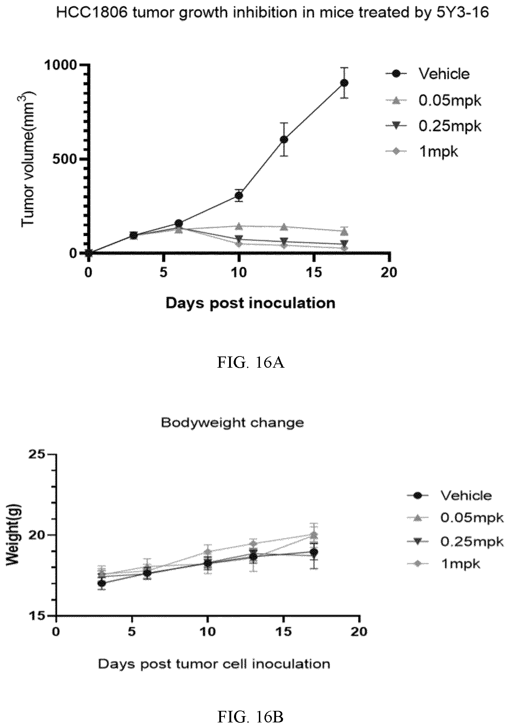

The results are shown in FIG. 16A. The 5Y3-16 antibody has good efficacy and the efficacy *in vivo* is dose-dependent. The tumor inhibition rates at three doses of 0.05 mg/kg, 0.25 mg/kg, and 1 mg/kg are 81%, 101%, and 106%, respectively. As shown in FIG. 16B, the tumor-bearing mice has good tolerance to the above doses and presented no adverse reactions such as weight loss.

### Example 11: CD3-PDL1-MSLN trispecific antibody-mediated TDCC with different structures

### 11.1 MC38-luc, CT26-luc, H2052-luc, HCC1806-luc cell preparation

The Luc sequence (NCBI: GenBank: MF062157.1) was cloned into the lentiviral vector (plvx-IRES-puro, psPAX2, pMD2G), and the constructed lentiviral plasmids and packaging plasmids were co-transfected into HEK293T cells. The cell supernatant was collected at 48 hours, and then added respectively to MC38 cells (ATCC, CRL-2640, mouse colon cancer cells), CT26 cells (ATCC, CRL-2638, mouse colon cancer cells), and H2052 cells (ATCC, CRL-5915, human mesothelioma cells), and 4 µg/mL puromycin was added for screening at 24 hours respectively. Single cells expressing Luc were sorted by a cell sorter (Sony, LESH800SBP), and finally stable MC38-luc, CT26-luc, and H2052-luc cells were obtained, and the HCC1806-luc cells prepared in Example 8 were obtained.

### 11.2 Preparation of PBMC

Frozen PBMC cells (Shanghai Sailybio) were resuscitated and centrifuged at 500 g for 3 minutes to remove the supernatant. The cells were then washed once with 1640 + 10% FBS (inactivated) and centrifuged at 500 g for 3 minutes to remove the supernatant. The cells were resuspended in an appropriate amount of 1640 + 10% FBS (inactivated), counted, diluted to the corresponding density, and added to the well plate with 25 µL per well, resulting in the total number of PBMC cells to be 1.25×10⁵ cells/well.

### 11.3 MC38-luc, CT26-luc, H2052-luc, HCC1806-luc cell plating

25 µL of MC38-luc, CT26-luc, HCC1806-luc, and H2052-luc cells were respectively added to the PBMC well plate in the above step 11.2, with 1.25 × 10⁴ cells per well.

### 11.4 Dilution of CD3-PDL1-MSLN trispecific antibodies with different structures

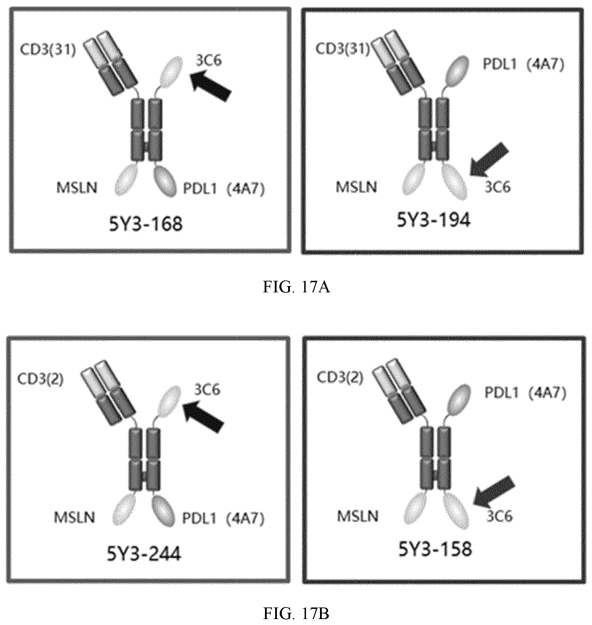

The structures of the 5Y3-158, 5Y3-168, 5Y3-194, and 5Y3-244 trispecific antibodies of this example are shown in FIGs. 17A-17B, wherein:
the structures of 5Y3-168 and 5Y3-244 are as in the structure of the trispecific antibody of 3.1 in Example 3.

5Y3-158 and 5Y3-194 both consist of three chains: 1. light chain, consisting of a CD3 antibody light chain variable region and a light chain CL1 constant region (SEQ ID NO: 44); 2. heavy chain 1, sequentially having a humanized CD3 heavy chain variable region, the C-terminus of mutant 1 of IgG1 (SEQ ID NO: 45) connected to a humanized MSLN single-domain antibody by (GGGGS)3; 3. heavy chain 2, sequentially having a PDL1 single-domain antibody connected to the N-terminus of mutant 2 of IgG1 (SEQ ID NO: 47) by (GGGGS)1, and the C-terminus of mutant 2 of IgG1 connected to a humanized MSLN single-domain antibody by (GGGGS)3. The sequences of 5Y3-158, 5Y3-168, 5Y3-194, and 5Y3-244 are shown in Table 18.

**Table 18. Trispecific antibody sequence combination**

| Trispecific antibody sequence combination | | | | |
|---|---|---|---|---|
| Name | 5Y3-158 | 5Y3-244 | 5Y3-168 | 5Y3-194 |
| CD3-VH | CD3-VH1 (SEQ ID NO: 40) | | CD3-VH4 (SEQ ID NO: 64) | |
| PDL1 | 4A7 (SEQ ID NO: 10) | | | |
| MSL N | 3C6 (SEQ ID NO: 9) | | | |
| CD3-VL | CD3-VL (SEQ ID NO: 43) | | | |

CD3-PDL1-MSLN trispecific antibodies with different structures (5Y3-158, 5Y3-168, 5Y3-194, and 5Y3-244) were gradient diluted in culture medium (RPMI-1640, Gibco 61870036); 50 µL of the diluted antibodies were respectively added to the cells containing PBMC and MC38-luc, CT26-luc, HCC1806-luc, and H2052-luc obtained in step 11.3.

### 11.5 Incubation

Incubation was carried out for 2 days in a 37°C, 5% CO₂ incubator.

### 11.6 Readings

The well plate was taken out, and 50 µL of luciferase substrate was added. The data were then read on the machine and analyzed.

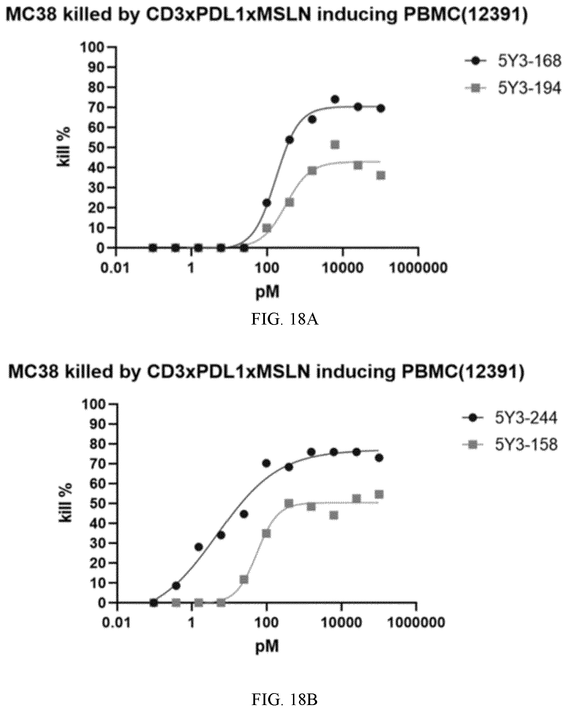

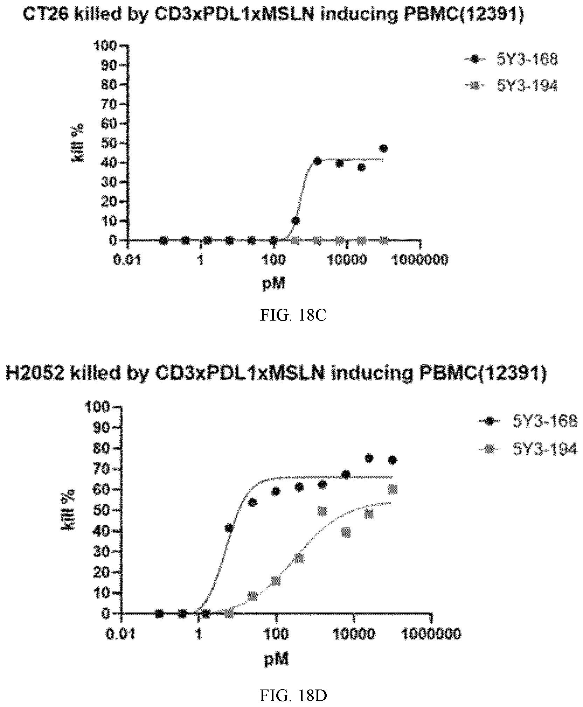

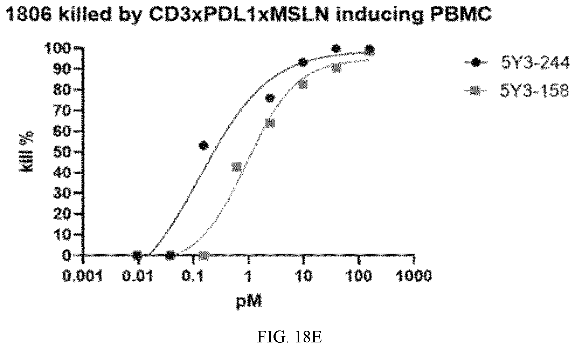

The experimental results are shown in FIGs. 18A-18E. The CD3-PDL1-MSLN trispecific antibodies with different structures (5Y3-158, 5Y3-168, 5Y3-194, and 5Y3-244) can exert a T-cell dependent cellular cytotoxicity (TDCC) effect on human tumor cells HCC1806 expressing MSLN, wherein the killing effect of 5Y3-168 is significantly superior to that of 5Y3-194, and the killing effect of 5Y3-244 is significantly superior to that of 5Y3-158. This indicates that with the same sequence, when one MSLN antibody is located at each of the N-terminus and the C-terminus of the Fc, the effect of killing the tumor is superior.

Although specific embodiments of the present disclosure have been described in detail, those skilled in the art will understand that: in accordance with all the published teachings, various modifications and changes to the details may be made, and these changes are within the scope of protection of the present disclosure. The entirety of the present disclosure is given by the following claims and any equivalents thereof.

## Claims

1. A humanized single-domain antibody or an antigen-binding fragment thereof capable of specifically binding to MSLN, wherein the humanized single-domain antibody or the antigen-binding fragment thereof comprises a VHH sequence as set forth in any one of SEQ ID NOs: 1-8 or a variant thereof,
wherein the variant has at least 70%, at least 80%, at least 90%, at least 91%, at least 92%, at least 93%, at least 94%, at least 95%, at least 96%, at least 97%, at least 98%, or at least 99% sequence identity compared to the sequence from which it is derived,
or, the variant has one or more amino acid substitutions, deletions, or additions compared to the sequence from which it is derived; preferably, the substitution is a conservative substitution.

2. A single-domain antibody or an antigen-binding fragment thereof capable of specifically binding to PD-L1, wherein the single-domain antibody or the antigen-binding fragment thereof comprises CDR1, CDR2, and CDR3 comprised in a VHH as set forth in any one of SEQ ID NOs: 10, 24-39, 67, and 81-88;
preferably, the CDRs are determined by IMGT, Kabat, AbM, Chothia, or Contact numbering system.

3. The single-domain antibody or the antigen-binding fragment thereof according to claim 2, wherein the single-domain antibody or the antigen-binding fragment thereof comprises:
(1) the following CDRs as defined by the IMGT numbering system:
(1A) CDR1 comprising the sequence as set forth in SEQ ID NO: 11 or 63, CDR2 comprising the sequence as set forth in SEQ ID NO: 12, and CDR3 comprising the sequence as set forth in SEQ ID NO: 13;
or,
(1B) CDR1 comprising the sequence as set forth in SEQ ID NO: 68, CDR2 comprising the sequence as set forth in SEQ ID NO: 69, and CDR3 comprising the sequence as set forth in SEQ ID NO: 70;
(2) the following CDRs as defined by the Kabat numbering system:
(2A) CDR1 comprising the sequence as set forth in SEQ ID NO: 14, CDR2 comprising the sequence as set forth in SEQ ID NO: 15, and CDR3 comprising the sequence as set forth in SEQ ID NO: 16;
or,
(2B) CDR1 comprising the sequence as set forth in SEQ ID NO: 71, CDR2 comprising the sequence as set forth in SEQ ID NO: 72, and CDR3 comprising the sequence as set forth in SEQ ID NO: 73;
(3) the following CDRs as defined by the AbM numbering system:
(3A) CDR1 comprising the sequence as set forth in SEQ ID NO: 17, CDR2 comprising the sequence as set forth in SEQ ID NO: 18, and CDR3 comprising the sequence as set forth in SEQ ID NO: 16;
or,
(3B) CDR1 comprising the sequence as set forth in SEQ ID NO: 74, CDR2 comprising the sequence as set forth in SEQ ID NO: 75, and CDR3 comprising the sequence as set forth in SEQ ID NO: 73;
(4) the following CDRs as defined by the Chothia numbering system:
(4A) CDR1 comprising the sequence as set forth in SEQ ID NO: 19, CDR2 comprising the sequence as set forth in SEQ ID NO: 20, and CDR3 comprising the sequence as set forth in SEQ ID NO: 16;
or,
(4B) CDR1 comprising the sequence as set forth in SEQ ID NO: 76, CDR2 comprising the sequence as set forth in SEQ ID NO: 77, and CDR3 comprising the sequence as set forth in SEQ ID NO: 73;
or,
(5) the following CDRs as defined by the Contact numbering system:
(5A) CDR1 comprising the sequence as set forth in SEQ ID NO: 21, CDR2 comprising the sequence as set forth in SEQ ID NO: 22, and CDR3 comprising the sequence as set forth in SEQ ID NO: 23;
or,
(5B) CDR1 comprising the sequence as set forth in SEQ ID NO: 78, CDR2 comprising the sequence as set forth in SEQ ID NO: 79, and CDR3 comprising the sequence as set forth in SEQ ID NO: 80.

4. The single-domain antibody or the antigen-binding fragment thereof according to claim 2 or 3, wherein the single-domain antibody or the antigen-binding fragment thereof comprises a VHH sequence as set forth in SEQ ID NO: 10 or 67, or a variant thereof,
wherein the variant has at least 70%, at least 80%, at least 85%, at least 90%, at least 91%, at least 92%, at least 93%, at least 94%, at least 95%, at least 96%, at least 97%, at least 98%, or at least 99% sequence identity compared to the sequence from which it is derived,
or, wherein the variant has one or more amino acid substitutions, deletions, or additions compared to the sequence from which it is derived; preferably, the substitution is a conservative substitution.

5. The single-domain antibody or the antigen-binding fragment thereof according to any one of claims 2 to 4, wherein the single-domain antibody or the antigen-binding fragment thereof is humanized;
preferably, the single-domain antibody or the antigen-binding fragment thereof comprises a heavy chain framework region of a human immunoglobulin, and the heavy chain framework region optionally comprises a reverse mutation from a human residue to a camelid residue.

6. The single-domain antibody or the antigen-binding fragment thereof according to claim 5, wherein the single-domain antibody or the antigen-binding fragment thereof comprises a VHH sequence as set forth in any one of SEQ ID NOs: 24-39 and 81-88, or a variant thereof,
wherein the variant has at least 70%, at least 80%, at least 85%, at least 90%, at least 91%, at least 92%, at least 93%, at least 94%, at least 95%, at least 96%, at least 97%, at least 98%, or at least 99% sequence identity compared to the sequence from which it is derived,
or, the variant has one or more amino acid substitutions, deletions, or additions compared to the sequence from which it is derived; preferably, the substitution is a conservative substitution.

7. The single-domain antibody or the antigen-binding fragment thereof according to any one of claims 2 to 6, wherein the PD-L1 is selected from human PD-L1, mouse PD-L1, camel PD-L1 and/or monkey PD-L1.

8. A polypeptide construct comprising the humanized single-domain antibody or the antigen-binding fragment thereof according to claim 1, and/or the single-domain antibody or the antigen-binding fragment thereof according to any one of claims 2 to 7, and an immunoglobulin Fc domain;
preferably, the immunoglobulin Fc domain is optionally linked to the N-terminus and/or C-terminus (e.g., C-terminus) of the humanized single-domain antibody or the antigen-binding fragment thereof or the single-domain antibody or the antigen-binding fragment thereof via a peptide linker;
preferably, the immunoglobulin Fc domain is an Fc domain of IgG (*e.g.,* the Fc domain of IgG1);
preferably, the immunoglobulin Fc domain comprises the sequence as set forth in SEQ ID NO: 48;
preferably, the peptide linker is a peptide linker comprising one or more glycines and/or one or more serines, e.g., (G4S)n, n being 1, 2, 3, or 4, *e.g.,* the sequence as set forth in SEQ ID NO: 50.

9. A multispecific antibody comprising: the humanized single-domain antibody or the antigen-binding fragment thereof capable of specifically binding to MSLN according to claim 1 and/or the single-domain antibody or the antigen-binding fragment thereof capable of specifically binding to PD-L1 according to any one of claims 2 to 7;
preferably, the multispecific antibody is a bispecific antibody, a trispecific antibody, or a tetraspecific antibody.

10. A multispecific antibody comprising: a first antigen-binding domain targeting CD3, a second antigen-binding domain targeting MSLN, and a third antigen-binding domain targeting PD-L1, wherein
(i) the first antigen-binding domain comprises a VH and a VL, wherein the VH comprises HCDR1 as set forth in SEQ ID NO: 55, HCDR2 as set forth in SEQ ID NO: 56, and HCDR3 as set forth in any one of SEQ ID NOs: 57-59 and 65; and/or, the VL comprises LCDR1 as set forth in SEQ ID NO: 60, LCDR2 as set forth in SEQ ID NO: 61, and LCDR3 as set forth in SEQ ID NO: 62; preferably, the VH comprises the sequence as set forth in any one of SEQ ID NOs: 40-42 and 64; preferably, the VL comprises the sequence as set forth in SEQ ID NO: 43;
(ii) the second antigen-binding domain comprises a VHH, wherein the VHH comprises CDR1, CDR2, and CDR3 comprised in the VHH as set forth in any one of SEQ ID NOs: 1-9; preferably, the VHH comprises CDR1 as set forth in SEQ ID NO: 52, CDR2 as set forth in SEQ ID NO: 53, and CDR3 as set forth in SEQ ID NO: 54; preferably, the second antigen-binding domain comprises the humanized single-domain antibody or the antigen-binding fragment thereof according to claim 1 or comprises the VHH sequence as set forth in SEQ ID NO: 9;
and/or,
(iii) the third antigen-binding domain comprises the single-domain antibody or the antigen-binding fragment thereof according to any one of claims 2 to 7.

11. A multispecific antibody comprising: a first antigen-binding domain targeting CD3, a second antigen-binding domain targeting a tumor-associated antigen (TAA), and a third antigen-binding domain targeting an immune checkpoint, wherein the first antigen-binding domain is Fab, and the second and third antigen-binding domains are VHH;
preferably, the multispecific antibody further comprises an Fc domain, wherein the Fc domain comprises a first Fc monomer and a second Fc monomer; wherein:
the N-terminus of the first Fc monomer is optionally linked to the first antigen-binding domain (e.g., the heavy chain CH1 domain thereof) via a linker, and the C-terminal thereof is optionally linked to the second antigen-binding domain via a linker;
the N-terminus of the second Fc monomer is optionally linked to another second antigen-binding domain via a linker, and the C-terminal thereof is optionally linked to the third antigen-binding domain via a linker.

12. The multispecific antibody according to claim 11, wherein the tumor-associated antigen comprises MSLN.

13. The multispecific antibody according to claim 11 or 12, wherein the immune checkpoint is selected from PD1, PD-L1, or a combination thereof;
preferably, the immune checkpoint is PD-L1.

14. The multispecific antibody according to any one of claims 11 to 13, wherein the Fc domain comprises a modification to promote dimerization of the first Fc monomer and the second Fc monomer;
preferably, the modification comprises an amino acid substitution in the CH3 domain of the Fc domain;
preferably, the modification comprises a "knob" modification in one of the two monomers and a "hole" modification in the other of the two monomers to form a "knob-into-hole" modification;
preferably, the first Fc monomer and the second Fc monomer of the Fc domain comprise amino acid sequences as set forth in SEQ ID NOs: 46 and 47, respectively.

15. The multispecific antibody according to any one of claims 11 to 14, wherein the linker is selected from a peptide linker comprising one or more glycines and/or one or more serines;
preferably, the peptide linker comprises (G4S)n, n being 1, 2, 3, or 4, *e.g.,* comprising the sequence as set forth in any one of SEQ ID NOs: 49-51.

16. The multispecific antibody according to any one of claims 11 to 15, wherein the multispecific antibody comprises:
(i) a first peptide chain comprising a VL and a light chain constant region (CL) of the first antigen-binding domain; preferably, the CL is a kappa light chain constant region;
(ii) a second peptide chain comprising a VH, CH1 of the first antigen-binding domain, and a first Fc monomer, and the second antigen-binding domain; preferably, the first Fc monomer is IgG; preferably, the first Fc monomer comprises a hinge region, CH2, and CH3; preferably, the second antigen-binding domain is linked to the C-terminus of the first Fc monomer by a linker (such as a flexible peptide comprising (G4S)n);
and
(iii) a third peptide chain comprising the second antigen-binding domain, the second Fc monomer, and the third antigen-binding domain; preferably, the second Fc monomer is IgG; preferably, the second Fc monomer comprises a hinge region, CH2, and CH3; preferably, the second antigen-binding domain is linked to the N-terminus of the second Fc monomer by a linker (such as a flexible peptide comprising (G4S)n); preferably, the third antigen-binding domain is linked to the C-terminus of the second Fc monomer by a linker (such as a flexible peptide comprising (G4S)n);
preferably, the second Fc monomer of the third peptide chain is capable of forming a dimer with the first Fc monomer of the second peptide chain.

17. The multispecific antibody according to claim 16, wherein the first Fc monomer of the second peptide chain and the second Fc monomer of the third peptide chain comprise a modification to promote dimerization;
preferably, the modification comprises an amino acid substitution in the CH3 domain of the Fc domain;
preferably, the modification comprises a "knob" modification in one of the two Fc monomers and a "hole" modification in the other of the two Fc monomers to form a "knob-into-hole" modification;
preferably, the two Fc monomers comprise amino acid sequences as set forth in SEQ ID NOs: 46 and 47, respectively;
preferably, the first Fc monomer of the second peptide chain comprises the amino acid sequence as set forth in SEQ ID NO: 46; preferably, the second peptide chain comprises the sequence of the heavy chain constant region as set forth in SEQ ID NO: 45;
preferably, the second Fc monomer of the third peptide chain comprises the amino acid sequence as set forth in SEQ ID NO: 47.

18. The multispecific antibody according to any one of claims 11 to 17, wherein the first antigen-binding domain targeting CD3 comprises a VH and a VL, wherein the VH comprises HCDR1 as set forth in SEQ ID NO: 55, HCDR2 as set forth in SEQ ID NO: 56, and HCDR3 as set forth in any one of SEQ ID NOs: 57-59 and 65; and/or, the VL comprises LCDR1 as set forth in SEQ ID NO: 60, LCDR2 as set forth in SEQ ID NO: 61, and LCDR3 as set forth in SEQ ID NO: 62;
preferably, the VH comprises the sequence as set forth in any one of SEQ ID NOs: 40-42 and 64;
preferably, the VL comprises the sequence as set forth in SEQ ID NO: 43.

19. The multispecific antibody according to any one of claims 11 to 18, wherein the second antigen-binding domain targeting MSLN comprises a VHH, wherein the VHH comprises CDR1, CDR2, and CDR3 comprised in the VHH as set forth in any one of SEQ ID NOs: 1-9; preferably, the VHH comprises CDR1 as set forth in SEQ ID NO: 52, CDR2 as set forth in SEQ ID NO: 53, and CDR3 as set forth in SEQ ID NO: 54;
preferably, the second antigen-binding domain comprises the humanized single-domain antibody or the antigen-binding fragment thereof according to claim 1 or a VHH sequence as set forth in SEQ ID NO: 9.

20. The multispecific antibody according to any one of claims 11 to 19, wherein the third antigen-binding domain targeting PD-L1 comprises the single-domain antibody or the antigen-binding fragment thereof capable of specifically binding to PD-L1 according to any one of claims 2 to 7.

21. The multispecific antibody according to any one of claims 11 to 20, wherein the multispecific antibody comprises:
(i) a first peptide chain having a structure shown in [VL]-[CL],
(ii) a second peptide chain having a structure shown in [VH]-[CH]-[L1]-[VHH1], and
(iii) a third peptide chain having a structure shown in [VHH1]-[L2]-[Fc monomer]-[L3]-[VHH2];
wherein the multispecific antibody is selected from any one of the following:
(1) the VL comprises the sequence as set forth in SEQ ID NO: 43, and the CL comprises the sequence as set forth in SEQ ID NO: 44; the VH comprises the sequence as set forth in SEQ ID NO: 40, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 81; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
(2) the VL comprises the sequence as set forth in SEQ ID NO: 43, and the CL comprises the sequence as set forth in SEQ ID NO: 44; the VH comprises the sequence as set forth in SEQ ID NO: 40, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 29; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
(3) the VL comprises the sequence as set forth in SEQ ID NO: 43, and the CL comprises the sequence as set forth in SEQ ID NO: 44; the VH comprises the sequence as set forth in SEQ ID NO: 41, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 29; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
(4) the VL comprises the sequence as set forth in SEQ ID NO: 43, and the CL comprises the sequence as set forth in SEQ ID NO: 44; the VH comprises the sequence as set forth in SEQ ID NO: 42, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 29; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
(5) the VL comprises the sequence as set forth in SEQ ID NO: 43, and the CL comprises the sequence as set forth in SEQ ID NO: 44; the VH comprises the sequence as set forth in SEQ ID NO: 40, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 30; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
(6) the VL comprises the sequence as set forth in SEQ ID NO: 43, and the CL comprises the sequence as set forth in SEQ ID NO: 44; the VH comprises the sequence as set forth in SEQ ID NO: 41, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 30; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
(7) the VL comprises the sequence as set forth in SEQ ID NO: 43, and the CL comprises the sequence as set forth in SEQ ID NO: 44; the VH comprises the sequence as set forth in SEQ ID NO: 42, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 30; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
(8) the VL comprises the sequence as set forth in SEQ ID NO: 43, and the CL comprises the sequence as set forth in SEQ ID NO: 44; the VH comprises the sequence as set forth in SEQ ID NO: 40, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 38; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
(9) the VL comprises the sequence as set forth in SEQ ID NO: 43, and the CL comprises the sequence as set forth in SEQ ID NO: 44; the VH comprises the sequence as set forth in SEQ ID NO: 41, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 38; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
(10) the VL comprises the sequence as set forth in SEQ ID NO: 43, and the CL comprises the sequence as set forth in SEQ ID NO: 44; the VH comprises the sequence as set forth in SEQ ID NO: 42, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 38; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
(11) the VL comprises the sequence as set forth in SEQ ID NO: 43, and the CL comprises the sequence as set forth in SEQ ID NO: 44; the VH comprises the sequence as set forth in SEQ ID NO: 40, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 31; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
(12) the VL comprises the sequence as set forth in SEQ ID NO: 43, and the CL comprises the sequence as set forth in SEQ ID NO: 44; the VH comprises the sequence as set forth in SEQ ID NO: 41, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 31; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
(13) the VL comprises the sequence as set forth in SEQ ID NO: 43, and the CL comprises the sequence as set forth in SEQ ID NO: 44; the VH comprises the sequence as set forth in SEQ ID NO: 42, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 31; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
(14) the VL comprises the sequence as set forth in SEQ ID NO: 43, and the CL comprises the sequence as set forth in SEQ ID NO: 44; the VH comprises the sequence as set forth in SEQ ID NO: 40, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 39; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
(15) the VL comprises the sequence as set forth in SEQ ID NO: 43, and the CL comprises the sequence as set forth in SEQ ID NO: 44; the VH comprises the sequence as set forth in SEQ ID NO: 41, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 39; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
(16) the VL comprises the sequence as set forth in SEQ ID NO: 43, and the CL comprises the sequence as set forth in SEQ ID NO: 44; the VH comprises the sequence as set forth in SEQ ID NO: 42, the CH comprises the sequence as set forth in SEQ ID NO: 45, and the VHH1 comprises the sequence as set forth in SEQ ID NO: 8; the Fc monomer comprises the sequence as set forth in SEQ ID NO: 47, and the VHH2 comprises the sequence as set forth in SEQ ID NO: 39; L1, L2, and L3 are peptide linkers, preferably each independently selected from peptide linkers comprising one or more glycines and/or one or more serines (such as peptide linkers shown in (G4S)n), preferably, L1 is SEQ ID NO: 51, L2 is SEQ ID NO: 49, and L3 is SEQ ID NO: 51;
(17) the VL comprises the sequence as set forth in SEQ ID NO: 43, and the CL comprises the sequence as set forth in SEQ ID NO: 44; the VH comprises the sequence as set forth in SEQ ID NO: 64, the CH comprises the sequence as set forth in SEQ ID NO: 45, and the VHH1 comprises the sequence as set forth in SEQ ID NO: 9; the Fc monomer comprises the sequence as set forth in SEQ ID NO: 47, and the VHH2 comprises the sequence as set forth in SEQ ID NO: 10; L1, L2, and L3 are peptide linkers, preferably each independently selected from peptide linkers comprising one or more glycines and/or one or more serines (such as peptide linkers shown in (G4S)n); preferably, L1 is SEQ ID NO: 51, L2 is SEQ ID NO: 49, and L3 is SEQ ID NO: 51; or,
(18) the VL comprises the sequence as set forth in SEQ ID NO: 43, and the CL comprises the sequence as set forth in SEQ ID NO: 44; the VH comprises the sequence as set forth in SEQ ID NO: 40, the CH comprises the sequence as set forth in SEQ ID NO: 45, and the VHH1 comprises the sequence as set forth in SEQ ID NO: 9; the Fc monomer comprises the sequence as set forth in SEQ ID NO: 47, and the VHH2 comprises the sequence as set forth in SEQ ID NO: 10; L1, L2, and L3 are peptide linkers, preferably each independently selected from peptide linkers comprising one or more glycines and/or one or more serines (such as peptide linkers shown in (G4S)n); preferably, L1 is SEQ ID NO: 51, L2 is SEQ ID NO: 49, and L3 is SEQ ID NO: 51.

22. An isolated nucleic acid molecule encoding: (i) the humanized single-domain antibody or the antigen-binding fragment thereof according to claim 1, (ii) the single-domain antibody or the antigen-binding fragment thereof according to any one of claims 2 to 7, (iii) the polypeptide construct according to claim 8, or (iv) the multispecific antibody or the polypeptide chain thereof according to any one of claims 9 to 21.

23. A vector comprising the isolated nucleic acid molecule according to claim 22;
preferably, the vector comprises a nucleotide sequence encoding each peptide chain of the multispecific antibody, and the nucleotide sequences encoding each peptide chain are present on the same or on different vectors.

24. A host cell comprising the isolated nucleic acid molecule according to claim 22 or the vector according to claim 23.

25. A method for preparing a single-domain antibody or an antigen-binding fragment thereof, a polypeptide construct, or a multispecific antibody, comprising: culturing the host cell according to claim 24 under conditions allowing protein expression, and collecting the single-domain antibody, the antigen-binding fragment thereof, the polypeptide construct, or the multispecific antibody from the cultured host cell culture.

26. A conjugate comprising: the humanized single-domain antibody or the antigen-binding fragment thereof according to claim 1, the single-domain antibody or the antigen-binding fragment thereof according to any one of claims 2 to 7, the polypeptide construct according to claim 8, or the multispecific antibody according to any one of claims 9 to 21, and a coupling moiety linked thereto;
preferably, the coupling moiety is selected from: a detectable label (such as a radioisotope, a fluorescent substance, a luminescent substance, a colored substance, or an enzyme) or a therapeutic agent (such as cytotoxic agents, cytokines, toxins, radionuclides, immune agonists, immunosuppressants, and other active substances that inhibit the growth of tumor cells, promote apoptosis or necrosis of tumor cells).

27. A pharmaceutical composition comprising the humanized single-domain antibody or the antigen-binding fragment thereof according to claim 1, the single-domain antibody or the antigen-binding fragment thereof according to any one of claims 2 to 7, the polypeptide construct according to claim 8, the multispecific antibody according to any one of claims 9 to 21, the isolated nucleic acid molecule according to claim 22, the vector according to claim 23, the host cell according to claim 24, or the conjugate according to claim 26, and a pharmaceutically acceptable carrier and/or excipient.

28. A use of the humanized single-domain antibody or the antigen-binding fragment thereof according to claim 1, the single-domain antibody or the antigen-binding fragment thereof according to any one of claims 2 to 7, the polypeptide construct according to claim 8, the multispecific antibody according to any one of claims 9 to 21, the isolated nucleic acid molecule according to claim 22, the vector according to claim 23, the host cell according to claim 24, the conjugate according to claim 26, or the pharmaceutical composition according to claim 27 in the preparation of a medicament for the prevention and/or treatment and/or neoadjuvant treatment and/or adjuvant treatment of a disease.

29. A method for the prevention and/or treatment and/or neoadjuvant treatment and/or adjuvant treatment of a disease in a subject, comprising administering an effective amount of the humanized single-domain antibody or the antigen-binding fragment thereof according to claim 1, the single-domain antibody or the antigen-binding fragment thereof according to any one of claims 2 to 7, the polypeptide construct according to claim 8, the multispecific antibody according to any one of claims 9 to 21, the isolated nucleic acid molecule according to claim 22, the vector according to claim 23, the host cell according to claim 24, the conjugate according to claim 26, or the pharmaceutical composition according to claim 27 to the subject in need thereof.

30. The use according to claim 28 or the method according to claim 29, wherein the disease is a tumor; preferably, the tumor is a solid tumor or a hematological tumor; more preferably, the solid tumor is selected from mesothelioma, ovarian cancer, pancreatic cancer, cervical cancer, breast cancer, prostate cancer, cholangiocarcinoma, colon cancer, gastric cancer, fallopian tube cancer, lung cancer, or colorectal cancer; the hematological tumor comprises acute myeloid leukemia.

31. A method for detecting the presence or level of PD-L1, MSLN, and/or CD3 in a sample, comprising contacting the sample with the humanized single-domain antibody or the antigen-binding fragment thereof according to claim 1, the single-domain antibody or the antigen-binding fragment thereof according to any one of claims 2 to 7, the polypeptide construct according to claim 8, the multispecific antibody according to any one of claims 9 to 21, the isolated nucleic acid molecule according to claim 22, the vector according to claim 23, the host cell according to claim 24, the conjugate according to claim 26, or the pharmaceutical composition according to claim 27 under conditions allowing the formation of an antibody-antigen immune complex, and detecting the formation of the complex.

32. A method for diagnosing or differentially diagnosing a disease associated with PD-L1, MSLN, and/or CD3, wherein the method comprises using the humanized single-domain antibody or the antigen-binding fragment thereof according to claim 1, the single-domain antibody or the antigen-binding fragment thereof according to any one of claims 2 to 7, the polypeptide construct according to claim 8, the multispecific antibody according to any one of claims 9 to 21, the isolated nucleic acid molecule according to claim 22, the vector according to claim 23, the host cell according to claim 24, the conjugate according to claim 26, or the pharmaceutical composition according to claim 27;
preferably, the method is used to detect the presence or level of PD-L1, MSLN, and/or CD3 in a sample from a subject by the method according to claim 31 to diagnose or differentially diagnose a disease;
preferably, the disease is a tumor; preferably, the tumor is a solid tumor or a hematological tumor; more preferably, the solid tumor is selected from mesothelioma, ovarian cancer, pancreatic cancer, cervical cancer, breast cancer, prostate cancer, cholangiocarcinoma, colon cancer, gastric cancer, fallopian tube cancer, lung cancer, or colorectal cancer; the hematological tumor comprises acute myeloid leukemia.

33. A kit comprising the humanized single-domain antibody or the antigen-binding fragment thereof according to claim 1, the single-domain antibody or the antigen-binding fragment thereof according to any one of claims 2 to 7, the polypeptide construct according to claim 8, the multispecific antibody according to any one of claims 9 to 21, the isolated nucleic acid molecule according to claim 22, the vector according to claim 23, the host cell according to claim 24, the conjugate according to claim 26, or the pharmaceutical composition according to claim 27.
